(19) Europäisches Patentamt / European Patent Office / Office européen des brevets

(11) **EP 4 453 014 B1**

(12) **EUROPEAN PATENT SPECIFICATION**

(45) Date of publication and mention of the grant of the patent:
**14.01.2026 Bulletin 2026/03**

(21) Application number: **22857116.2**

(22) Date of filing: **23.12.2022**

(51) International Patent Classification (IPC):
**C07K 14/47** (2006.01)      **C07K 17/10** (2006.01)
**C07K 16/06** (2006.01)      **B01D 15/38** (2006.01)
**B01J 20/289** (2006.01)      **B01J 20/32** (2006.01)

(52) Cooperative Patent Classification (CPC):
**C07K 17/10; B01D 15/3804; B01J 20/289;
B01J 20/3212; B01J 20/3219; B01J 20/3274;
C07K 14/47; C07K 16/065;** C07K 2317/92

(86) International application number:
**PCT/US2022/053926**

(87) International publication number:
**WO 2023/122327 (29.06.2023 Gazette 2023/26)**

(54) **CH1 DOMAIN AFFINITY LIGANDS AND AGENTS**

CHI-DOMÄNENAFFINITÄTSLIGANDEN UND MITTEL

LIGANDS ET AGENTS D'AFFINITÉ DANS LE DOMAINE CHI

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **24.12.2021 US 202163293718 P
10.02.2022 US 202263308707 P
16.09.2022 US 202263407563 P**

(43) Date of publication of application:
**30.10.2024 Bulletin 2024/44**

(73) Proprietor: **Repligen Corporation
Waltham, MA 02453 (US)**

(72) Inventors:
• **SCANLON, Thomas**
  **Lebanon, New Hampshire 03766 (US)**
• **BELK, Jonathan**
  **Lebanon, New Hampshire 03766 (US)**
• **KEARNS, Kelley**
  **Lebanon, New Hampshire 03766 (US)**
• **VALENTINI, Sarah**
  **Lebanon, New Hampshire 03766 (US)**

(74) Representative: **Arnold & Siedsma
Bezuidenhoutseweg 57
2594 AC The Hague (NL)**

(56) References cited:
**WO-A1-2012/105833      WO-A2-2010/115118
WO-A2-2020/242988**

• **DERRICK JEREMY P & WIGLEY DALE B:
"Crystal structure of a streptococcal protein G
domain bound to an Fab fragment", NATURE,
vol. 359, 22 October 1992 (1992-10-22), pages 752
- 754, XP093041792, Retrieved from the Internet
<URL:https://www.nature.com/articles/
359752a0> [retrieved on 20230425]**
• **JACKREL MEREDITH E. ET AL: "Redesign of a
protein - peptide interaction: Characterization
and applications", PROTEIN SCIENCE, vol. 18,
12 February 2009 (2009-02-12), US, pages 762 -
774, XP093041417, ISSN: 0961-8368, Retrieved
from the Internet <URL:https://www.ncbi.nlm.
nih.gov/pmc/articles/PMC2762588/pdf/
pro0018-0762.pdf> DOI: 10.1002/pro.75**
• **MAGLIERY THOMAS J ET AL: "Sequence
variation in ligand binding sites in proteins",
BMC BIOINFORMATICS, BIOMED CENTRAL ,
LONDON, GB, vol. 6, no. 1, 30 September 2005
(2005-09-30), pages 240, XP021000844, ISSN:
1471-2105, DOI: 10.1186/1471-2105-6-240**

**Description**

**FIELD OF THE DISCLOSURE**

The present disclosure relates to the field of chromatography, and more specifically to novel affinity ligands and affinity agents which are suitable for use in isolation of antibody and antibody fragments that contain the heavy chain first constant region (CH1). The disclosure encompasses affinity ligands as such, chromatography separation matrices (affinity agents) comprising an affinity ligand according to the disclosure, and a process of isolating antibodies, especially IgGs, and CH1-containing antibody fragments thereof using an affinity ligand according to the disclosure. Methods of making the affinity ligands and separation matrices are also provided.

**BACKGROUND OF THE DISCLOSURE**

The purity of biologically produced therapeutics is tightly scrutinized and regulated by authorities to ensure safety and efficacy. Thus, there is a need to efficiently purify biologically produced therapeutics to a high degree of purity.

To support the clinical efforts for therapeutic proteins, compositions and methods to efficiently purify proteins from recombinant sources are needed. Affinity purification is a means to isolate and/or achieve desired purity of a protein in few steps, or a single step. However, the development of affinity agents (e.g., comprising an affinity ligand) can be a resource intensive and time-consuming task and hence affinity agents exist for very few proteins. In the absence of an affinity agent, purification typically involves inefficient processes, such as a multi-column process.

Exemplary therapeutic proteins include, but are not limited to, bioactive polypeptides/proteins, fusion proteins, enzymes, hormones, antibodies, and antibody fragments. Certain proteins, such as fusion proteins, present additional challenges for purification due to product homogeneity, or the existence of product related impurities. Some impurities may arise from incorrectly assembled fusion proteins and proteolytic cleavage which can be especially difficult to remove because they are closely related to the desired product.

Affinity agents that bind proteins and are useful for isolation and/or affinity purification are described herein. In some embodiments, an affinity agent comprises a solid support and a ligand.

Human monoclonal antibody therapy is a rapidly growing segment of the biologic pharmaceutical industry with very promising clinical applications in cancer therapy. Traditional methods for recombinant antibody purification utilize affinity chromatography resins based on Protein A ligands. Protein A based affinity resins provide highly efficient, high yield processes for purification of monoclonal antibodies, their utility in industrial antibody production processes highlights a need for suitable affinity purification devices.

A major drawback of monoclonal antibody therapy based on immunoglobulin G (IgG) is poor tumor penetration by the very large IgG molecule (~ 150 kDa). Increasingly, antibody fragments are being investigated that lack the large constant domain (Fc domain) of IgGs. Antibody fragments thus constructed maintain their native antigen-specificity but lack the ability to bind the Fc receptor. However, such antibody fragments also lack the Protein A binding site that enables purification with Protein A-based affinity resins, and thus new methods are needed for purification of antibody fragments that cannot bind to Protein A resins.

Exemplary antibody fragment formats currently being investigated in clinical trials include, for example, mono-specific Fab, monospecific $Fab_2$, bispecific Fab, bispecific $Fab_2$, mono and bi-specific diabody, triabody, antibody fragment - drug conjugates, etc. (reviewed in Nelson, 2010, mAbs, and others).

WO 2012/105833 A1 discloses a method for the purification of a molecule comprising a human IgG-CHl domain.

Many of the aforementioned antibody fragments contain a conserved region on the heavy chain, the CH1 domain. Affinity agents that bind antibodies or antibody fragments in the CH1 domain would be useful for isolation and/or affinity purification of such fragments. Affinity agents that bind antibodies or antibody fragments and are useful for isolation and/or purification are described herein. In some embodiments an affinity agent comprises a solid support and a ligand.

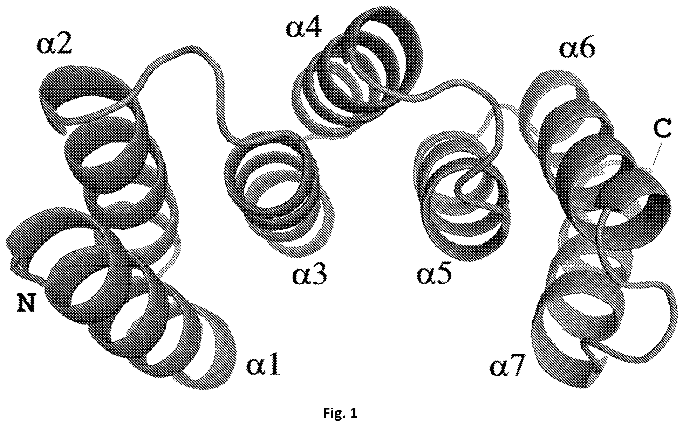

The tetratricopeptide repeat (TPR) proteins offer a potential scaffold to engineer binding specificities and create new binding ligands suitable for such affinity purification. TPRs contain repeating units of a 34-amino-acid sequence that adopts a helix-turn-helix structure. Combining three such domains with a seventh helix provides forms an elongated superhelical structure where helices 1, 3, 5 and 7 form an inner surface of the superhelix and the binding interface for its target (see Fig. 1). In accordance with this disclosure, a series of affinity ligands based on non-naturally occurring TPRs have been developed for binding antibodies or antibody fragments in the CH1 domain. Moreover, the resulting TPR domain-based ligands have high stability including the ability to withstand CIP with NaOH. Collectively, these properties make possible the use of affinity ligands that can withstand the rigors of modern bioprocessing.

**SUMMARY OF THE DISCLOSURE**

The present disclosure provides affinity ligands which bind antibodies and antibody fragments which have or comprise a CH1 domain. Such affinity ligands comprise an amino acid sequence represented by the formula, from N-terminus to C-terminus,

[A]-AERWYDLGAAYAARGDX$_{17a}$DRAX$_{21a}$EX$_{23a}$YX$_{25a}$RX$_{27a}$LEX$_{30a}$DPND-AWAW-WELGX$_{9b}$AYAARGDYDRAIEYYQRALELDX$_{32b}$NN-AVAWARLGIAYAX$_{13c}$RGDYDRAIEYYQRALELDPNN-EVARX$_{5d}$ALEYARRX$_{13d}$-[B]

wherein X$_{17a}$ is F or Y; X$_{21a}$ is I or T; X$_{23a}$ is Y or F; X$_{25a}$ is R or Q; X$_{27a}$ is T or A; and X$_{30a}$ is L or H; wherein X$_{9b}$ is V or I; and X$_{32b}$ is L, P or T; wherein X$_{13c}$ is T or A; wherein X$_{5d}$ is D or G and X$_{13d}$ is A or V;

wherein [A] is present or absent, and when present comprises M, MDE, MGGGGSAAAGDE, MGDE, MGHHHHHHDE, MGLAEAAAREAAARAADE, or MAWAEFRQRLAAIRTRLEALGGSEAELAAFEREIAAFESELQ AYAGAGNPEVEALRREA AAIRDELQAYRHNDE; and wherein [B] is present or absent, and when present comprises a cysteine, RRC, RRCHHHHHH, or RRGQAGQGGGSGLNDIFEAQKIEWHECHHHHHH.

In some embodiments of the affinity ligands, X$_{17a}$ is F; X$_{21a}$ is I; X$_{23a}$ is Y; X$_{25a}$ is R; X$_{27a}$ is T; X$_{30a}$ is L; X$_{9b}$ is V; X$_{32b}$ is P; X$_{13c}$ is T; X$_{5d}$ is D; and X$_{13d}$ is V.

In some embodiments of the affinity ligands, X$_{17a}$ is Y; X$_{21a}$ is T; X$_{23a}$ is F; X$_{25a}$ is Q; X$_{27a}$ is A; X$_{30a}$ is L; X$_{9b}$ is I; X$_{32b}$ is P; X$_{13c}$ is A; X$_{5d}$ is D; and X$_{13d}$ is A.

In some embodiments of the affinity ligands, X$_{17a}$ is Y; X$_{21a}$ is I; X$_{23a}$ is Y; X$_{25a}$ is Q; X$_{27a}$ is A; X$_{30a}$ is H; X$_{9b}$ is V; X$_{32b}$ is T; X$_{13c}$ is A; X$_{5d}$ is G; and X$_{13d}$ is A.

In some embodiments of the affinity ligands, X$_{17a}$ is Y; X$_{21a}$ is I; X$_{23a}$ is Y; X$_{25a}$ is Q; X$_{27a}$ is A; X$_{30a}$ is L; X$_{9b}$ is V; X$_{32b}$ is L; X$_{13c}$ is A; X$_{5d}$ is D; and X$_{13d}$ is A.

In certain embodiments [A] is MGHHHHHHDE and [B] is RRC. In certain embodiments, [A] is MGLAEAAAREAAARAADE and [B] is RRCHHHHHH.

In some embodiments the affinity ligands comprise any of those in Table 9.

In preferred embodiments, the affinity ligands comprise any one of SEQ ID NOS: 89, 128, 129, 130, 131 or 132 ("the ligand 129 group").

In preferred embodiments, the affinity ligands comprise any one of SEQ ID NOS: 90, 133, 134, 135, 136 or 137 ("the ligand 134 group")

In preferred embodiments, the affinity ligands comprise any one of SEQ ID NOS: 139, 140, 141 or 143 ("the ligand 141 group").

In preferred embodiments, the affinity ligands comprise any one of SEQ ID NOS: 86, 91, 138, 142, 144, 145, 146, 147 or 148 ("the ligand 144 group").

In more preferred embodiments, the affinity ligands comprise any one of SEQ ID NOS: 129, 134, 141 or 144.

In some embodiments, an affinity ligand of the disclosure comprises a C-terminal cysteine or lysine.

Further aspects of the disclosure relate to multimers comprising a plurality of affinity ligands according to the disclosure. Such multimers include dimers, trimers, tetramers, pentamers, hexamers, heptamers, octamers and nonamers.

In other aspects described herein, an affinity ligand or multimer disclosed herein further comprises at least one heterologous agent operably linked to said affinity ligand or multimer to form a conjugate or a fusion protein.

Yet other aspects of the disclosure relate to separation matrices. Such separation matrices comprise at least one affinity ligand of the disclosure or at least one multimer of the disclosure. In embodiments, the ligands or multimers are coupled to a solid support, optionally via thiol linkages.

Further still, the disclosure provides methods of isolating an antibody comprising a CH1 domain, or a fragment thereof comprising a CH1 domain, which comprises contacting said antibody or said fragment with a separation matrix of the disclosure.

In some embodiments the method is directed to isolating or purifying an antibody comprising a CH1 domain, or a fragment thereof comprising a CH1 domain, which method comprises (a) contacting a separation matrix of the disclosure with a composition comprising the said antibody or said fragment, (b) washing said separation matrix with a washing buffer, (c) eluting said antibody or said fragment from the separation matrix with an elution buffer, and (d) recovering said antibody or said fragment.

In some embodiments, the method which further comprises (e) treating the separation matrix with an alkaline cleaning solution for a time sufficient to clean said matrix of residual material and to regenerate at least 80% of the antibody- or the antibody fragment-binding capacity of said separation matrix. In some embodiments, the alkaline cleaning solution comprises from about 0.1 M NaOH to about 0.5 M NaOH. For example, the separation matrix can retain at least 80% of its antibody- or the antibody fragment-binding capacity when steps (a)-(e) are repeated at least 10 times. In some embodiments, binding capacities of at least 85, 90 and 95% are retained. As such the same column can be repeatedly use for purification, up to at least 10 times or more provided the binding capacity of the resin is retained. Such separation matrices are alkaline stable and provide reduce the purification costs.

Further aspects of the disclosure related to nucleic acids or vectors encoding an affinity ligand of the disclosure or a multimer of the disclosure as well as expression vector comprising those nucleic acids or vectors having the coding region of the affinity ligand or multimer operably linked to one or more expression control elements. Additional embodiments of the disclosure are directed to host cells, particularly, *E. coli or P. pastoris* for recombinant production of an affinity ligand or multimer of the disclosure.

In some embodiments, the disclosure relates to methods of making a separation matrix comprising conjugating a ligand according to the disclosure or a multimer according to the disclosure to a solid surface.

## BRIEF DESCRIPTION OF THE DRAWINGS

**Figure 1** shows a general ribbon structure for a TPR domain having seven alpha helices as exemplified by the affinity ligands of the disclosure. The 7 alpha helices are labelled consecutively, $\alpha 1$ - $\alpha 7$, beginning at the N-terminus. The N- and C-termini are also indicated by "N" and "C", respectively.

**Figure 2** shows sensorgrams for exemplary affinity agents. Sensorgrams are for the biotinylated ligands corresponding to SEQ ID No. 89, SEQ ID No. 90, and SEQ ID No. 92 titrated with the indicated concentrations of Herceptin Fab in solution (dark blue, maroon and light blue lines). The light red lines are best fit curves.

**Figure 3** shows sensorgrams for exemplary affinity agents. Sensorgrams are for the biotinylated ligands corresponding to SEQ ID No. 89, SEQ ID No. 90, and SEQ ID No. 92 titrated with the indicated concentrations of Tremfya Fab in solution (dark blue, maroon and light blue lines). The light red lines are best fit curves.

**Figure 4** shows the adsorption isotherms of resin prepared from SEQ ID No. 129 before (■) and after (•) incubation in 0.1 M NaOH + 1 M NaCl for 24 hours.

**Figure 5** shows the adsorption isotherms of resin prepared from SEQ ID No. 134 before (■) and after (•) incubation in 0.1 M NaOH + 1 M NaCl for 24 hours.

**Figure 6** shows the adsorption isotherms of resin prepared from SEQ ID No. 144 before (■) and after (•) incubation in 0.1 M NaOH + 1 M NaCl for 24 hours.

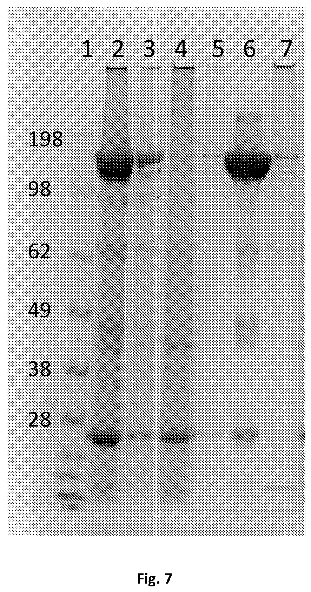

**Figure 7** shows an SDS-PAGE gel run under non-reducing conditions for various fractions obtained from a column purification run of a monoclonal antibody produced in a CHO cell line using an affinity resin with the ligand of SEQ ID No. 89. The samples are in each lane are (1) SeeBlue Plus2 molecular weight markers, (2) crude feedstream (also known as CCCF) , (3) 10x-diluted crude feedstream, (4) flow through fraction pool, (5) Wash fraction pool, (6) elution fraction pool (6.4 ug), and (7) strip fraction pool.

**Figure 8** shows the residual HCP (top panel) and residual HCDNA (bottom panel) measured across several cycles of the purification of a monoclonal antibody produced in a CHO cell line the affinity resins with the ligands of SEQ ID No. 129 (blue lines), 134 (red lines) and 144 (yellow lines).

**Figure 9** shows the yield measured across several cycles of the purification of an IgG antibody as described in Example 8.

**Figure 10** shows the ForteBio dose response of the binding of Trastuzumab and Trastuzufab protein to biotinylated ligand corresponding to SEQ ID No: 157 immobilized on sensors.

**Figure 11** shows the breakthrough curves obtained during a DBC determination of an affinity resin prepared with the ligand corresponding to SEQ ID No: 181 and packed into a 3 x 50 mm column. The breakthrough curves are shown for Trastuzumab (Upper graph) and Trastuzufab, Nucala® and Benlysta® (Lower graph) obtained at 4 minute residence time using a challenge concentration of 2 g/L.

**Figure 12** shows the stability of the DBC towards exposure with NaOH. An affinity resin prepared with the ligand corresponding to SEQ ID No: 181 was exposed to 1 M NaOH containing 1 M NaCl and the DBC was determined at the time points indicated.

**Figure 13** shows the relative yield obtained for each cycle during the purification of Trastuzumab from crude HCCF with repetitive cycling of the column. The column was exposed to 1 M NaOH containing 1 M NaCl for 3.5 hours at the end of each cycle.

**Figure 14** shows the residual HCP obtained for each cycle during the purification of Trastuzumab from crude HCCF with repetitive cycling of the column. The column was exposed to 1 M NaOH containing 1 M NaCl for 3.5 hours at the end of each cycle.

**Figure 15** shows the residual HCP obtained for each cycle during the purification of Trastuzumab from crude HCCF with repetitive cycling of the column. The column was exposed to 1 M NaOH containing 1 M NaCl for 3.5 hours at the end of each cycle.

## DETAILED DESCRIPTION OF THE DISCLOSURE

## Definitions

In order for the present disclosure to be more readily understood, certain terms are defined below. Unless defined otherwise herein, technical and scientific terms used herein have the same meaning as commonly understood by one of ordinary skill in the art to which this disclosure is related.

Units, prefixes, and symbols are denoted in their Systeme International de Unites (SI) accepted form. Numeric ranges are inclusive of the numbers defining the range. Unless otherwise indicated, amino acid sequences are written left to right in amino to carboxy orientation. The headings provided herein are not limitations of the various aspects or embodiments of the disclosure, which can be had by reference to the specification as a whole. Accordingly, the terms defined immediately below are more fully defined by reference to the specification in its entirety.

It is to be noted that the term "a" or "an" entity refers to one or more of that entity; for example, "an affinity ligand" is understood to represent one or more affinity ligands. As such, the terms "a" (or "an"), "one or more," and "at least one" can be used interchangeably herein.

Approximately or about: As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

Biologically active: As used herein, the term "biologically active" refers to a characteristic of any agent that has activity in a biological system, and particularly in an organism. For instance, an agent that, when administered to an organism, has a biological or physiological effect on that organism, is considered to be biologically active.

Variant and Mutant: The term "variant" is usually defined in the scientific literature and used herein in reference to an organism that differs genetically in some way from an accepted standard, "Variant" can also be used to describe phenotypic differences that are not genetic (King and Stansfield, 2002, A dictionary of genetics, 6th ed., New York, New York, Oxford University Press.

The term "mutation" is defined by most dictionaries and used herein in reference to the process that introduces a heritable change into the structure of a gene (King & Stansfield, 2002) thereby producing a "mutant." The term "variant" is increasingly being used in place of the term "mutation" in the scientific and non-scientific literature. The terms are used interchangeably herein.

Conservative and non-conservative substitution: A "conservative" amino acid substitution is one in which one amino acid residue is replaced with another amino acid residue having a similar side chain. Families of amino acid residues having similar side chains have been defined in the art, including basic side chains (e.g., lysine (K), arginine (R), histidine (H)); acidic side chains (e.g., aspartic acid (D), glutamic acid (E)); uncharged polar side chains (e.g., glycine (G); asparagine (N), glutamine (Q), serine (S), threonine (T), tyrosine (Y), cysteine (C)); nonpolar side chains (e.g., alanine (A), valine (V), leucine (L), isoleucine (I), proline (P), phenylalanine (F), menine (M), tryptophan (W), beta-branched side chains (e.g., threonine (T), valine (V), isoleucine (I)); and aromatic side chains (e.g., tyrosine (Y), phenylalanine (F), tryptophan (W), histidine (H)). For example, substitution of a phenylalanine for a tyrosine is a conservative substitution. In some embodiments, conservative amino acid substitutions in the sequence of a ligand confer or improve specific binding of the ligand a target of interest. In some embodiments, conservative amino acid substitutions in the sequences of a ligand do not reduce or abrogate the binding of the ligand to a target of interest. In some embodiments, conservative amino acid substitutions do not significantly affect specific binding of a ligand to a target of interest. Methods of identifying nucleotide and amino acid conservative substitutions and non-conservative substitutions which confer, alter or maintain selective binding affinity are known in the art (see, e.g., Brummell, Biochem. 32:1180-1187 (1993); Kobayashi, Protein Eng. 12(10):879-884 (1999); and Burks, PNAS 94:412-417 (1997)). In some embodiments, non-conservative amino acid substitutions in the sequence of a ligand confer or improve specific binding of the ligand a target of interest. In some embodiments, non-conservative amino acid substitutions in the sequences of a ligand do not reduce or abrogate the binding of the ligand to a target of interest. In some embodiments, non-conservative amino acid substitutions do not significantly affect specific binding of a ligand to a target of interest.

Affinity chromatography: As used herein the term "affinity chromatography" refers to the specific mode of chromatography in which an affinity ligand interacts with a target via biological affinity in a "lock-key" fashion. Examples of useful interactions in affinity chromatography are e.g., enzyme-substrate interaction, biotin-avidin interaction, antibody-antigen interaction, etc.

Affinity ligand and Ligand: The terms "affinity ligand" and "ligand" are used interchangeably herein. These terms are used herein to refer to molecules that are capable of reversibly binding with high affinity to a moiety specific for it, e.g., a polypeptide or protein.

Protein-based ligand: The term "protein-based ligands" as used herein means ligands which comprise a peptide or protein or a part of a peptide or protein that binds reversibly to a target polypeptide or protein. It is understood that the "ligands" of the disclosure are protein-based ligands.

Affinity agent: As used herein, the term "affinity agent" is in reference to a solid support or matrix to which a biospecific affinity ligand is covalently attached. Typically, the solid support or matrix is insoluble in the system in which the target molecule is purified. The terms "affinity agent" and "affinity separation matrix(ces)" and "separation matrix(ces)" are used interchangeably herein.

Linker: As used herein a "linker" refers to a peptide or other chemical linkage that functions to link otherwise independent functional domains. In some embodiments, a linker is located between a ligand and another polypeptide component containing an otherwise independent functional or structural domain. In some embodiments, a linker is a peptide or other chemical linkage located between a ligand and a surface.

Naturally occurring: The term "naturally occurring" when used in connection with biological materials such as a nucleic acid molecules, polypeptides, and host cells, refers to those which are found in nature and not modified by a human being. Conversely, "non-natural" or "synthetic" when used in connection with biological materials refers to those which are not found in nature and/or have been modified by a human being.

"Non-natural amino acids," "amino acid analogs" and "non-standard amino acid residues" are used interchangeably herein. Non-natural amino acids that can be substituted in a ligand as provided herein are known in the art. In some embodiments, a non-natural amino acid is 4-hydroxyproline which can be substituted for proline; 5-hydroxylysine which can be substituted for lysine; 3-methylhistidine which can be substituted for histidine; homoserine which can be substituted for serine; and ornithine which can be substituted for lysine. Additional examples of non-natural amino acids that can be substituted in a polypeptide ligand include, but are not limited to molecules such as: D-isomers of the common amino acids, 2,4-diaminobutyric acid, alpha-amino isobutyric acid, A-aminobutyric acid, Abu, 2-amino butyric acid, gamma-Abu, epsilon-Ahx, 6-amino hexanoic acid, Aib, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, beta-alanine, lanthionine, dehydroalanine, $\gamma$-aminobutyric acid, selenocysteine and pyrrolysine fluoro-amino acids, designer amino acids such as beta-methyl amino acids, C alpha-methyl amino acids, and N alpha-methyl amino acids.

"Polynucleotide" and "nucleic acid molecule": As used interchangeably herein, polynucleotide and nucleic acid molecule refer to a polymeric form of nucleotides of any length, either ribonucleotides or deoxyribonucleotides. These terms include, but are not limited to, DNA, RNA, cDNA (complementary DNA), mRNA (messenger RNA), rRNA (ribosomal RNA), shRNA (small hairpin RNA), snRNA (small nuclear RNA), snoRNA (short nucleolar RNA), miRNA (microRNA), genomic DNA, synthetic DNA, synthetic RNA, and/or tRNA (transfer RNA).

Operably linked: The term "operably linked," as used herein, indicates that two or more components are arranged such that the components function normally and allow the possibility that at least one of the components can mediate a function that is exerted upon at least one of the other components. Two molecules are "operably linked" whether they are attached directly or indirectly.

Peptide tag: The term "peptide tag" as used herein refers to a peptide sequence that is part of or attached (for instance through genetic engineering) to another protein, to provide a function to the resultant fusion. Such functions include but are not limited to, altering solubility of the protein, moderating expression of the protein, and facilitating attachment or interaction of the protein to another entity. Peptide tags are usually, but not always, relatively short in comparison to a protein to which they are fused. In some embodiments, a peptide tag is four or more amino acids in length, such as, 5, 6, 7, 8, 9, 10, 15, 20, or 25 or more amino acids. In some embodiments, a peptide tag as used herein, includes a second protein that can act as a "tag" (e.g., GFP) or facilitator of a particular property. In some embodiments, a ligand is a protein that contains a peptide tag. Numerous peptide tags that have uses as provided herein are known in the art. Examples of peptide tags that may be a component of a ligand fusion protein or a target bound by a ligand (e.g., a ligand fusion protein) include but are not limited to HA (hemagglutinin), c-myc, the Herpes Simplex virus glycoprotein D (gD), T7, GST, GFP, MBP, Strep-tags, His-tags, Myc-tags, TAP-tags and FLAG tag (Eastman Kodak, Rochester, N.Y.) Likewise, antibodies to the tag epitope allow detection and localization of the fusion protein in, for example, affinity purification, Western blots, ELISA assays, and immunostaining of cells.

Polypeptide: The term "polypeptide" as used herein refers to a sequential chain of amino acids linked together via peptide bonds. The term is used to refer to an amino acid chain of any length, but one of ordinary skill in the art will understand that the term is not limited to lengthy chains and can refer to a minimal chain comprising two amino acids linked together via a peptide bond. As is known to those skilled in the art, polypeptides may be processed and/or modified.

Protein: The term "protein" as used herein refers to one or more polypeptides that function as a discrete unit. If a single polypeptide is the discrete functioning unit and does not require permanent or temporary physical association with other polypeptides in order to form the discrete functioning unit, the terms "polypeptide" and "protein" may be used interchangeably. If the discrete functional unit is comprised of more than one polypeptide that physically associate with one another, the term "protein" refers to the multiple polypeptides that are physically coupled and function together as the discrete unit.

Specifically binds: As used herein in reference to ligands, the term "specifically binds" or "has selective affinity for" means a ligand reacts or associates more frequently, more rapidly, with greater duration, with greater affinity, or combinations of the above to a particular epitope, protein, or target molecule than with alternative substances, including unrelated proteins. Because of the sequence identity between homologous proteins in different species, specific binding can include a binding agent that recognizes a protein or target in more than one species, e.g., is bi- or tri-specific. Likewise, because of homology within certain regions of polypeptide sequences of different proteins, specific binding can include a binding agent that recognizes more than one protein or target. It is understood that, in certain embodiments, a binding agent that specifically binds a first target may or may not specifically bind a second target. As such, "specific binding" does not necessarily require (although it can include) exclusive binding, i.e., binding to a single target. Thus, a ligand or affinity agent may, in certain embodiments, specifically bind more than one target. In certain embodiments, multiple targets may be bound by the same binding site on an affinity agent.

Substantially: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

### CH1 Domain Affinity Ligands

The affinity ligands of the various aspects and embodiments of the disclosure are high affinity protein ligands that reversibly bind antibody and antibody fragments that contain the heavy chain first constant region (CH1) (referred to herein as a "CH1 domain"). Such binding allows these protein ligands to be used, *inter alia,* to isolate and/or purify antibodies and any antibody fragment lacking an Fc domain but retaining the CH1 region of the heavy chain.

The affinity ligands of the disclosure comprise an amino acid sequence represented by the formula, from N-terminus to C-terminus,

[A]-AERWYDLGAAYAARGDX$_{17a}$DRAX$_{21a}$EX$_{23a}$YX$_{25a}$RX$_{27a}$LEX$_{30a}$DPND-AWAW-WELGX$_{9b}$AYAARGDYDRAIEYYQRALELDX$_{32b}$NN-AVAWARLGIAYAX$_{13c}$RGDYDRAIEYYQRALELDPNN-EVARX$_{5d}$ALEYARRV-[B]

wherein X$_{17a}$ is F or Y; X$_{21a}$ is I or T; X$_{23a}$ is Y or F; X$_{25a}$ is R or Q; X$_{27a}$ is T or A; X$_{30a}$ is L or H; wherein X$_{9b}$ is V or I; and X$_{32b}$ is L, P or T;
wherein X$_{13c}$ is T or A;
wherein X$_{5d}$ is D or G and X$_{13d}$ is A or V; and
wherein [A] is present or absent, and when present comprises M, MDE, MGGGGSAAAGDE, MGDE, MGHHHHHHDE, MGLAEAAAREAAARAADE, or MAWAEFRQRLAAIRTRLEALGGSEAELAAFEREIAAFESELQ AYAGAGNPEVEALRREA AAIRDELQAYRHNDE; and [B] is present or absent, and when present comprises a cysteine, RRC, RRCHHHHHH, or RRGQAGQGGGSGLNDIFEAQKIEWHECHHHHHH. These affinity ligands bind antibody and antibody fragments that comprise a CH1 domain.

The affinity ligands of the disclosure are derived from TPR-domain containing proteins having three repeats of a 34 amino acid TPR domain followed by a seventh helix. Each repeat encodes a helix-turn-helix motif typical of those that occur in TPR-domain containing proteins. For clarity, the three 34 amino acid domains are colinear in the ligand but are presented on a single line such that the first amino acid on that line is amino acid 1 of that 34 amino acid domain. Thus, the first TPR domain (as shown above) contains helix 1 and helix 2, the second TPR domain contains helix 3 and helix 4 and the third TPR domain contains helix 5 and helix 6. In this disclosure, "a", "b" and "c" are also used to denote the three TPR domains in these affinity ligands.

In some embodiments, an affinity ligand of the disclosure has the general formula above wherein X$_{17a}$ is F; X$_{21a}$ is I; X$_{23a}$ is Y; X$_{25a}$ is R; X$_{27a}$ is T; X$_{30a}$ is L; X$_{9b}$ is V; X$_{32b}$ is P; X$_{13c}$ is T; X$_{5d}$ is D; and X$_{13d}$ is V. In some embodiment, these affinity ligands include, but are not limited to, those comprising SEQ ID NOS. 89, 128, 129, 130, 131 or 132. Such embodiments are also referred to as the Ligand 129 group.

In some embodiments, an affinity ligand of the disclosure has the general formula above wherein X$_{17a}$ is Y; X$_{21a}$ is T; X$_{23a}$ is F; X$_{25a}$ is Q; X$_{27a}$ is A; X$_{30a}$ is L; X$_{9b}$ is I; X$_{32b}$ is P; X$_{13c}$ is A; X$_{5d}$ is D; and X$_{13d}$ is A. In some embodiment, these affinity ligands include, but are not limited to, those comprising SEQ ID NOS. 90, 133, 134, 135, 136 or 137. Such embodiments are also referred to as the Ligand 134 group.

In some embodiments, an affinity ligand of the disclosure has the general formula above wherein X$_{17a}$ is Y; X$_{21a}$ is I; X$_{23a}$ is Y; X$_{25a}$ is Q; X$_{27a}$ is A; X$_{30a}$ is H; X$_{9b}$ is V; X$_{32b}$ is T; X$_{13c}$ is A; X$_{5d}$ is G; and X$_{13d}$ is A. In some embodiment, these affinity ligands include, but are not limited to, those comprising SEQ ID NOS. 139, 140, 141 or 143. Such embodiments are also referred to as the Ligand 141 group.

In some embodiments, an affinity ligand of the disclosure has the general formula above wherein $X_{17a}$ is Y; $X_{21a}$ is I; $X_{23a}$ is Y; $X_{25a}$ is Q; $X_{27a}$ is A; $X_{30a}$ is L; $X_{9b}$ is V; $X_{32b}$ is L; $X_{13c}$ is A; $X_{5d}$ is D; and $X_{13d}$ is A. In some embodiment, these affinity ligands include, but are not limited to, those comprising SEQ ID NOS. 86, 91, 138, 142 or 144-148. Such embodiments are also referred to as the Ligand 144 group.

In some embodiments of the affinity ligand, [A] is MGHHHHHHDE and [B] is RRC. In some embodiments of the affinity ligand, [A] is MGLAEAAAREAAARAADE and [B] is RRCHHHHHH.

In some embodiments, provided herein are affinity agents comprising a TPR ligand comprising 7 helices in which the 1st helix comprises the sequence SEQ ID NO: 1 $X^1RWX^2X^3$, where $X^1$ is A, D, E, G, N, Q, S or V, ; $X^2$ is A, E, W or Y; $X^3$ is D, G, I, N, T, V, W or Y;

the 3rd helix comprises the sequence SEQ ID NO: 2
$X^4AWX^5X^6LGX^7$ where $X^4$ is W; $X^5$ is E or W; $X^6$ is D, E or H; $X^7$ is T, V, W;
the 5th helix comprises the sequence SEQ ID NO: 3
$X^8AWX^9X^{10}LGX^{11}$, where $X^8$ is A, H, I, T or V; $X^9$ is A, F, N, S or R; $X^{10}$ is R; $X^{11}$ is F, I, H or W; and
the 7th helix comprises the sequence SEQ ID NO: 4
$X^{12}ARX^{13}X^{14}LEX^{15}$, where $X^{12}$ is A, L, T or V; $X^{13}$ is A, D, E, H, Q, R, S or Y; $X^{14}$ is A, E, I, L, N or Q; $X^{15}$ is A, D, E, H, N, R, S, V or Y.

In some embodiments, provided herein are affinity agents comprising a TPR ligand comprising 7 helices in which the 1st helix comprises the sequence SEQ ID NO: 1

$X^1RWX^2X^3$, where $X^1$ is D or E, Q or V; $X^2$ is E or Y; $X^3$ is D, I or N;
the 3rd helix comprises the sequence SEQ ID NO: 2
$X^4AWX^5X^6LGX^7$, where $X^4$ is W; $X^5$ is W; $X^6$ is E; $X^7$ is I or V;
the 5th helix comprises the sequence SEQ ID NO: 3
$X^8AWX^9X^{10}LGX^{11}$, where $X^8$ is H, T or V; $X^9$ is A, F or S; $X^{10}$ is R; $X^{11}$ is H, I, or W; and
the 7th helix comprises the sequence SEQ ID NO: 4
$X^{12}ARX^{13}X^{14}LEX^{15}$, where $X^{12}$ is A, L, T or V; $X^{13}$ is D, H, R, or Y: $X^{14}$ is A, I, V or L; $X^{15}$ is D, H, V or Y.

In some embodiments, provided herein are affinity agents comprising a TPR ligand comprising 7 helices in which the 1st helix comprises the sequence SEQ ID NO: 1

$X^1RWX^2X^3$, where $X^1$ is N; $X^2$ is Y; $X^3$ is V or Y;
the 3rd helix comprises the sequence SEQ ID NO: 2
$X^4AWX^5X^6LGX^7$, where $X^4$ is F, or Y; $X^5$ is D; $X^6$ is E; $X^7$ is N or S;
the 5th helix comprises the sequence SEQ ID NO: 3
$X^8AWX^9X^{10}LGX^{11}$, where $X^8$ is V; $X^9$ is N, or S; $X^{10}$ is R; $X^{11}$ is H, or Q and
the 7th helix comprises the sequence SEQ ID NO: 4
$X^{12}ARX^{13}X^{14}LEX^{15}$, where $X^{12}$ is T or V; $X^{13}$ is H or T: $X^{14}$ is L; $X^{15}$ is Q or W.

In some embodiments, provided herein are affinity agents comprising a TPR ligand comprising 7 helices in which the 1st helix comprises the sequence SEQ ID NO: 1

$X^1RWX^2X^3$, where $X^1$ is A; $X^2$ is E; $X^3$ is F or V;
the 3rd helix comprises the sequence SEQ ID NO: 2
$X^4AWX^5X^6LGX^7$, where $X^4$ is W; $X^5$ is E; $X^6$ is H; $X^7$ is W;
the 5th helix comprises the sequence SEQ ID NO: 3
$X^8AWX^9X^{10}LGX^{11}$, where $X^8$ is H; $X^9$ is F; $X^{10}$ is R; $X^{11}$ is F; and
the 7th helix comprises the sequence SEQ ID NO: 4
$X^{12}ARX^{13}X^{14}LEX^{15}$, where $X^{12}$ is L or Q; $X^{13}$ is A, or E: $X^{14}$ is E or Q; $X^{15}$ is S or H.

In some embodiments, provided herein are affinity agents comprising a TPR ligand comprising 7 helices in which the 1st helix comprises the sequence SEQ ID NO: 1

$X^1RWX^2X^3$, where $X^1$ is A, D, E, L, Q or V; $X^2$ is A, D, E, or W; $X^3$ is A, D, F, I, N, V, W or Y;
the 3rd helix comprises the sequence SEQ ID NO: 2
$X^4AWX^5X^6LGX^7$, where $X^4$ is W; $X^5$ is E or W; $X^6$ is D, E or H; $X^7$ is L, V or W;
the 5th helix comprises the sequence SEQ ID NO: 3

$X^8AWX^9X^{10}LGX^{11}$, where $X^8$ is H, R, or V; $X^9$ is F; $X^{10}$ is R; $X^{11}$ is F or W; and
the 7th helix comprises the sequence SEQ ID NO: 4
$X^{12}ARX^{13}X^{14}LEX^{15}$, where $X^{12}$ is L, or T; $X^{13}$ is A, D, E, or R; $X^{14}$ is E, I, Q, or V; $X^{15}$ is D, E, G, H, S or N.

In some embodiments, provided herein are affinity agents comprising a TPR ligand comprising 7 helices in which the 1st helix comprises the sequence SEQ ID No: 1

$X^1RWX^2X^3$, where $X^1$ is D, E or S; $X^2$ is Y; $X^3$ is D, Y or H;
The 3rd helix comprises the sequence SEQ ID No: 2
$X^4AWX^5X^6LGX^7$, where $X^4$ is W; $X^5$ is W; $X^6$ is E; $X^7$ is V, T or A;
The 5th helix comprises the sequence SEQ ID No: 3
$X^8AWX^9X^{10}LGX^{11}$, where $X^8$ is V or I; $X^9$ is A, F or S; $X^{10}$ is R; $X^{11}$ is H or W;
The 7th helix comprises the sequence SEQ ID No: 4
$X^{12}ARX^{13}X^{14}LEX^{15}$, where $X^{12}$ is A, L or V; $X^{13}$ is A, H, or S; $X^{14}$ is A, E, L or N; $X^{15}$ is A, N, V or Y.

In some embodiments, the affinity agents bind the CH1 domain of monoclonal antibody molecules and fragments.
In some embodiments provided herein are affinity agents comprising a ligand that bind to antibodies or antibody fragments comprising the sequence SEQ ID No: 153 &&&&&&$X^1RWX^2X^3$&&&&&&&&&&&&&&&&&&&&&&&&&&&&&& $X^4AWX^5X^6LG$   $X^7$&&&&&&&&&&&&&&&&&&&&&&&&&$X^8AWX^9X^{10}LGX^{11}$&&&&&&&&&&&&   &&&&&&&&&&&&&& $X^{12}ARX^{13}X^{14}LEX^{15}$, where & denotes any amino acid; $X^1$ is A, D, E, G, N, Q, S or V, ; $X^2$ is A, E, W or Y; $X^3$ is D, G, H, I, N, T, V, W or Y; $X^4$ is W; $X^5$ is E or W; $X^6$ is D, E or H; $X^7$ is T, V, W; $X^8AWX^9X^{10}LGX^{11}$, where $X^8$ is A, H, I, T or V; $X^9$ is A, F, N, S or R; $X^{10}$ is R; $X^{11}$ is F, I, H or W; $X^{12}ARX^{13}X^{14}LEX^{15}$, where $X^{12}$ is A, L, T or V; $X^{13}$ is A, D, E, H, Q, R, S or Y; $X^{14}$ is A, E, I, L, N or Q; $X^{15}$ is A, D, E, H, ,N R, S, V or Y.
In some embodiments, provided herein are affinity agents comprising a ligand that bind to antibodies or antibody fragments comprising the sequence SEQ ID NO: 5 $X^1RWX^2X^3$LGAAYAARGDYDRAIEYYQRALELDPNNA$X^4$AW$X^5X^6$LG$X^7$AYAARGDYDR AIEYYQRALELDPNNA$X^8$AW$X^9X^{10}$LG$X^{11}$AYAARGDYDRAIEYYQRALELDPNNE$X^{12}$AR$X^{13}X^{14}$LE$X^{15}$, where $X^1$ is Q, D, A, G, E, V, N; $X^2$ is E, A, Y, Q; $X^3$ is D, N, G, V, I, T, W, Y; $X^4$ is W; $X^5$ is W, E; $X^6$ is E, D, H; $X^7$ is V, W, T; $X^8$ is V, H, T, A, I; $X^9$ is F, R, S, A, N; $X^{10}$ is R; $X^{11}$ is H, W, F, I; $X^{12}$ is L, T, V; $X^{13}$ is E, Y, R, A, D, S, Q; $X^{14}$ is Q, L, E, I, A; $X^{15}$ is H, D, Y, S, E, or R.
In some embodiments, an affinity ligand of the disclosure comprises a C-terminal cysteine or lysine, and preferably a cysteine.
Another aspect of the disclosure provides multimers comprising at least two subunits (e.g., at least two affinity ligands of the disclosure). Such plurality can be a homogenous (i.,e, a single ligand of the disclosure) or heterogenous (i.e., including two or more different ligands of the disclosure). In other words, in some embodiments, provided herein are affinity agents that comprise multimer polypeptides where the sub-units are not all the same. In embodiments, a multimers can be a dimer, trimer, tetramer, pentamer, hexamer, heptamer, octamer or nonamer.
In further aspects of the disclosure, the affinity ligand or multimer hereof further comprises at least one heterologous agent operably linked to said affinity ligand to thereby form a conjugate or a fusion protein. Examples of heterologous agent include, but are not limited to, one or more small molecule diagnostic or therapeutic agents; peptides tags (as defined herein) a DNA, RNA, or hybrid DNA-RNA molecule; traceable marker; radioactive agent; an antibody; a single chain variable domain; or an immunoglobulin fragment. Such conjugates and fusion proteins can be made by methods known in the art.

**Ligand Binding to an Antibody or Antibody Fragment with a CH1 Domain**

The characteristics of a ligand or multimer that binds to a target, such as an antibody or a fragment thereof with a CH1 domain can be determined using known or modified assays, bioassays, and/or animal models known in the art for evaluating such activity.
As used herein, terms such as "binding affinity for a target," "binding to a target, "binding to an antibody or a fragment thereof with a CH1 domain," and the like refer to a property of a ligand of the disclosure which may be directly measured, for example, through the determination of affinity constants (e.g., the amount of ligand that associates and dissociates at a given antigen concentration). Several methods are available to characterize such molecular interactions, for example, competition analysis, equilibrium analysis and microcalorimetric analysis, and real-time interaction analysis based on surface plasmon resonance interaction (for example using a BIACORE instrument). These methods are well-known to those of skill in the art and are discussed in publications such as Neri D et al. (1996) Tibtech 14:465-470 and Jansson M et al. (1997) J Biol Chem 272:8189-8197.
Affinity requirements for a given ligand binding event are contingent on a variety of factors including, but not limited to the composition and complexity of the binding matrix, the valency and density of both the ligand and target molecules, and the functional application of the ligand. In some embodiments, a ligand of the disclosure binds an antibody or a fragment thereof with a CH1 domain with a dissociation constant (KD) of less than or equal to $5 \times 10^{-3}$ M, $10^{-3}$ M, $5 \times 10^{-4}$ M, $10^{-4}$ M, $5 \times 10^{-5}$ M, or $10^{-5}$ M. In some embodiments, a ligand binds a target of interest with a KD of less than or equal to $5 \times 10^{-6}$ M, $10^{-6}$ M, $5 \times 10^{-7}$ M, $10^{-7}$ M, $5 \times 10^{-8}$ M, or $10^{-8}$ M. In some embodiments, a ligand binds a target of interest with a KD less than or equal to $5 \times 10^{-9}$ M, $10^{-9}$ M, $5 \times 10^{-10}$ M, $10^{-10}$ M, $5 \times 10^{-11}$ M, $10^{-11}$ M, $5 \times 10^{-12}$ M, $10^{-12}$ M, $5 \times 10^{-13}$ M, $10^{-13}$ M, $5 \times 10^{-14}$ M, $10^{-14}$ M, $5 \times 10^{-15}$ M, or $10^{-15}$ M. In some embodiments, a ligand generated by methods disclosed herein has a dissociation constant of from about $10^{-4}$ M to about $10^{-5}$ M, from about $10^{-5}$ M to about $10^{-6}$ M, from about $10^{-6}$ M to about $10^{-7}$ M, from about $10^{-7}$ M to about $10^{-8}$ M, from about $10^{-8}$ M to about $10^{-9}$ M, from about $10^{-9}$ M to about $10^{-10}$ M, from about $10^{-10}$ M to about $10^{-11}$ M, or from about $10^{-11}$ M to about $10^{-12}$ M.

Binding experiments to determine $K_D$ and off-rates can be performed in a number of conditions. The buffers in which to make these solutions can readily be determined by one of skill in the art, and depend largely on the desired pH of the final solution. Low pH solutions (<pH 5.5) can be made, for example, in citrate buffer, glycine-HCl buffer, or in succinic acid buffer. High pH solutions can be made, for example, in Tris-HCl, phosphate buffers, or sodium bicarbonate buffers. A number of conditions may be used to determine $K_D$ and off-rates for the purpose of determining, for example, optimal pH and/or salt concentrations.

In some embodiments, a ligand or multimer of the disclosure specifically binds an antibody or a fragment thereof with a CH1 domain with a koff ranging from 0.1 to $10^{-7}$ sec$^{-1}$, $10^{-2}$ to $10^{-7}$ sec$^{-1}$, or 0.5 x $10^{-2}$ to $10^{-7}$ sec$^{-1}$. In some embodiments, a ligand binds a target of interest with an off rate ($k_{off}$) of less than 5 x$10^{-2}$ sec$^{-1}$, $10^{-2}$ sec$^{-1}$, 5 x$10^{-3}$ sec$^{-1}$, or $10^{-3}$ sec$^{-1}$. In some embodiments a ligand binds a target of interest with an off rate ($k_{off}$) of less than 5 x$10^{-4}$ sec$^{-1}$, $10^{-4}$ sec$^{-1}$, 5 x$10^{-5}$ sec$^{-1}$, or $10^{-5}$ sec$^{-1}$, 5 x$10^{-6}$ sec$^{-1}$, $10^{-6}$ sec$^{-1}$, 5 x$10^{-7}$ sec$^{-1}$, or $10^{-7}$ sec$^{-1}$.

In some embodiments, a ligand or multimer specifically binds an antibody or a fragment thereof with a CH1 domain with a $k_{on}$ ranging from about $10^3$ to $10^7$ M$^{-1}$sec$^{-1}$, $10^3$ to $10^6$ M$^{-1}$sec$^{-1}$, or $10^3$ to $10^5$ M$^{-1}$sec$^{-1}$. In some embodiments, a ligand (e.g., a ligand fusion protein) binds the target of interest with an on rate ($k_{on}$) of greater than $10^3$ M$^{-1}$sec$^{-1}$, 5 x$10^3$ M$^{-1}$sec$^{-1}$, $10^4$ M$^{-1}$sec$^{-1}$, or 5 x$10^4$ M$^{-1}$sec$^{-1}$. In an additional embodiment, a ligand, binds a target of interest with a $k_{on}$ of greater than $10^5$ M$^{-1}$sec$^{-1}$, 5 x$10^5$ M$^{-1}$sec$^{-1}$, $10^6$ M$^{-1}$ sec$^{-1}$, 5 x$10^6$ M$^{-1}$ sec$^{-1}$, or $10^7$ M$^{-1}$ sec$^{-1}$.

## Linkers

The terms "linker" and "spacer" are used interchangeably herein to refer to a peptide or other chemical linkage that functions to link otherwise independent functional domains. In some embodiments, a linker is located between a ligand and another polypeptide component containing an otherwise independent functional domain. Suitable linkers for coupling two or more linked ligands may generally be any linker used in the art to link peptides, proteins or other organic molecules. In some embodiments, such a linker is suitable for constructing proteins or polypeptides that are intended for pharmaceutical use.

Suitable linkers for operably linking a ligand and an additional component of a ligand fusion protein in a single-chain amino acid sequence include but are not limited to, polypeptide linkers such as glycine linkers, serine linkers, mixed glycine/serine linkers, glycine- and serine-rich linkers or linkers composed of largely polar polypeptide fragments.

In some embodiments, a linker comprises a majority of amino acids selected from glycine, alanine, proline, asparagine, glutamine, and lysine. In some embodiments, a linker comprises a majority of amino acids selected from glycine, alanine, proline, asparagine, aspartic acid, threonine, glutamine, and lysine. In some embodiments, a ligand linker is made up of a majority of amino acids that are sterically unhindered. In some embodiments, a linker comprises a majority of amino acids selected from glycine, serine, and/or alanine. In some embodiments, a linker is selected from polyglycines (such as (Gly)$_5$, and (Gly)s, poly(Gly-Ala), and polyalanines.

Linkers can be of any size or composition so long as they are able to operably link a ligand in a manner that permits the ligand to bind a target of interest. In some embodiments, linkers are from about 1 to 50 amino acids, from about 1 to 20 amino acids, from about 1 to 15 amino acids, from about 1 to 10 amino acids, from about 1 to 5 amino acids, from about 2 to 20 amino acids, from about 2 to 15 amino acids, from about 2 to 10 amino acids, or from about 2 to 5 amino acids. It should be clear that the length, the degree of flexibility and/or other properties of the linker(s) may influence certain properties of a ligand for use in an affinity agent, such as affinity, specificity or avidity for a target of interest, or for one or more other target proteins of interest, or for proteins not of interest (i.e., non-target proteins). In some embodiments, two or more linkers are utilized. In some embodiments, two or more linkers are the same. In some embodiments, two or more linkers are different.

In some embodiments, a linker is a non-peptide linker such as an alkyl linker, or a PEG linker. For example, alkyl linkers such as -NH-(CH$_2$)$_s$-C(O)-, wherein s = 2-20 can be used. These alkyl linkers may further be substituted by any non-sterically hindering group such as lower alkyl e.g., C$_1$ - C$_6$) lower acyl, halogen (e.g., Cl, I, Br, F), CN, NH$_2$, phenyl, etc. An exemplary non- peptide linker is a PEG linker. In some embodiments, a PEG linker has a molecular weight of from about 100 to 5000 kDa, or from about 100 to 500 kDa.

Linkers can be evaluated using techniques described herein and/or otherwise known in the art. In some embodiments, linkers do not alter (e.g., do not disrupt) the ability of a ligand to bind a target molecule.

**Affinity agents comprising conjugated ligands: Affinity separation matrices**

Ligands or multimers that promote specific binding to targets of interest can be chemically conjugated to a variety of surfaces used in chromatography, e.g., beads, resins, gels, membrane, monoliths, etc. to prepare an affinity agent. Affinity agents of the disclosure are particularly useful for an antibody or a fragment thereof with a CH1 domain purification and manufacturing applications.

In some embodiments, a ligand of the disclosure (e.g., a ligand fusion protein) contains at least one reactive residue. Reactive residues are useful, for example, as sites for the attachment of conjugates such as chemotherapeutic drugs or diagnostic agents. Exemplary reactive amino acid residues include lysine or cysteine, for example. A reactive residue can be added to a ligand at either end, or within the ligand sequence and/or can be substituted for another amino acid within the ligand sequence. A suitable reactive residue (e.g., lysine, cysteine, etc.) can also be located within the sequence of an identified ligand without need for addition or substitution.

Attachment to a solid surface

"Solid surface," "support," or "matrix" are used interchangeably herein and refer to, without limitation, any column (or column material), bead, test tube, microtiter dish, solid particle (for example, agarose or sepharose), microchip (for example, silicon, silicon-glass, or gold chip), or membrane (synthetic (e.g. a filter) or biological (e.g. liposome or vesicle) in origin to which a ligand or multimer of the disclosure may be attached (i.e., coupled, linked, or adhered), either directly or indirectly (for example, through other binding partner intermediates such as a linker), or in which a ligand or multimer may be embedded (for example, through a receptor or channel). Reagents and techniques for attaching polypeptides to solid supports are well-known in the art, e.g., carbamate coupling. Suitable solid supports include, but are not limited to, a chromatographic resin or matrix (e.g., SEPHAROSE-4 FF agarose beads), the wall or floor of a well in a plastic microtiter dish, a silica-based biochip, polyacrylamide, agarose, silica, nitrocellulose, paper, plastic, nylon, metal, and combinations thereof. Ligands and other compositions may be attached on a support material by a non-covalent association or by covalent bonding, using reagents and techniques known in the art. In some embodiments, a ligand is coupled to a chromatography material using a linker.

In one aspect, the disclosure provides an affinity agent (affinity separation matrix) comprised of a ligand or multimer as described above coupled to an insoluble support. Such a support may be one or more particles, such as beads; membranes; filters; capillaries; monoliths; and any other format commonly used in chromatography. In an advantageous embodiment of the affinity separation matrix, the support is comprised of substantially spherical particles, also known as beads. Suitable particle sizes may be in the diameter range of 5-500 $\mu$m, such as 10-100 $\mu$m, e.g., 20-80 $\mu$m. In an alternative embodiment, the support is a membrane. To obtain high adsorption capacities, the support is preferably porous, and ligands may be coupled to the external surfaces as well as to the pore surfaces. In an advantageous embodiment of this aspect, the support is porous.

In another aspect, the disclosure relates to a method of preparing a chromatography affinity agent, which method comprises providing ligands as described above, and coupling the ligands to a support. Coupling may be carried out via a nitrogen or sulfur atom of the ligand for example. The ligands may be coupled to the support directly or indirectly via a spacer element to provide an appropriate distance between the support surface and the ligand. Methods for immobilization of protein ligands to porous or non-porous surfaces are well known in this field.

**Production of ligands**

The production of ligands and multimers, useful in practicing several embodiments of the disclosure, may be carried out using a variety of standard techniques for chemical synthesis, semi-synthetic methods, and recombinant DNA methodologies known in the art. Also provided are methods for producing a ligand or multimer, individually or as part of multi-domain fusion protein, as soluble agents and cell associated proteins. In some embodiments, the overall production scheme for a ligand or multimer comprises obtaining a reference protein scaffold and identifying a plurality of residues within the scaffold for modification. Depending on the embodiment, the reference scaffold may comprise a protein structure with one or more alpha-helical regions, or other tertiary structure. Once identified, any of a plurality of residues can be modified, for example by substitution of one or more amino acids. In some embodiments, one or more conservative substitutions are made. In some embodiments, one or more non-conservative substitutions are made. In some embodiments a natural amino acid (e.g., one of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, or valine) is substituted into a reference scaffold at targeted positions for modification. In some embodiments, modifications do not include substituting in either a cysteine or a proline. After modifications have been made at identified positions desired in a particular embodiment, the resulting modified polypeptides (e.g., candidate ligands) can be recombinantly expressed, for example in a plasmid, bacteria, phage, or other vector (e.g., to increase the number of each of the modified polypeptides). The modified polypeptides can then be purified and screened to identify those modified polypeptides that have specific binding to a particular target of interest, e.g., an antibody or a fragment thereof with a CH1 domain. Modified polypeptides may show enhanced binding specificity for an antibody or a fragment thereof with a CH1 domain as compared to a reference scaffold, or may exhibit little or no binding to a given target of interest (or to a non-target protein). In some embodiments, depending on the target of interest, the reference scaffold may show some interaction (e.g., nonspecific interaction) with the target of interest, while certain modified polypeptides will exhibit at least about two-fold, at least about five-fold, at least about tenfold, at least about 20- fold, at least about 50-fold, or at least about 100-fold (or more) increased binding specificity for the target of interest. Additional details regarding production, selection, and isolation of ligand are provided in more detail below.

Recombinant expression of **ligands**

In some embodiments, a ligand such as a ligand fusion protein is "recombinantly produced," (i.e., produced using recombinant DNA technology). Exemplary recombinant methods available for synthesizing ligand fusion proteins, include, but are not limited to polymerase chain reaction (PCR) based synthesis, concatemerization, seamless cloning, and recursive directional ligation (RDL) (see, e.g., Meyer et al., Biomacromolecules 3:357-367 (2002), Kurihara et al., Biotechnol. Lett. 27:665-670 (2005), Haider et al., Mol. Pharm. 2:139-150 (2005); and McMillan et al., Macromolecules 32(11):3643-3646 (1999).

In another aspect, nucleic acids comprising a polynucleotide sequence encoding a ligand or multimer according to the embodiments disclosed above are also provided. Thus, the disclosure encompasses all forms of the present nucleic acid sequence such as RNA and DNA encoding the polypeptide (ligand) or multimer. The disclosure provides vectors, such as plasmids, which in addition to the coding sequence comprise the required signal sequences for expression of the polypeptide or multimer according the disclosure. Such polynucleotides optionally further comprise one or more expression control elements. For example, a polynucleotide can comprise one or more promoters or transcriptional enhancers, ribosomal binding sites, transcription termination signals, and polyadenylation signals, as expression control elements. A polynucleotide can be inserted within any suitable vector, which can be contained within any suitable host cell for expression. In one embodiment, the vector comprises nucleic acid encoding a multimer according to the disclosure, wherein the separate nucleic acids encoding each unit may have homologous or heterologous DNA sequences.

The expression of nucleic acids encoding ligands and multimers is typically achieved by operably linking a nucleic acid encoding the ligand to a promoter in an expression vector. Typical expression vectors contain transcription and translation terminators, initiation sequences, and promoters useful for regulation of the expression of the desired nucleic acid sequence. Exemplary promoters useful for expression in E. coli include, for example, the T7 promoter.

Methods known in the art can be used to construct expression vectors containing the nucleic acid sequence encoding a ligand along with appropriate transcriptional/ translational control signals. These methods include, but are not limited to in vitro recombinant DNA techniques, synthetic techniques and *in vivo* recombination/genetic recombination. The expression of the polynucleotide can be performed in any suitable expression host known in the art including, but not limited to, bacterial cells, yeast cells, insect cells, plant cells or mammalian cells. In some embodiments, a nucleic acid sequence encoding a ligand is operably linked to a suitable promoter sequence such that the nucleic acid sequence is transcribed and/or translated into ligand in a host.

A variety of host-expression vector systems can be utilized to express a nucleic acid encoding a ligand. Vectors containing the nucleic acids encoding a ligand (e.g., individual ligand subunits or ligand fusions) or portions or fragments thereof, include plasmid vectors, a single and double-stranded phage vectors, as well as single and double-stranded RNA or DNA viral vectors. Phage and viral vectors may also be introduced into host cells in the form of packaged or encapsulated virus using known techniques for infection and transduction. Moreover, viral vectors may be replication competent or alternatively, replication defective. Alternatively, cell-free translation systems may also be used to produce the protein using RNAs derived from the DNA expression constructs (see, e.g., WO86/05807 and WO89/01036; and U.S. Pat. No. 5,122,464).

Generally, any type of cell or cultured cell line can be used to express a ligand or multimer provided herein. In some embodiments a background cell line used to generate an engineered host cell is a phage, a bacterial cell, a yeast cell or a mammalian cell. A variety of host-expression vector systems may be used to express the coding sequence a ligand fusion protein. Mammalian cells can be used as host cell systems transfected with recombinant plasmid DNA or cosmid DNA expression vectors containing the coding sequence of the target of interest and the coding sequence of the fusion polypeptide. The cells can be primary isolates from organisms, cultures, or cell lines of transformed or transgenic nature.

Suitable host cells include but are not limited to microorganisms such as, bacteria (e.g., E. coli, B. subtilis) transformed with recombinant bacteriophage DNA, plasmid DNA or cosmid DNA expression vectors containing ligand coding sequences; yeast (e.g., Saccharomyces, Pichia) transformed with recombinant yeast expression vectors containing ligand coding sequences; insect cell systems infected with recombinant virus expression vectors (e.g., Baculovirus) containing ligand coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing ligand coding sequences.

Prokaryotes useful as host cells in producing a ligand include gram negative or gram positive organisms such as, E. coli and B. subtilis. Expression vectors for use in prokaryotic host cells generally contain one or more phenotypic selectable marker genes (e.g., genes encoding proteins that confer antibiotic resistance or that supply an autotrophic requirement). Examples of useful prokaryotic host expression vectors include the pKK223-3 (Pharmacia, Uppsala, Sweden), pGEMI (Promega, Wis., USA), pET (Novagen, Wis., USA) and pRSET (Invitrogen, Calif., USA) series of vectors (see, e.g., Studier, J. Mol. Biol. 219:37 (1991) and Schoepfer, Gene 124:83 (1993)). Exemplary promoter sequences frequently used in prokaryotic host cell expression vectors include T7, (Rosenberg et al., Gene 56:125-135 (1987)), beta-lactamase (penicillinase), lactose promoter system (Chang et al., Nature 275:615 (1978)); and Goeddel et al., Nature 281 :544 (1979)), tryptophan (trp) promoter system (Goeddel et al., Nucl. Acids Res. 8:4057, (1980)), and tac promoter (Sambrook et al., 1990, Molecular Cloning, A Laboratory Manual, 2d Ed., Cold Spring Harbor Laboratory, Cold Spring Harbor, N.Y.).

In some embodiments, a eukaryotic host cell system is used, including yeast cells transformed with recombinant yeast expression vectors containing the coding sequence of a ligand. Exemplary yeast that can be used to produce compositions of the disclosure, include yeast from the genus Saccharomyces, Pichia, Actinomycetes and Kluyveromyces. Yeast vectors typically contain an origin of replication sequence from a 2mu yeast plasmid, an autonomously replicating sequence (ARS), a promoter region, sequences for polyadenylation, sequences for transcription termination, and a selectable marker gene. Examples of promoter sequences in yeast expression constructs include promoters from metallothionein, 3-phosphoglycerate kinase (Hitzeman, J. Biol. Chem. 255:2073 (1980)) and other glycolytic enzymes, such as, enolase, glyceraldehyde-3-phosphate dehydrogenase, hexokinase, pyruvate decarboxylase, phosphofructokinase, glucose-6-phosphate isomerase, 3-phospho glycerate mutase, pyruvate kinase, triosephosphate isomerase, phosphoglucose isomerase, and glucokinase. Additional suitable vectors and promoters for use in yeast expression as well as yeast transformation protocols are known in the art. See, e.g., Fleer, Gene 107:285-195 (1991) and Hinnen, PNAS 75:1929 (1978).

Insect and plant host cell culture systems are also useful for producing the compositions of the disclosure. Such host cell systems include for example, insect cell systems infected with recombinant virus expression vectors (e.g., baculovirus) containing the coding sequence of a ligand; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing the coding sequence of a ligand, including, but not limited to, the expression systems taught in U.S. Pat. No. 6,815,184; U.S. Publ. Nos. 60/365,769, and 60/368,047; and WO2004/057002, WO2004/024927, and WO2003/078614.

In some embodiments, host cell systems may be used, including animal cell systems infected with recombinant virus expression vectors (e.g., adenoviruses, retroviruses, adeno-associated viruses, herpes viruses, lentiviruses) including cell lines engineered to contain multiple copies of the DNA encoding a ligand either stably amplified (CHO/dhfr) or unstably amplified in double-minute chromosomes (e.g., murine cell lines). In some embodiments, a vector comprising a polynucleotide(s) encoding a ligand is polycistronic. Exemplary mammalian cells useful for producing these compositions include 293 cells (e.g., 293T and 293F), CHO cells, BHK cells, NS0 cells, SP2/0 cells, YO myeloma cells, P3X63 mouse myeloma cells, PER cells, PER.C6 (Crucell, Netherlands) cells VERY, Hela cells, COS cells, MDCK cells, 3T3 cells, W138 cells, BT483 cells, Hs578T cells, HTB2 cells, BT20 cells, T47D cells, CRL7O30 cells, HsS78Bst cells, hybridoma cells, and other mammalian cells. Additional exemplary mammalian host cells that are useful in practicing the embodiments of the disclosure include but are not limited, to T cells. Exemplary expression systems and selection methods are known in the art and, including those described in the following references and references cited therein: Borth et al., Biotechnol. Bioen. 71(4):266-73 (2000), in Werner et al., Arzneimittelforschung/Drug Res. 48(8):870-80 (1998), Andersen et al., Curr. Op. Biotechnol. 13:117-123 (2002), Chadd et al., Curr. Op, Biotechnol. 12:188-194 (2001), and Giddings, Curr. Op. Biotechnol. 12:450-454 (2001). Additional examples of expression systems and selection methods are described in Logan et al., PNAS 81:355-359 (1984), Birtner et al. Methods Enzymol. 153:51-544 (1987)). Transcriptional and translational control sequences for mammalian host cell expression vectors are frequently derived from viral genomes. Commonly used promoter sequences and enhancer sequences in mammalian expression vectors include, sequences derived from Polyoma virus, Adenovirus 2, Simian Virus 40 (SV40), and human cytomegalovirus (CMV). Exemplary commercially available expression vectors for use in mammalian host cells include pCEP4 (Invitrogen) and pcDNA3 (Invitrogen).

Physical methods for introducing a nucleic acid into a host cell (e.g., a mammalian host cell) include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York).

Biological methods for introducing a polynucleotide of interest into a host cell include the use of DNA and RNA vectors. Viral vectors, and especially retroviral vectors, have become the most widely used method for inserting genes into mammalian (e.g., human) cells. Other viral vectors can be derived from lentivirus, poxviruses, herpes simplex virus I, adenoviruses and adeno-associated viruses, and the like. See, for example, U.S. Pat, Nos. 5,350,674 and 5,585,362.

Methods for introducing a DNA and RNA polynucleotides of interest into a host cell include electroporation of cells, in which an electrical field is applied to cells in order to increase the permeability of the cell membrane, allowing chemicals, drugs, or polynucleotides to be introduced into the cell. Ligand containing DNA or RNA constructs may be introduced into mammalian or prokaryotic cells using electroporation.

In some embodiments, electroporation of cells results in the expression of a ligand-CAR on the surface of T cells, NK cells, NKT cells. Such expression may be transient or stable over the life of the cell. Electroporation may be accomplished with methods known in the art including MaxCyte GT® and STX® Transfection Systems (MaxCyte, Gaithersburg, MD, USA).

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle). In the case where a non-viral delivery system is utilized, an exemplary delivery vehicle is a liposome. The use of lipid formulations is contemplated for the introduction of the nucleic acids into a host cell (in vitro, ex vivo or in vivo). In some embodiments, the nucleic acid is associated with a lipid. A nucleic acid associated with a lipid can be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they can be present in a bilayer structure, as micelles, or with a "collapsed" structure. They can also simply be interspersed in a solution, possibly forming aggregates that are not uniform in size or shape. Lipids are fatty substances which can be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristoyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristoyl phosphatidylglycerol ("DMPG") and other lipids may be obtained from Avanti Polar Lipids, Inc. (Birmingham, AL). Stock solutions of lipids in chloroform or chloroform/methanol can be stored at about -20°C. Chloroform may be used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., Glycobiology 5:505-510 (1991)). However, compositions that have different structures in solution than the normal vesicular structure are also encompassed. For example, the lipids can assume a micellar structure or merely exist as non-uniform aggregates of lipid molecules. Also contemplated are lipofectamine-nucleic acid complexes.

Regardless of the method used to introduce exogenous nucleic acids into a host cell, the presence of the recombinant nucleic acid sequence in the host cell can routinely be confirmed through a variety of assays known in the art. Such assays include, for example, "molecular biological" assays, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the disclosure.

Reporter genes are used for identifying potentially transfected cells and for evaluating the functionality of regulatory sequences. In general, a reporter gene is a gene that is not present in or expressed by the recipient organism, tissue, or cell and that encodes a polypeptide whose expression is manifested by some easily detectable property, e.g., enzymatic activity. Expression of the reporter gene is assayed at a suitable time after the DNA has been introduced into the recipient cells. Suitable reporter genes include, but are not limited to, genes encoding luciferase, beta-galactosidase, chloramphenicol acetyl transferase, secreted alkaline phosphatase, or the green fluorescent protein gene (e.g., Ui-Tei et al., FEBS Lett. 479:79-82 (2000)). Suitable expression systems are known in the art and can be prepared using known techniques or obtained commercially. In general, the construct with the minimal 5' flanking region showing the highest level of expression of reporter gene is identified as the promoter. Such promoter regions can routinely be linked to a reporter gene and used to evaluate agents for the ability to modulate promoter-driven transcription.

A number of selection systems can be used in mammalian host-vector expression systems, including, but not limited to, the herpes simplex virus thymidine kinase, hypoxanthine-guanine phosphoribosyltransferase and adenine phosphoribosyltransferase (Lowy et al., Cell 22:817 (1980)) genes. Additionally, antimetabolite resistance can be used as the basis of selection for e.g., dhfr, gpt, neo, hygro, trpB, hisD, ODC (ornithine decarboxylase), and the glutamine synthase system.

In some embodiments, the initiator N-terminal methionine is included at the NH-terminus of the ligand. In many instances the ligand is isolated without the N-terminal methionine residue, which is presumed to be cleaved during expression. In many instances a mixture is obtained with only a proportion of the purified ligand contains the N-terminal methionine. It is obvious to those skilled in the art that the presence or absence of the N-terminal methionine does not affect the functionality of the ligands and affinity agents described herein.

## Ligand purification

Once a ligand or a ligand fusion protein or multimer has been produced by recombinant expression, it can be purified by methods known in the art for purification of a recombinant protein, for example, by chromatography (e.g., ion exchange, affinity, and sizing column chromatography), centrifugation, differential solubility, or by any other standard technique for the purification of proteins. In some embodiments, a ligand is optionally fused to heterologous polypeptide sequences specifically disclosed herein or otherwise known in the art to facilitate purification. In some embodiments, ligands (e.g., antibodies and other affinity matrices) for ligand affinity columns for affinity purification and that optionally, the ligand or other components of the ligand fusion composition that are bound by these ligands are removed from the composition prior to final preparation of the ligand using techniques known in the art.

## Chemical synthesis of ligand

In addition to recombinant methods, ligand production may also be carried out using organic chemical synthesis of the desired polypeptide using a variety of liquid and solid phase chemical processes known in the art. Various automatic synthesizers are commercially available and can be used in accordance with known protocols. See, for example, Tam et al., J. Am. Chem. Soc., 105:6442 (1983); Merrifield, Science, 232:341-347 (1986); Barany and Merrifield, The Peptides, Gross and Meienhofer, eds, Academic Press, New York, 1- 284; Barany et al., Int. J. Pep. Protein Res., 30:705 739 (1987); Kelley et al. in Genetic Engineering Principles and Methods, Setlow, J. K., ed. Plenum Press, NY. 1990, vol. 12, pp. 1-19; Stewart et al., Solid-Phase Peptide Synthesis, W.H. Freeman Co., San Francisco, 1989. One advantage of these methodologies is that they allow for the incorporation of non-natural amino acid residues into the sequence of the ligand.

The ligands and multimers that are used in the methods of the disclosure may be modified during or after synthesis or translation, e.g., by glycosylation, acetylation, benzylation, phosphorylation, amidation, pegylation, formylation, derivatization by known protecting/blocking groups, proteolytic cleavage, linkage to an antibody molecule, hydroxylation, iodination, methylation, myristoylation, oxidation, pegylation, proteolytic processing, phosphorylation, prenylation, racemization, selenoylation, sulfation, ubiquitination, etc. (See, e.g., Creighton, Proteins: Structures and Molecular Properties, 2d Ed. (W.H. Freeman and Co., N.Y., 1992); Postranslational Covalent Modification of Proteins, Johnson, ed. (Academic Press, New York, 1983), pp. 1-12; Seifter, Meth. Enzymol., 182:626-646 (1990); Rattan, Ann. NY Acad. Sci., 663:48-62 (1992).) In some embodiments, the peptides are acetylated at the N-terminus and/or amidated at the C-terminus.

Any of numerous chemical modifications may be carried out by known techniques, including, but not limited to acetylation, formylation, etc. Additionally, derivatives may contain one or more non-classical amino acids.

In some embodiments, cyclization, or macrocyclization of the peptide backbone is achieved by sidechain-to-sidechain linkage formation. Methods for achieving this are well known in the art and may involve natural as well as unnatural amino acids. Approaches includes disulfide formation, lanthionine formation or thiol alkylations (e.g. Michael addition), amidation between amino and carboxylate sidechains, click chemistry (e.g. azide - alkyne condensation), peptide stapling, ring closing metathesis and the use of enzymes.

## Affinity agents for purification

In purification based on affinity chromatography, a target of interest (e.g. protein or molecule) is selectively isolated according to its ability to specifically and reversibly bind to a ligand that has typically been covalently coupled to a chromatographic matrix. The affinity ligands of the disclosure can be used as reagents for affinity purification of an antibody or a fragment thereof with a CH1 domain from clarified cell culture fluids (CCCF), or natural sources such as biological samples (e.g.,serum), for example, from synthetic or recombinant production of such antibody fragments, or classic enzyme digestion of whole antibodies.

In some embodiments, a ligand or multimer that specifically binds an antibody or a fragment thereof with a CH1 domain is immobilized on beads, such as agarose beads, to form an affinity separation matrix, and then used to affinity purify the target.

Methods of covalently coupling proteins to a surface are known by those of skill in the art, and peptide tags that can be used to attach a ligand to a solid surface are known to those of skill in the art. Further, ligands can be attached (i.e., coupled, linked, or adhered) to a solid surface using any reagents or techniques known in the art. In some embodiments, a solid support comprises beads, glass, slides, chips and/or gelatin. Thus, a series of ligands can be used to make an array on a solid surface using techniques known in the art. For example, U.S. Publ. No. 2004/0009530 discloses methods for preparing arrays.

In some embodiments, a ligand or multimer is used to isolate an antibody or a fragment thereof with a CH1 domain by affinity chromatography. In some embodiments, a ligand or multimer is immobilized on a solid support. The ligand or multimer can be immobilized on the solid support using techniques and reagents described herein or otherwise known in the art. Suitable solid supports are described herein or otherwise known in the art and in specific embodiments are suitable for packing a chromatography column. The affinity agent can be packed in columns of various sizes and operated at various linear velocities or immobilized affinity ligand can be contacted with a solution under conditions favorable to form a complex between the ligand and an antibody or a fragment thereof with a CH1 domain. Non-binding materials can be washed away. Suitable wash conditions can readily be determined by one of skill in the art. Examples of suitable wash conditions are described in Shukla and Hinckley, Biotechnol Prog. 2008 Sep-Oct;24(5):1115-21. doi: 10.1002/btpr.50.

In some embodiments, chromatography is carried out by mixing a solution containing the target of interest and the ligand, then isolating complexes of the target of interest and ligand, e.g., an antibody or a fragment thereof with a CH1 domain and ligand. For example, a ligand or multimer is immobilized on a solid support such as beads, then separated from a solution along with the an antibody or a fragment thereof with a CH1 domain by filtration. In some embodiments, the ligand or multimer is a fusion protein that contains a peptide tag, such as a poly-His tail or streptavidin binding region, which can be used to isolate the ligand or multimer after complexes have formed using an immobilized metal affinity chromatographic resin or streptavidin-coated substrate. Once separated, the an antibody or a fragment thereof with a CH1 domain can be released from the ligand or multimer under elution conditions and recovered in a purified form.

In some embodiments, a ligand or multimer of the disclosure is coupled to a highly cross-linked agarose base matrix which is useful for bioprocess applications. Attachment of the ligand or multimer to the base matrix may be through a flexible spacer that ensures ligand accessibility and subsequently leads to high binding capacities. The affinity of the ligands and multimers of the disclosure ensures highly specific binding of an antibody or a fragment thereof with a CH1 domain at near neutral pH (pH 6-9), while enabling elution at pH as high as 4.5. Moreover, the ligands of the disclosure are designed for enhanced alkali stability, enabling the repeated use of from 0.1 M to 0.5 M NaOH in cleaning-in-place (CIP) and sanitization applications.

In another aspect, the disclosure provides, a method of isolating an antibody or a fragment thereof with a CH1 domain, wherein a separation matrix as disclosed above is used. In certain embodiments, the method comprises the steps of (a) contacting a liquid sample comprising an antibody or a fragment thereof with a CH1 domain with a separation matrix as disclosed above, (b) washing the separation matrix with a washing liquid, (c) eluting the an antibody or a fragment thereof with a CH1 domain from the separation matrix with an elution liquid, and (d) cleaning the separation matrix with a cleaning liquid, which can alternatively be called a cleaning-in-place (CIP) liquid, e.g. with a contact (incubation) time of at least one minute, e.g., for one to four minutes or more.

Suitable compositions of the liquid sample, the washing liquid and the elution liquid, as well as the general conditions for performing the separation are well known in the art of affinity chromatography. A liquid sample comprising an antibody or a fragment thereof with a CH1 domain may comprise host cell proteins (HCP), such as HEK293T cells for example. The host cell proteins may be desorbed during step (b).

Binding of an antibody or a fragment thereof with a CH1 domain has been demonstrated with buffers at near-neutral pH (6-9) over a wide range of ionic strength (e.g., 100-400 mM NaCl). Conventional buffers, e.g., phosphate, citrate, acetate, Tris, may be used for equilibration and loading.

In some embodiments, a solution or sample containing an antibody or a fragment thereof with a CH1 domain is concentrated, for example by ultrafiltration, prior to contacting the solution with the separation matrix. For example, the antibody or antibody fragment-containing solution, e.g., a clarified cell culture feed, may be concentrated up to 20-fold. Concentration of the antibody or antibody fragments reduces the load time for affinity chromatography. Increase in concentration may also have a positive effect on the binding capacity due to thermodynamic equilibrium effects, which may lead to a lower volume of separation matrix needed for purification. Concentrating the feed of antibody or antibody fragment solution can also lead to a significant gain in the processing time.

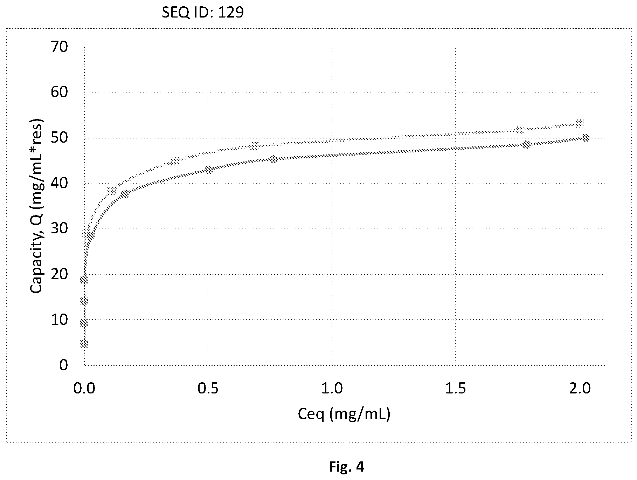

Alternatively, the solution or sample containing an antibody or a fragment thereof with a CH1 domain is a non-concentrated or diluted solution, e.g., a clarified cell culture feed (CCCF). As shown in Figure 4, the affinity separation matrix of the disclosure is characterized by an ability to process CCCF at high volumetric flow rates, enabling capture from dilute CCCF feed streams.

Elution of antibodies or fragments thereof is generally achieved by lowering the pH, e.g., 2.0-3.0, although higher pH may be used. Optimal conditions for elution of an antibody or a fragment thereof with a CH1 domain can be readily determined by those of skill in this field.

The affinity agents of the disclosure are alkali-tolerant, enabling the use of NaOH up to concentrations of 0.5 M for cleaning. In certain embodiments, a CIP regimen of 0.5 M NaOH exposure for up to 30 to 60 minutes per cycle, for example, ensures consistent chromatographic performance for several cycles, e.g., 15-30 cycles, including up to 70% - 90% of the initial binding capacity and low residual DNA and HCP levels, as well as substantially no change in flow capacity.

## EXAMPLES

The examples presented herein represent certain embodiments of the present invention. However, it is to be understood that these examples are for illustration purposes only and do not intend, nor should any be construed, to be wholly definitive as to conditions and scope of this invention. The examples were carried out using standard techniques, which are well known and routine to those of skill in the art, except where otherwise described in detail.

### EXAMPLE 1. Methods

Peptides are synthesized by standard Fmoc solid phase peptide synthesis techniques and purified by preparative reverse phase HPLC. The purity of peptides is assessed by RP-UPLC with both UV and quadrupole time-of-flight mass spectrometric detection.

Recombinant affinity ligands are expressed in E. coli or Pichia pastoris using standard techniques. Ligands can be purified using multi-column chromatography. For His-tagged ligands, immobilized metal affinity chromatography (IMAC) is used as the primary capture step. Biotinylated ligands are generated with the AviTag™ system (Avidity, Aurora, CO). Non-biotinylated ligands bearing the AviTag™ sequence are prepared by omitting exogenous biotin. The purity and identity of recombinant protein ligands is assessed by a combination of SDS-PAGE, RP UPLC, quadrupole time-of-flight mass spectrometry and size exclusion chromatography (SEC); Sephadex S75, Cytiva, Marlborough, MA). In many instances the ligand is isolated without the N-terminal methionine residue, which is presumed to be cleaved during expression.

### EXAMPLE 2. Exemplary CH1 domain Affinity Ligands

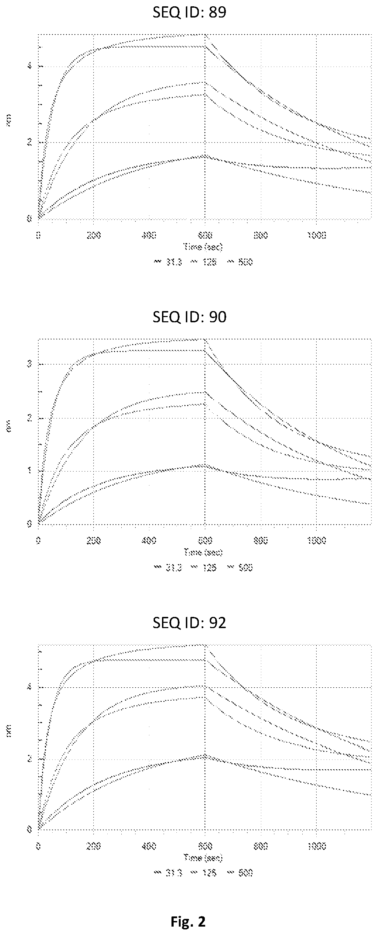

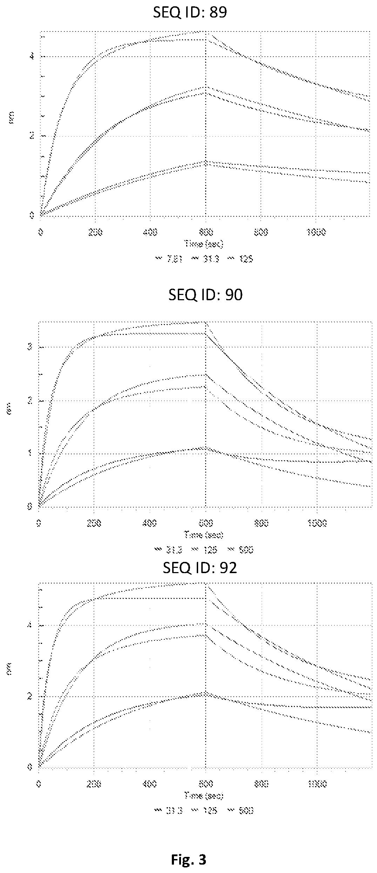

This example demonstrates the binding of biotinylated ligands to Fab fragments or IgG having a CH1 domain using biolayer interferometry (ForteBio, Menlo park, CA). Biotinylated affinity ligands were immobilized on sensors and incubated with solutions containing the Fab fragments or IgG at various concentrations. To demonstrate that the binding site does not occur on either light chain or on a paratope (antigen-binding site), two Fabs with different light chains and paratopes were interrogated for binding. Fig. 2 and Fig. 3 show the sensorgram plots for the binding of affinity ligands 89, 90 and 92 to (a) Herceptin Fab with a kappa light chain ($\kappa$) and binding specificity for Her2/neu (from the monoclonal antibody trastuzumab) or (b) Tremfya Fab with a lambda light chain ($\lambda$) and binding specificity for IL23 (from the monoclonal antibody guselkumab), respectively.

The binding curves were fit with a 1:1 binding model using the ForteBio software to determine the binding affinities and the resulting data is shown in Table 1. As expected, the affinity ligands agents bound to both Fabs with differing light chains, at binding affinity generally less than 100 nM and in many cases less than 10 nM. Similar experiments Binding to the dimeric human IgG-lambda (Southern Biotech, Birmingham AL) had the highest affinity of all reagents tested.

**Table 1**

| Affinity ($K_D$) of selected Ligands for Fabs and Antibodies | | | |
|---|---|---|---|
| Ligand (SEQ ID NO.) | Tremfya Fab $K_D$ (nM) | Herceptin Fab $K_D$ (nM) | IgG $K_D$ (nM) |
| 79 | 28 | 67 | < 1 |
| 80 | 4 | 7 | < 1 |
| 81 | 8 | 31 | < 1 |
| 82 | 6 | 23 | < 1 |
| 83 | 8 | 22 | < 1 |
| 84 | 18 | 32 | < 1 |
| 85 | 26 | 53 | < 1 |
| 86 | 8 | 28 | < 1 |

(continued)

| Affinity ($K_D$) of selected Ligands for Fabs and Antibodies | | | |
|---|---|---|---|
| Ligand (SEQ ID NO.) | Tremfya Fab $K_D$ (nM) | Herceptin Fab $K_D$ (nM) | IgG $K_D$ (nM) |
| 87 | 10 | 84 | < 1 |
| 88 | 10 | 32 | < 1 |
| 89 | 8 | 41 | < 1 |
| 90 | 12 | 51 | < 1 |
| 91 | 11 | 46 | < 1 |
| 92 | 8 | 28 | < 1 |
| 93 | 4 | 15 | < 1 |
| 94 | 3 | 14 | < 1 |

**EXAMPLE 3. Alkaline Stability of Affinity Ligands**

This example demonstrates the sodium hydroxide stability of the affinity ligands. The indicated affinity ligands were incubated in 0.1 M NaOH for 24 hours and then neutralized. The binding of the NaOH treated ligands was measured as described in Example 2 and compared to untreated ligand. The binding retained was calculated according to the following formula:

% binding retained = (measured response after NaOH treatment) ÷ (measured response of untreated) × 100

The data in Table 2 indicate that many of the affinity ligands tested exhibit high stability under tested conditions.

**Table 2**

| Percentage (%) binding retained under Alkaline Conditions | |
|---|---|
| Ligand (SEQ ID NO.) | % Binding retained |
| 79 | 63 |
| 80 | 66 |
| 81 | 69 |
| 82 | 59 |
| 83 | 55 |
| 84 | 100 |
| 85 | 64 |
| 86 | 56 |
| 87 | 2 |
| 88 | 32 |
| 89 | 53 |
| 90 | 54 |
| 91 | 68 |
| 92 | 53 |
| 93 | 61 |
| 94 | 100 |

## EXAMPLE 4. Construction of Exemplary Affinity Resins

This example demonstrates the production and characterization of affinity agents comprising ligands identified and described herein. Affinity resins were prepared by conjugating ligands to either bromoacetyl or epoxy activated activated Praesto® Jetted A50 beads (Purolite, King of Prussia, PA) or ABT700 (Agarose Bead Technologies, Madrid, Spain) agarose beads.

Bromoacetyl activated beads were activated with disuccinimidyl carbonate and coupled with excess ethylenediamine, followed by with bromoacetate washing to functionalize the free amine. The affinity ligand was conjugated at room temperature to the bromoacetyl-activated beads via it's carboxy terminal cysteine using EDC activation (EDC is 1-ethyl-3-(3-dimethylaminopropyl)carbodiimide hydrochloride). Epoxy activated beads were either obtained from the vendor or prepared using standard methods well known in the art. Ligands were coupled to epoxy activated beads at 37 - 40 °C. Targeted ligand densities varied from 5 - 40 g/L. After washing, the beads were deactivated with excess thioglycerol. The actual ligand density for was measured using a subtractive RP-HPLC method according to the following formula:

$$\text{Actual Ligand Density} = (\text{Measured [ligand] in feed} - \text{Measured [ligand] in effluent}).$$

## EXAMPLE 5. Alkaline Stability of Affinity Resins

This example demonstrates the binding characteristics and sodium hydroxide stability of affinity chromatography resins prepared from affinity ligands described herein. Briefly, 10 µL of the affinity resin was aliquoted into replicate wells on a 96-well filter plate and incubated in a neutral pH buffer or in 0.1 M NaOH with 1 M NaCl for 24 hours. After incubation, the affinity resins were extensively washed with a neutral pH buffer and interrogated for binding in a batch format. As outlined in Table 3, following equilibration with PBS, the resins were challenged with IgG at concentrations ranging from 0.5 - 2 mg/mL for 15 minutes per binding cycle in iterative cycles of binding. After each binding challenge, the multi-well filter plate was centrifuged to separate the unbound IgG from the resin, and the amount of unbound IgG was quantified by absorbance measurement at 280 nm of the effluent.

**Table 3**

| Procedure for testing binding characteristics and Alkaline Stability of Affinity Resins | | | | |
|---|---|---|---|---|
| **Step** | **Buffer** | **Volume (µL)** | **Time (min)** | **# of Cycles** |
| Equilibrate | PBS | 100 | Vacuum | |
| NaOH treat | 0.1 M+ 1M NaCl, 24 hours | 100 | 2+ | 3+ |
| Equilibrate | PBS | 100 | 2 | 5 |
| Load Sample | 0.5 mg/mL mAb | 100 | 15 | 4 |
| Load Sample | 1.0 mg/mL mAb | 100 | 15 | 4 |
| Load Sample | 2.0 mg/mL mAb | 100 | 15 | 4 |

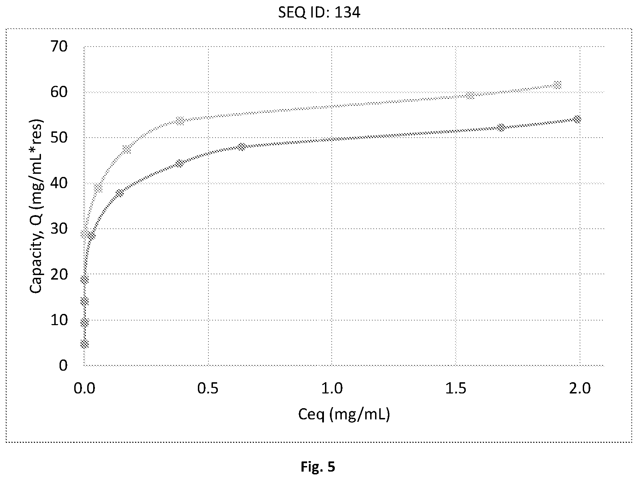

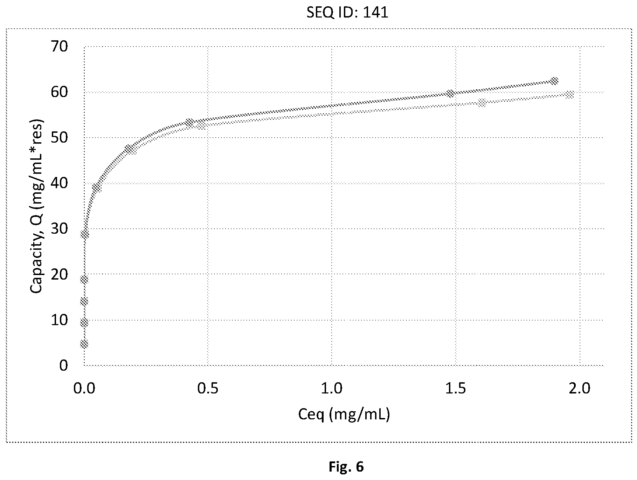

An adsorption isotherm curve was constructed by plotting the concentration of IgG remaining in solution after incubation with resin against amount of IgG bound to resin, at the various challenge conditions. Figs. 4-6 show the adsorption isotherms for affinity resins with ligands having SEQ ID NOS. 129, 134 and 144, respectively. The results showed that the resin with SEQ ID. 129 retains over 94% binding, that with SEQ ID. 134 retains over 88% binding and that with SEQ ID. 144 retains over 95% binding relative to the initial Qmax after 24 hours exposure to 0.1 M NaOH with 1 M NaCl.

## EXAMPLE 6. Monoclonal Antibody Purification

A clarified cell culture feed stream (CCCF) from a CHO cell line containing monoclonal antibody (MAb1) at a titer of 2.5 g/L was used loaded onto an affinity resin prepared from the ligand corresponding to SEQ ID No. 129. The resin was packed into an 0.3 x 10 cm (0.707 mL) column and operated as shown in Table 4.

**Table 4**

| Affinity purification of MAb1 | | | |
|---|---|---|---|
| **Step** | **Buffer** | **CV** | **V (cm/hr)** |
| Equilibration | PBS | 2 | 150 |
| Load Sample | CCCF (2.5 g/L) | 10.4 | 150 |
| Chase | PBS | 8.5 | 150 |
| Wash | 50 mM Tris, pH 7.5, 500 mM NaCl | 8.5 | 150 |
| Elute | 100 mM Sodium Acetate, pH 3.5 | 5 | 150 |
| CIP | 0.1 M NaOH, 1 M NaCl | 5.7 | 600 |
| Abbreviations for **Tables 4-6:** CV, column volume; V, velocity; CIP, clean in place. | | | |

The fractions were analyzed on a 4-12% Bis-Tris SDS-PAGE gel run under non-reducing conditions. The samples loaded in each lane are (1) SeeBlue Plus2 molecular weight markers, (2) crude feedstream (i.e., the CCCF), (3) 10x-diluted crude feedstream, (4) flow through fraction pool (i.e., chase), (5) wash fraction pool, (6) elution fraction pool (6.4 ug), and (7) strip fraction pool (i.e., CIP pool).

**EXAMPLE 7. Monoclonal Antibody Purification on CIP Resins**

Packed columns were used to purify a monoclonal antibody at a titer of 2.5 mg/mL in a CHO CCCF. Resins were prepared from ligands corresponding to SEQ ID Nos. 129, 134 and 144 as described in Example 4, packed into 0.3 x 10 cm (0.7 mL) columns and was operated as shown in **Table 5.**

**Table 5**

| Affinity purification of a Monoclonal Antibody | | | |
|---|---|---|---|
| **Step** | **Buffer** | **CV** | **V (cm/hr)** |
| Equilibration | PBS | 2 | 150 |
| Load Sample | CCCF (2.5 g/L) | 11.9 | 150 |
| Chase | PBS | 8.5 | 150 |
| Wash | 50 mM Tris, pH 7.5, 500 mM NaCl | 8.5 | 150 |
| Elute | 100 mM Sodium Acetate, pH 3.5 | 5 | 150 |
| CIP | 0.1 M NaOH, 1 M NaCl | 5.7 | 600 |
| Re-equilibration | PBS | 14.1 | 600 |
| Between each purification cycle, the resins were exposed to the CIP agent for a total of 60 minutes to simulate 4 CIP cycles. | | | |

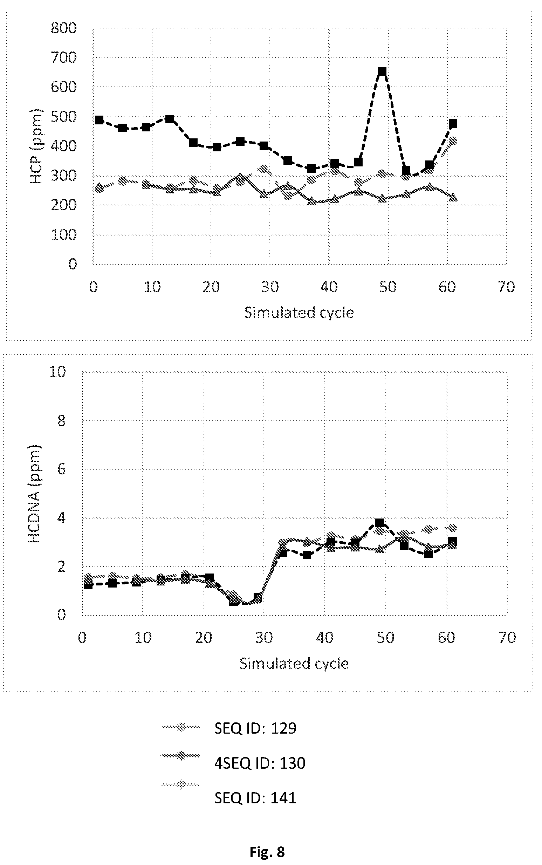

Eluate fractions from each purification cycle were collected and purity was analyzed for residual host cell proteins (HCP) and residual host cell DNA (HCDNA) using the Cygnus™ CHO Host Cell Proteins 3rd Generation assay, and the ThermoFisher Quant-iT™ PicoGreen™ assay, respectively. **Figure 8,** top panel, a illustrates the purity of eluate fractions obtained from the affinity resins agents over 60 simulated cycles, with all cycles having HCP less than 700 ppm, and most cycles having HCP less than 300 ppm. Similarly, as shown in **Figure 8,** bottom panel, HCDNA was reduced below 5 ppm for the three resins in all cycles. The data confirm that the resin can be cleaned with NaOH and reused for multiple cycles.

**EXAMPLE 8. Purification Yields.**

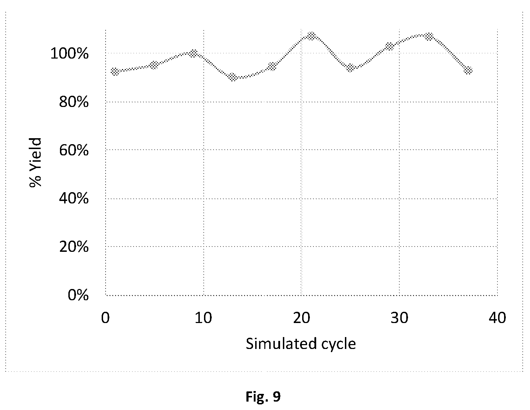

For this study, a purified monoclonal antibody at a concentration of 2.36 mg/mL was loaded onto a 0.3 x 10 cm (0.7 mL) column packed with an affinity resin with the affinity ligand of SEQ ID. 144. Ten purification cycles were performed as described in **Table 6.** Between each cycle, the resin was exposed to the CIP agent 0.1 M NaOH, 1 M NaCl for 60 minutes, which is equivalent to 4 simulated CIP cycles. Yields were determined after each of the 10 cycles and plotted as simulated cycles in **Figure 9.** The data indicate that the resin can be cleaned with NaOH and reused for multiple cycles while maintaining consistent yield.

**Table 6**

| Operating Conditions for Yield Determination | | | |
|---|---|---|---|
| Step | Buffer | CV | V (cm/hr) |
| Equilibration | PBS | 2 | 150 |
| Load Sample | Monoclonal antibody in PBS (2.36 g/L) | 11.9 | 150 |
| Chase | PBS | 8.5 | 150 |
| Wash | 50 mM Tris, pH 7.5, 500 mM NaCl | 8.5 | 150 |
| Elute | 100 mM Sodium Acetate, pH 3.5 | 5 | 150 |
| CIP | 0.1 M NaOH, 1 M NaCl | 5.7 | 600 |
| Re-equilibration | PBS | 14.1 | 600 |

**EXAMPLE 9. Role of Helix Resins**

This example demonstrates that helices 2,4 and 6 are not critical to the binding. Modifications may be made to helices 2,4 or 6 and as long as these modifications still enable the TPR to form a stacked helical bundle with helices 1, 3, 5 and 7 forming an inner surface, the ligands maintain binding.

**EXAMPLE 10. Role of Helix Resins**

The role of sequence variation in helix 2 of the 7 helices of these TPR domain-containing ligands was examined by determining the binding affinity of four biotinylated ligands which have identical sequences for helices 1, 3, 5 and 7 but which differ in the sequences for helix 2. Binding affinities were determined as described in Example 2 and the results are shown in **Table 7.** These results show that modifications made in helix 2 maintain ligand binding.

**Table 7**

| Affinity ($K_D$) of Ligands with Helix 2 for Fabs and IgG | | | |
|---|---|---|---|
| Ligand (SEQ ID NO.) | Tremfya Fab $K_D$ (nM) | Herceptin Fab $K_D$ (nM) | IgG $K_D$ (nM) |
| 41 | 24 | 15 | 3 |
| 89 | 8 | 41 | < 1 |
| 90 | 12 | 51 | < 1 |
| 92 | 8 | 28 | < 1 |

**EXAMPLE 11. BINDING OF BIOTINYLATED LIGANDS TO MONOCLONAL ANTIBODIES**

This example demonstrates the binding of biotinylated ligands to a IgG1 (Praluent®), IgG2 (Ajovy®) and IgG4 (Dupixent®) monoclonal antibodies using biolayer interferometry. Antibodies were obtained from McKesson Supply Manager (Irving, Tx). Biotinylated ligands were immobilized on sensors and incubated with solutions containing monoclonal antibody protein at various concentrations. The binding affinities are shown in the following table.

| | Binding affinity (nM) | | |
|---|---|---|---|
| SEQ ID | Praluent | Ajovy | Dupixent |
| 154 | <1 | 92 | 13 |
| 155 | <1 | 117 | 32 |
| 156 | <1 | 224 | 7 |
| 157 | <1 | 270 | 8 |
| 158 | <1 | 282 | 176 |

(continued)

| SEQ ID | Binding affinity (nM) | | |
| --- | --- | --- | --- |
| | Praluent | Ajovy | Dupixent |
| 159 | <1 | 436 | 9 |
| 160 | <1 | 543 | 24 |
| 161 | <1 | 654 | 31 |
| 162 | <1 | 665 | 25 |
| 163 | <1 | >1000 | 13 |
| 164 | <1 | >1000 | 82 |
| 165 | <1 | >1000 | 31 |
| 166 | <1 | >1000 | 120 |
| 167 | <1 | >1000 | 31 |
| 168 | <1 | >1000 | 232 |
| 169 | <1 | >1000 | 31 |
| 170 | <1 | >1000 | 28 |
| 171 | <1 | >1000 | 242 |
| 172 | <1 | >1000 | 215 |
| 173 | <1 | >1000 | 95 |
| 174 | <1 | >1000 | 221 |

## EXAMPLE 12. BINDING OF BIOTINYLATED LIGANDS TO mAB and Fab

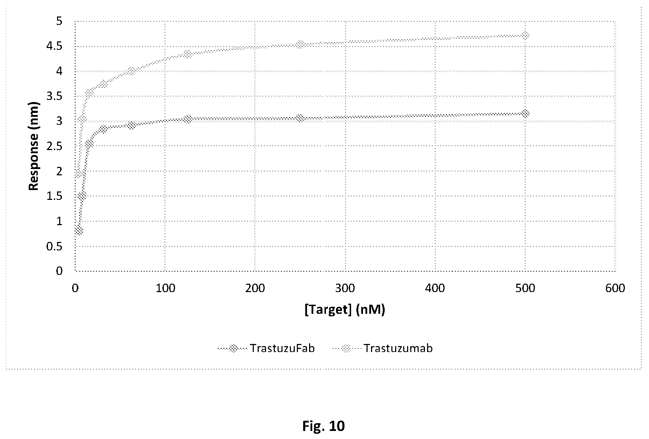

This example demonstrates the binding of biotinylated ligands to full mAb and Fab molecules. Biotinylated ligand corresponding to SEQ ID No: 181 was immobilized on sensors and incubated with solutions containing either Trastuzumab or Trastuzufab protein at various concentrations. It is evident from the dose response curve for binding shown in Figure 10 that the ligand binds with high affinity to both molecules.

## EXAMPLE 13. AFFINITY RESINS WITH HIGH BINDING CAPACITY

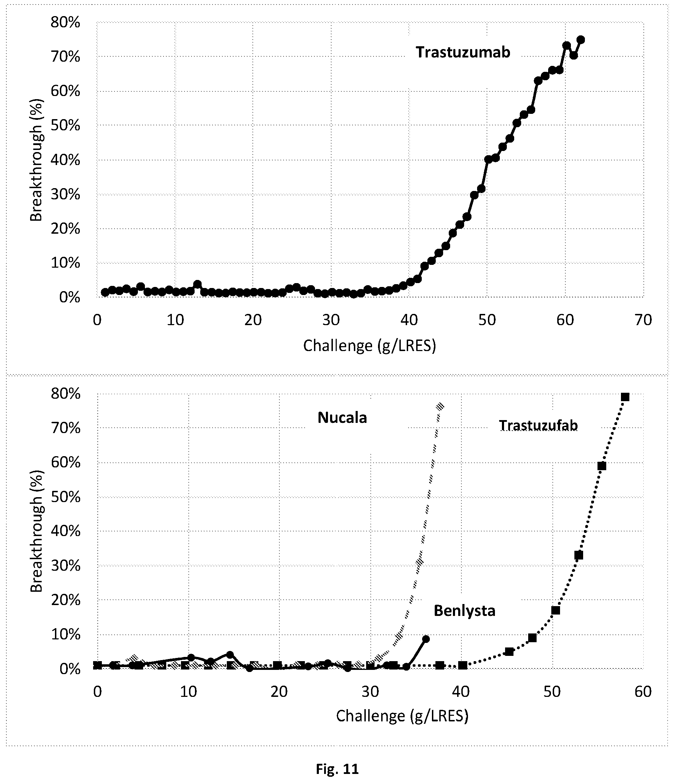

This example demonstrates that affinity resins with high binding capacities can be achieved with ligands of the invention. An affinity resin was prepared with the ligand corresponding to SEQ ID No: 181 conjugated to epoxy activated Jetted A50 agarose beads and packed into a 3 x 50 mm column. The binding capacity of Trastuzumab, Trastuzufab, Nucala® and Benlysta® was obtained at 4 minute residence time using a challenge concentration of 2 g/L. The breakthrough curves are shown in Figure 11 and the 10% breakthrough values are listed in the following table.

| Analyte | DBC @ 10% Breakthrough |
| --- | --- |
| Trastuzumab | 43 g/L |
| Trastuzufab | 48 g/L |
| Nucala® | 34 g/L |
| Benlysta® | 37 g/L |

## EXAMPLE 14. NaOH STABILITY OF THE AFFINITY RESIN COLUMN

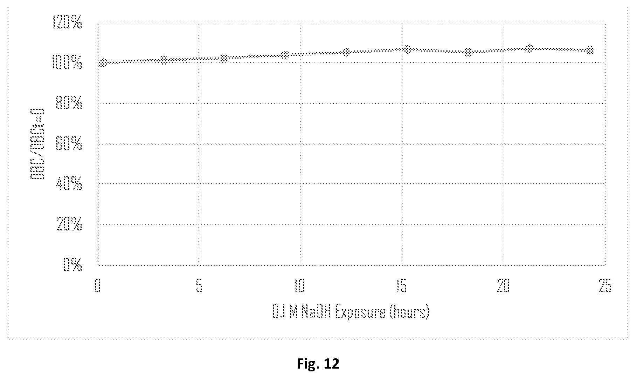

This **example** demonstrates the NaOH stability of the affinity resin column in the previous example. Repeated DBC cycles were performed at the indicated time points after exposure of the resin to 0.1 M NaOH containing 1 M NaCl and the DBC at 10% breakthrough determined. The data shown in Figure 12 demonstrates that the resin shows no loss in binding capacity over 24-hour exposure to 0.1 M NaOH containing 1 M NaCl.

## EXAMPLE 15. PURIFICATION OF AN ANTIBODY

This example demonstrates the purification of an antibody from a crude feedstream and that the resin can be reused many times. The affinity resin of Example 13 was packed into a 0.3 x 10 cm column and challenged with a host cell clarified feed (HCCF) containing Trastuzumab at a titer of 0.8 g/L. The column was operated as shown in the following table.

| Step | Buffer | Residence Time (min) | CV |
|---|---|---|---|
| Equilibration | 20 mM Tris, pH 7.4 | 1 | 7 |
| Load Sample | HCCF (0.8 mg/mL) | 4 | 44 |
| Chase | 20 mM Tris, pH 7.4 | 4 | 5 |
| Wash | 50mM HEPES, 1M CaCl2 pH 7.5 | 1 | 7 |
| Elution | 100mM glycine, 150mM NaCl pH 3 | 2 | 7 |
| Strip | 0.1 M NaOH, 1 M NaCl | 4 | 7.6 |
| Re-Equilibration | 20 mM Tris, pH 7.4 | 2 | 14.3 |

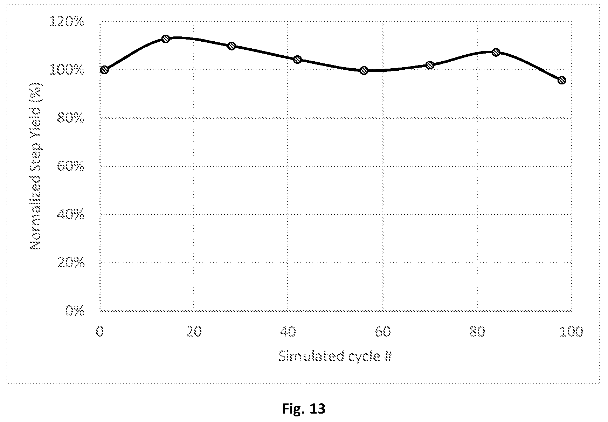

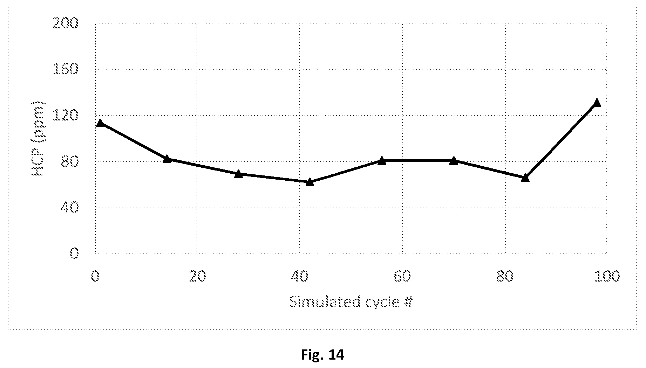

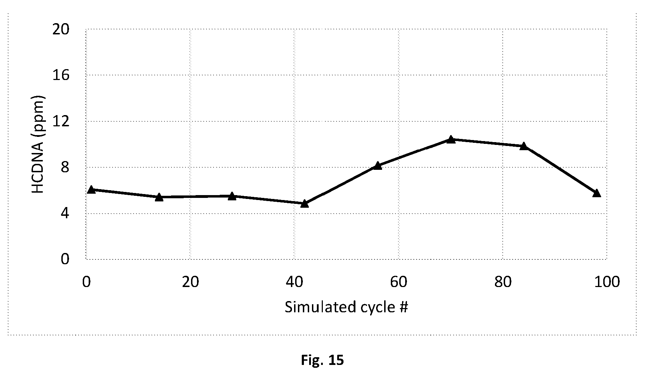

During the "Strip" step the flow was stopped so that the total exposure of the resin to 0.1 M NaOH containing 1 M NaCl was 3.5 hours per cycle and the column was operated for 8 cycles. The yield obtained at each cycle is shown in Figure 13. The residual HCP and DNA impurity levels obtained at each cycle are shown in Figures 14 and 15, respectively. Collectively, the data show high purity and the ability to cycle the column without loss in performance.

Any methods disclosed herein need not be performed in the order recited. The methods disclosed herein include certain actions taken by a practitioner; however, they can also include any third-party instruction of those actions, either expressly or by implication.

### TABLE 8

| SEQ ID | AA Sequence |
|---|---|
| | **SEQUENCES** |
| 1 | $X^1RWX^2X^3$ |
| 2 | $X^4AWX^5X^6LGX^7$ |
| 3 | $X^8AWX^9X^{10}LGX^{11}$ |
| 4 | $X^{12}ARX^{13}X^{14}LEX^{15}$ |
| 5 | $X^1RWX^2X^3$LGAAYAARGDYDRAIEYYQRALELDPNNAX$^4$AWX$^5$X$^6$LGX$^7$AYAARGDYDRAIE YYQRALELDPNNAX$^8$AWX$^9$X$^{10}$LGX$^{11}$AYAARGDYDRAIEYYQRALELDPNNEX$^{12}$ARX$^{13}$X$^{14}$ LEX$^{15}$ |
| 6 | MAQAAAGDEADRWADLGAAYAARGDYDRAIEYYERALELDPNNAWAWWDLGVAYAARGDY DRAIEYYERALELDPDDATAWSRLGHAYAARGDYDRAIEYYERALELDPEDETARYLLEH ARRARQGQAGQGGGGSGLNDIFEAQKIEWHECHHHHHH |
| 7 | MAQAAAGDEADRWADLGAAYAARGDYDRAIEYYQRALELDPNNAWAWWDLGVAYAARGDY DRAIEYYQRALELDPNNATAWNRLGHAYAARGDYDRAIEYYERALELDPEDETARYLLEH ARRARQGQAGQGGGGSGLNDIFEAQKIEWHECHHHHHH |
| 8 | MAQAAAGDEAGRWEGQGAAYAARGDYDRAIEYYQRALELDPNNAWAWWELGVAYAARGDY DRAIEYYQRALELDPNNATAWNRLGHAYAARGDYDRAIEYYQRALELDPNNEVARSQLEH ARRARRGQAGQGGGGSGLNDIFEAQKIEWHECHHHHHH |
| 9 | MAQAAAGDEAGRWEGQGAAYAARGDYDRAIEYYERALELDPNNAWAWWDLGVAYAARGDY DRAIEYYERALELDPDDATAWSRLGHAYAARGDYDRAIEYYERALELDPEDETARYLLEH ARRARQGQAGQGGGGSGLNDIFEAQKIEWHECHHHHHH |

(continued)

| SEQ ID | AA Sequence |
|---|---|
| | **SEQUENCES** |
| 10 | MAQAAAGDEAGRWENQGAAYAARGDYDRAIEYYERALELDPNNAWAWWELGVAYAARGDYDRAIEYYERALELDPDDAHAWRRLGFAYAARGDYDRAIEYYERALELDPEDEVAREVLEEARRARQGQAGQGGGSGLNDIFEAQKIEWHECHHHHHH |
| 11 | MAQAAAGDEAARWEWQGAAYAARADYDRAIEYYERALELDPNNAWAWWELGVAYAARGELDRAIEYYERALELDPDDAHAWFRLGWAYAARGDYDRAIEYYQRALELDPEDELAREELEEARRARQGQAGQGGGSGLNDIFEAQKIEWHECHHHHHH |
| 12 | MAQAAAGDEAQRWENQGAAYAARGDYDRAIEYYERALELDPNNAWAWWELGVAYAARGDYDRAIEYYERALELDPDDATAWSRLGHAYAARGDYDRAIEYYERALELDPEDETARYLLEHARRARQGQAGQGGGSGLNDIFEAQKIEWHECHHHHHH |
| 13 | MAQAAAGDEAARWEWQGAAYAARGDYDRAIEYYERALELDPNNAWAWWELGVAYAARGELDRAIEYYERALELDPDDAHAWFRLGWAYAARGDYDRAIEYYERALELDPEDELAREELEEARRARQGQAGQGGGSGLNDIFEAQKIEWHECHHHHHH |
| 14 | MAQAAAGDEAQRWENQGAAYAARGDYDRAIEYYERALELDPNNAWAWWELGVAYAARGDYDRAIEYYERALELDPDDAAAWRRLGHAYAARGDYDRAIEYYERALELDPEDETARYLLEHARRARQGQAGQGGGSGLNDIFEAQKIEWHECHHHHHH |
| 15 | MAQAAAGDEAQRWENQGAAYAARGDYDRAIEYYERALELDPNNAWAWWDLGVAYAARGDYDRAIEYYERALELDPDDAAAWRRLGHAYAARGDYDRAIEYYERALELDPEDELARRILEDARRARQGQAGQGGGSGLNDIFEAQKIEWHECHHHHHH |
| 16 | MAQAAAGDEAGRWEGQGAAYAARGDYDRAIEYYERALELDPNNAWAWWELGVAYAARGDYDRAIEYYERALELDPDDAAAWRRLGHAYAARGDYDRAIEYYERALELDPEDELARRILEDARRARQGQAGQGGGSGLNDIFEAQKIEWHECHHHHHH |
| 17 | MAQAAAGDEAGRWEGQGAAYAARGDYDRAIEYYERALELDPNNAWAWWELGVAYAARGELDRAIEYYERALELDPDDAHAWFRLGWAYAARGDYDRAIEYYQRALELDPNNEVARSQLEHARRARRGQAGQGGGSGLNDIFEAQKIEWHECHHHHHH |
| 18 | MAQAAAGDEAQRWENQGAAYAARGDYDRAIEYYERALELDPNNAWAWWELGVAYAARGELDRAIEYYERALELDPDDAHAWFRLGWAYAARGDYDRAIEYYERALELDPEDELAREELEEARRARRGQAGQGGGSGLNDIFEAQKIEWHECHHHHHH |
| 19 | MAQAAAGDEAGRWEGQGAAYAARGDYDRAIEYYERALELDPNNAWAWWDLGVAYAARGDYDRAIEYYERALELDPDDATAWSRLGHAYAARGDYDRAIEYYERALELDPEDELARQQLEWARRARQGQAGQGGGSGLNDIFEAQKIEWHECHHHHHH |
| 20 | MAQAAAGDEAQRWETLGAAYAARGDYDRAIEYYQRALELDPNNAWAWWELGVAYAARGDYDRAIEYYERALELDPDDATAWSRLGHAYAARGDYDRAIEYYQRALELDPNNEVARSQLEHARRARRGQAGQGGGSGLNDIFEAQKIEWHECHHHHHH |
| 21 | MAQAAAGDEALRWWDQGAAYAARGDYDRAIEYYERALELDPNNAWAWWDLGVAYAARGDYDRAIEYYERALELDPDDAHAWFRLGWAYAARGDYDRAIEYYERALELDPEDELAREELEEARRARQGQAGQGGGSGLNDIFEAQKIEWHECHHHHHH |
| 22 | MAQAAAGDEAGRWEGQGAAYAARGDYDRAIEYYERALELDPNNAWAWWELGTAYAARGDYDRAIEYYERALELDPDDAIAWGRLGHAYAARGDYDRAIEYYERALELDPEDELARQQLEWARRARQGQAGQGGGSGLNDIFEAQKIEWHECHHHHHH |

(continued)

| SEQ ID | AA Sequence |
|---|---|
| 23 | MAQAAAGDEAGRWEGQGAAYAARGDYDRAIEYYERALELDPNNAWAWWDLGVAYAARGDY DRAIEYYERALELDPDDATAWNRLGHAYAARGDYDRAIEYYQRALELDPNNEVARSQLEH ARRARRGQAGQGGGSGLNDIFEAQKIEWHECHHHHHH |
| 24 | MAQAAAGDEAQRWENQGAAYAARGDYDRAIEYYERALELDPNNAWAWWDLGVAYAARGDY DRAIEYYERALELDPDDATAWSRLGHAYAARGDYDRAIEYYERALELDPEDETARYLLEH ARRARQGQAGQGGGSGLNDIFEAQKIEWHECHHHHHH |
| 25 | MAQAAAGDEAQRWETLGAAYAARGDYDRAIEYYQRALELDPNNAWAWWELGVAYAARGDY DRAIEYYQRALELDPDDAAAWRRLGHAYAARGDYDRAIEYYERALELDPEDELARRILED ARRARQGQAGQGGGSGLNDIFEAQKIEWHECHHHHHH |
| 26 | MAQAAAGDEAGRWEGQGAAYAARGDYDRAIEYYERALELDPNNAWAWWDLGVAYAARGDY DRAIEYYERALELDPDDAAAWNRLGHAYAARGDYDRAIEYYQRALELDPNNEVARSQLEH ARRARRGQAGQGGGSGLNDIFEAQKIEWHECHHHHHH |
| 27 | MAQAAAGDEAQRWENQGAAYAARGDYDRAIEYYERALELDPNNAWAWWELGVAYAARGDY DRAIEYYERALELDPDDAAAWRRLGHAYAARGDYDRAIEYYERALELDPEDELAREELEE ARRARQGQAGQGGGSGLNDIFEAQKIEWHECHHHHHH |
| 28 | MAQAAAGDEAQRWENQGAAYAARGDYDRAIEYYERALELDPNNAWAWWELGVAYAARGDY DRAIEYYERALELDPDDATAWNRLGHAYAARGDYDRAIEYYERALELDPEDETARYLLEH ARRARQGQAGQGGGSGLNDIFEAQKIEWHECHHHHHH |
| 29 | MAQAAAGDEAGRWEGQGAAYAARGDYDRAIEYYERALELDPNNAWAWWDLGVAYAARGDY DRAIEYYERALELDPDDAAAWRRLGHAYAARGDYDRAIEYYERALELDPEDELARRILED ARRARQGQAGQGGGSGLNDIFEAQKIEWHECHHHHHH |
| 30 | MAQAAAGDEAARWEWQGAAYAARGDYDRAIEYYERALELDPNNAWAWWELGNAYAARGDY DRAIEYYERALELDPDDATAWSRLGHAYAARGDYDRAIEYYERALELDPEDETARYLLEH ARRARRGQAGQGGGSGLNDIFEAQKIEWHECHHHHHH |
| 31 | MAQAAAGDEAARWEWQGAAYAARGDYDRAIEYYERALELDPNNAWAWWELGVAYAARGEL DRAIEYYERALELDPDDAHAWFRLGWAYAARGDYDRAIEYYQRALELDPEDELARRILED ARRARQGQAGQGGGSGLNDIFEAQKIEWHECHHHHHH |
| 32 | MAQAAAGDEAVRWEIQGAAYAARGDYDRAIEYYERALELDPNNAWAWWELGVAYAARGDY DRAIEYYERALELDPDDAHAWFRLGWAYAARGDYDRAIEYYERALELDPEDELARRILED ARRARQGQAGQGGGSGLNDIFEAQKIEWHECHHHHHH |
| 33 | MAQAAAGDEAQRWETLGAAYAARGDYDRAIEYYQRALELDPNNAWAWWELGVAYAARGDY DRAIEYYQRALELDPNNATAWNRLGHAYAARGDYDRAIEYYQRALELDPNNEVARSQLEH ARRARRGQAGQGGGSGLNDIFEAQKIEWHECHHHHHH |
| 34 | MAQAAAGDEAGRWEGQGAAYAARGDYDRAIEYYERALELDPNNAWAWWDLGVAYAARGDY DRAIEYYERALELDPDDAAAWRRLGHAYAARGDYDRAIEYYERALELDPEDETARYLLEH ARRARQGQAGQGGGSGLNDIFEAQKIEWHECHHHHHH |
| 35 | MAQAAAGDEAARWEWQGAAYAARGDYDRAIEYYERALELDPNNAWAWWELGVAYAARGEL DRAIEYYERALELDPDDAHAWFRLGWAYAARGDYDRAIEYYERALELDPEDELARRILED ARRARQGQAGQGGGSGLNDIFEAQKIEWHECHHHHHH |

| SEQUENCES | |
|---|---|
| **SEQ ID** | **AA Sequence** |
| 36 | MAQAAAGDEAQRWENQGAAYAARGDYDRAIEYYERALELDPNNAWAWWELGVAYAARGDY DRAIEYYERALELDPDDAAAWRRLGHAYAARGDYDRAIEYYERALELDPEDELARRILED ARRARQGQAGQGGGSGLNDIFEAQKIEWHECHHHHHH |
| 37 | MAQAAAGDEAQRWENQGAAYAARGDYDRAIEYYERALELDPNNAWAWWELGVAYAARGEL DRAIEYYERALELDPDDAHAWFRLGWAYAARGDYDRAIEYYERALELDPEDELARRILED ARRARQGQAGQGGGSGLNDIFEAQKIEWHECHHHHHH |
| 38 | MAQAAAGDEAQRWETLGAAYAARGDYDRAIEYYQRALELDPNNAWAWWELGVAYAARGDY DRAIEYYERALELDPDDAAAWRRLGHAYAARGDYDRAIEYYERALELDPEDELARRILED ARRARQGQAGQGGGSGLNDIFEAQKIEWHECHHHHHH |
| 39 | MAQAAAGDEAQRWEHLGAAYAARGDYDRAIEYYQRALELDPNNAWAWWELGVAYAARGDY DRAIEYYQRALELDPNNAVAWNRLGHAYAARGDYDRAIEYYQRALELDPNNEFARQSLEE ARRARRGQAGQGGGSGLNDIFEAQKIEWHECHHHHHH |
| 40 | MAQAAAGDEANRWYVLGAAYAARGDYDRAIEYYQRALELDPNNAYAWDELGSAYAARGDY DRAIEYYQRALELDPNNAVAWSRLGQAYAARGDYDRAIEYYQRALELDPNNETARHLLEW ARRARRGQAGQGGGSGLNDIFEAQKIEWHECHHHHHH |
| 41 | MAQAAAGDEAERWYDLGAAYAARGDYDRAIEYYQRALELDPNNAWAWWELGVAYAARGDY DRAIEYYQRALELDPNNAVAWARLGIAYAARGDYDRAIEYYQRALELDPNNEVARDALEY ARRARRGQAGQGGGSGLNDIFEAQKIEWHECHHHHHH |
| 42 | MAQAAAGDEAERWESQGAAYAARGDYDRAIEYYERALELDPNNAWAWWELGTAYAARGDY DRAIEYYERALELDPDDARAWSRLGHAYAARGDYDRAIEYYERALELDPEDETARANLED ARRARRGQAGQGGGSGLNDIFEAQKIEWHECHHHHHH |
| 43 | MAQAAAGDEAQRWEEQGAAYAARGDYDRAIEYYERALELDPNNAWAWWELGTAYAARGDY DRAIEYYERALELDPDDARAWSRLGHAYAARGDYDRAIEYYERALELDPEDETARFLLEQ ARRARRGQAGQGGGSGLNDIFEAQKIEWHECHHHHHH |
| 44 | MGGGGSAAAGDEAIRWNTLGYAYGWRGDYDRAIEYYQRALELDPNNATAWAALGYAYQER GDYDRAIEYYQRALELDPNNAHAWQYLGFAYWNRGDYDRAIEYYQRALELDPNNESARSY LEDARRTRRGQAGQGGGSGLNDIFEAQKIEWHECHHHHHH |
| 45 | MGGGGSAAAGDEADRWYRLGSAYWDRGDYDRAIEYYQRALELDPNNAGAWYWLGSAYWHR GDYDRAIEYYQRALELDPNNAYAWSELGHAYIDRGDYDRAIEYYQRALELDPNNEFARGQ LEGARRLRRGQAGQGGGSGLNDIFEAQKIEWHECHHHHHH |
| 46 | MGGGGSAAAGDEADRWSTQGAAYAARGDYDRAIEYYQRALELDPNNAWAWWDLGVAYAAR GDYDRAIEYYQRALELDPNNAVAWARLGIAYAARGDYDRAIEYYQRALELDPNNEVARDA LEYARRARRGQAGQGGGSGLNDIFEAQKIEWHECHHHHHH |
| 47 | MGGGGSAAAGDEAGRWEGQGAAYAARGDYDRAIEYYERALELDPNNAWAWWDLGVAYAAR GDYDRAIEYYQRALELDPNDATAWNRLGHAYAARGDYDRAIEYYQRALELDPNNEVARQL LEYARRARRGQAGQGGGSGLNDIFEAQKIEWHECHHHHHH |
| 48 | MGGGGSAAAGDEAVRWEELGAAYAARGDYDRAIEYYQRALKLDPNNAWAWWELGVAYAAR GDYDRAIEYYQRALELDPNNAVAWARLGWAYAARGDYDRAIEYYQRALELDPNNESARTG LKLARRARRGQAGQGGGSGLNDIFEAQKIEWHECHHHHHH |

(continued)

| SEQ ID | AA Sequence |
|---|---|
| | **SEQUENCES** |
| 49 | MGGGGSAAAGDEAFRWWDQGAAYTARGDYDRAIEYYQRALELDPNNAWAWWELGVAYAAR GDYDRAIEYYQRALELDPNNAVAWNRLGHAYAARGDYDRAIEYYQRALELDPNNEVARDQ LERARRARRGQAGQGGGSGLNDIFEAQKIEWHECHHHHHH |
| 50 | MGGGGSAAAGDEAARWEWQGAAYAARGDYDHAIEYYERALELDPNNAWAWWELGVAYAAR GELDRAIEYYERALELDPDDAHAWFRLGWAYAARGDYDRAIEYYERALELDPEDELAREE LEEARRARQGQAGQGGGSGLNDIFEAQKIEWHECHHHHHH |
| 51 | MGGGGSAAAGDEADRWADLGAAYAARGDYDRAIEYYQRALELDPNNAWAWWDLGVAYAAR GDYDRAIEYYQRALELDPNNAVAWRRLGHAYAARGDYDRAIEYYQRALELDPNNELAREQ LERARRARRGQAGQGGGSGLNDIFEAQKIEWHECHHHHHH |
| 52 | MGGGGSAAAGDEADRWADLGAAYAARGDYDRAIEYYQRALELDPNNAWAWWDLGVAYAAR GDYDRAIEYYQRALELDPNNAVAWRRLGHAYAARGDYDRATEYYQRALELDPNNELAREQ LEHARRARRGQAGQGGGSGLNDIFEAQKIEWHECHHHHHH |
| 53 | MGGGGSAAAGDEAERWYDLGAAYAARGDYDRAIEYYQRALELDPNNAWAWWELGVAYAAR GDYDRAIEYYQRALELDPNNAVAWSRLGQAYAARGDYDRAIEYYQRALELDPNNETARHL LEWARRARRGQAGQGGGSGLNDIFEAQKIEWHECHHHHHH |
| 54 | MGGGGSAAAGDEAARWEVQGAAYAARGDYDRAIEYYERALELDPNNAWAWEHLGWAYAAR GDYDRAIEYYERALELDPDDAHAWFRLGFAYAARGDYDRAIEYYERALELDPEDELARAE LESARRARQGQAGQGGGSGLNDIFEAQKIEWHECHHHHHH |
| 55 | MGGGGSAAAGDEANRWYYLGAAYAARGDYDRAIEYYQRALELDPNNAFAWDELGNAYAAR GDYDRAIEYYQRALELDPNNAVAWNRLGHAYAARGDYDRAIEYYQRALELDPNNEVARTL LEQARRARRGQAGQGGGSGLNDIFEAQKIEWHECHHHHHH |
| 56 | MGGGGSAAAGDEAWRWYSQGAAYAARGDYDRAIEYYERALELDPNNAWAWWELGTAYAAR GDYDRAIEYYERALELDPDDAIAWGRLGHAYAARGDYDRAIEYYERALELDPEDELARQQ LEWARRARQGQAGQGGGSGLNDIFEAQKIEWHECHHHHHH |
| 57 | MGGGGSAAAGDEADRWWALGAAYAARGDYDRAIEYYQRALELDPNNAWAWWDLGVAYAAR GDYDRAVEYYQRALELDPNNAVAWRRLGHAYAARGDYDRAIEYYQRALELDPNNELAREQ LERARRARHGQAGQGGGSGLNDIFEAQKIEWHECHHHHHH |
| 58 | MGGGGSAAAGDEANRWYYLGAAYAARGDYDRAIEYYQRALELDPNNAFAWDELGNAYAAR EDYDRAIEYYQRALELDPNNAVAWNRLGHAYAARGDYDRAIEYYQRALELDPNNEVARTL LEQARRARRGQAGQGGGSGLNDIFEAQKIEWHECHHHHHH |
| 59 | MGGGGSAAAGDEAERWYDLGAAYAARGDYDRAIEYYQRALELDPNNAWAWWELGVAYAAR GDYDRAIEYYQRALELDPNNAVAWARLGIAYAARGDYDRAIEYYQRALELDPNNEVARTL LEQARRARRGQAGQGGGSGLNDIFEAQKIEWHECHHHHHH |
| 60 | MGGGGSAAAGDEAGRWEGQGAAYAARGDYDRAIEYYERALELDPNNAWAWWDLGVAYAAR GDYDRAIEYYERALELDPDDATAWSRLGHAYAARGDYDRAIEYYERALQLDPEDETARYL LEHARRARQGQAGQGGGSGLNDIFEAQKIEWHECHHHHHH |
| 61 | MGGGGSAAAGDEADRWYELGAGYAARGDYDRAIEYYQRALELDPNNAWAWWELGVAYAAR GDYDRAIEYYQRALELDPNNATAWQRLGHAYAARGDYDRAIEYYQRALELDPNNEAARYQ LEFARRARRGQAGQGGGSGLNDIFEAQKIEWHECHHHHHH |

(continued)

| SEQ ID | AA Sequence |
|---|---|
| | **SEQUENCES** |
| 62 | MGGGGSAAAGDEASRWEIQGAAYAARGDYDRAIEYYERALELDPNNAWAWWDLGVAYAAR GDYDRAIEYYQRALELDPNNAVAWRRLGHAYAARGDYDRAIEYYQRALELDPNNELAREQ LERARRARRGQAGQGGGSGLNDIFEAQKIEWHECHHHHHH |
| 63 | MGGGGSAAAGDEADRWADLGAAYAARGDYDRAIEYYQRALELDPNNAWAWWDLGVAYAAR GDYDRAIEYYQRALELDPNNAVAWRRLGHAYAARGDYDRAIEYYQRALELDPNNELAREQ LERARRARHGQAGQGGGSGLNDIFEAQKIEWHECHHHHHH |
| 64 | MGGGGSAAAGDEADRWAALGAAYAARGDYDRAIEYYQRALELDPNNAWAWWDLGVAYAAR GDYDRAIEYYERALELDPDDAHAWFRLGWAYAARGDYDRAIEYYERALELDPEDETARDV LEGARRARQGQAGQGGGSGLNDIFEAQKIEWHECHHHHHH |
| 65 | MGGGGSAAAGDEAERWYDLGAAYAARGDYDRAIEYYQRALELDPNNAWAWWELGVAYAAR GDYDRAIEYYQRALELDPNNAVAWRRLGIAYAARGDYDRAIEYYQRALELDPNNEVARDA LEYARRARRGQAGQGGGSGLNDIFEAQKIEWHECHHHHHH |
| 66 | MGGGGSAAAGDEAGRWYDLGAAYAARGDYDRAIEYYQRALELDPNNAWAWWELGVAYAAR GDYDRAIEYYQRALELDPNNAVAWARLGIAYAARGDYDRAIEYYQRALELDPNNEVARDA LEYARRARRGQAGQGGGSGLNDIFEAQKIEWHECHHHHHH |
| 67 | MGGGGSAAAGDEADRWADLGAAYAARGDYDRAIEYYQRALELDPNNAWAWWDLGVAYAAR GDYDRAIEYYQRALELDPNNAVAWRRLGHAYAARGDYDRAIEYYQRALELDPNNELAREQ LGRARRARRGQAGQGGGSGLNDIFEAQKIEWHECHHHHHH |
| 68 | MGGGGSAAAGDEGGRWEGQGAAYAARGDYDRAIEYYERALELDPNNAWAWWDLGVAYAAR GDYDRAIEYYERALELDPDDATAWSRLGHAYAARGDYDRAIEYYERALELDPEDETARYL LEHARRARQGQAGQGGGSGLNDIFEAQKIEWHECHHHHHH |
| 69 | MGGGGSAAAGDEAERWDYLGAAYAARGDYDRAIEYYQRALELDPNNAWAWWDLGLAYAAR GDYDRAIEYYQRALELDPNNAVAWFRLGWAYAARGDYDRAIEYYQRALELDPNNELARSE LENARRARRGQAGQGGGSGLNDIFEAQKIEWHECHHHHHH |
| 70 | MGGGGSAAAGDEAQRWENQGAAYAARGDYDRAIECYESALELDPNNAWAWWELGVAYAAR GDYDRAIEYYERALELDPDDAIAWSRLGHAYAARGDYDRAIEYYERALELDPEDETARYL LEHARRARQGQAGQGGGSGLNDIFEAQKIEWHECHHHHHH |
| 71 | MGGGGSAAAGDEAQHWENQGAAYAARGDYDRAIEYYERALELDPNNAWAWWELGVAYAAR GDYDRAIEYYERALELDPDDATVWSRLGHAYAARGDYDRAIEYYERALELDPEDETARYL LEHARRARQGQAGQGGGSGLNDIFEAQKIEWHECHHHHHH |
| 72 | MGGGGSAAAGDEAQCWENQGAAYAARGDYDRAIEYYERALELDPNNAWAWWELGVAYAAR GDYDRAIEYYERALELDPDDATAWSRLGHAYAARGDYDRAIEYYERALELDPEDETARYL LEHARRARQGQAGQGGGSGLNDIFEAQKIEWHECHHHHHH |
| 73 | MGGGGSAAAGDEAQHWENQGAAYAARGDYDRAIEYYGRALELDPNNAWAWWELGVAYAAR GDYDRAIEYYERALELDPDDATTWSRLGHAYAARGDYDRAIEYYERALELDPEDETARYL LEHARRARQGQAGQGGGSGLNDIFEAQKIEWHECHHHHHH |
| 74 | MGGGGSAAAGDEAQCWENQGAAYAARGDYDRAIEYYERALELDPNNAWAWWELGVAYAAR GDYDRAIEYYERALELDPDDATTWSRLGHAYAARGDYDRAIEYYVRALELDPEDETARYL LEHARRARQGQAGQGGGSGLNDIFEAQKIEWHECHHHHHH |

(continued)

| SEQ ID | AA Sequence |
|---|---|
| 75 | MGGGGSAAAGDEAQRWENQGAAYAARGDYDRAIEYYGRALELDPNNAWAWWELGVAYAAR GDYDRAIEYYERALELDPDDATAWSRLGHAYAARGDYDRAIEYYERALELDPENETARYL LEHARRARQGQAGQGGGSGLNDIFEAQKIEWHECHHHHHH |
| 76 | MGGGGSAAAGDEAQRWENQGAAYAARGDYDRAIEYYERALELEPNNAWAWWELGVAYAAR GDYDRAIEYYERALELDPDDATAWSRLGHAYAARGDYDRAIGYYERALELDPENETARYL LEHARRARQGQAGQGGGSGLNDIFEAQKIEWHECHHHHHH |
| 77 | MGGGGSAAAGDEAQRWENQGAAYAARGDYDRAIEYYERALELDPNNAWAWWELGVAYAAR GDFDRAIEYYERALELDPDDATVWSRLGHAYAARGDYDRAIEYYERALELDPVDETARYL LEHARRARQGQAGQGGGSGLNDIFEAQKIEWHECHHHHHH |
| 78 | MGGGGSAAAGDEAVRWEIQGAAYAARGDYDRAIEYYERALELDPNNAWAWWELGVAYAAR GDYDRAIEYYERALELDPDDAHVWFRLGWAYAVRGDYDRAIEYYERALELDPEDELARRI LEDARRARQGQAGQGGGSGLNDIFEAQKIEWHECHHHHHH |
| 79 | MGGGGSAAAGDEAVRWEIQGAAYAARGDYNRAIEYYERALELDPNNAWAWWELGVAYAAR GDYDRAIEYYERALNLDPDDAHTWFRLGWAYAARGDYARAIEYYERALELDPEDELVRRI LEDARRARQGQAGQGGGSGLNDIFEAQKIEWHECHHHHHH |
| 80 | MGGGGSAAAGDEAVRWEIQGAAYAARGDYDLAIEYYERTLELDPDNAWAWWELGVAYAAR GDYDRAIEYYERALELDPDDAHVWFRLGWAYATRGDYDRAIEYYERALELDPEDELARRI LEDARRARQGQAGQGGGSGLNDIFEAQKIEWHECHHHHHH |
| 81 | MGGGGSAAAGDEAARWEIQGAAYAARGDYDRAIEYYERALELDPNNAWAWWELGVAYAAR GDYDRAIEYYERALELDPDDAHTWFRLGWAYAARGDYDRAIAYYERALELDPEDELTRRI LGDARRVRQGQAGQGGGSGLNDIFEAQKIEWHECHHHHHH |
| 82 | MGGGGSAAAGDEAVRWEIQGAAYAARGDYDRAIEYYERALELDPNNAWAWWELGVAYAAR GDYDRAIEYYERALELDPDDARVWFRLGWAYAARGDYDRAIEYYERALELDPEDELARRI LEDTRRARQGQAGQGGGSGLNDIFEAQKIEWHECHHHHHH |
| 83 | MGGGGSAAAGDEVVHWEIQGAAYAARGDYDRAIEYYERALELDPNNAWAWWELGVAYAAR GDYDRAIEYYERALELDPDDAHVWFRLGWAYAARGDYDRAIEYYERALELDPEDELARRI LEDARRARQGQAGQGGGSGLNDIFEAQKIEWHECHHHHHH |
| 84 | MGGGGSAAAGDEAVRWEIQGAAYAARGDYDRAIEYYERALELDPNNAWAWWELGVAYAAR GDYDRAIEYYERALELDPDDAHVWFRLGWAYATRGDYDRAIEYYERALELDPEDELARRI LEDARRARQGQAGQGGGSGLNDIFEAQKIEWHECHHHHHH |
| 85 | MGGGGSAAAGDEALRWEIQGAAYAARGDYDRAIEYYERALELDPNNAWAWWELGVAYAAR GDYDRAIEYYERALELDPDDAHVWFRLGWAYAARGDHDRAIEYYERALELDPEDELARRI LEDARRARQGQAGQGGGSGLNDIFEAQKIEWHECHHHHHH |
| 86 | MGGGGSAAAGDEAERWYDLGAAYAARGDYDRAIEYYQRALELDPNDAWAWWELGVAYAAR GDYDRAIEYYQRALELDLNNAVAWARLGIAYAARGDYDRAIEYYQRALELDPNNEVARDA LEYARRARRGQAGQGGGSGLNDIFEAQKIEWHECHHHHHH |
| 87 | MGGGGSAAAGDEAERWYDLGAAYAARGDYDRAFEYYQRALELDPNNAWAWWELGVANAAR GDYDRAIENYQRALELDPNNAVAWARLGVAYAARGDYDRAIEYYQRALELDPNQVIARGA LEYARRARRGQAGQGGGSGLNDIFEAQKIEWHECHHHHHH |

(continued)

| SEQ ID | AA Sequence |
|---|---|
| | **SEQUENCES** |
| 88 | MGGGGSAAAGDEAERWYDLGAAYAARGDYDRAIEYYQRVLELDPNNAWAWWELGVGYAAR GDYDRAIEYYQRALELDPNNTVAWARLGIAYAARGDYDRAIEYYQRALELDPNNEVARDA LEYARRARRGQAGQGGGSGLNDIFEAQKIEWHECHHHHHH |
| 89 | MGGGGSAAAGDEAERWYDLGAAYAARGDFDRAIEYYRRTLELDPNDAWAWWELGVAYAAR GDYDRAIEYYQRALELDPNNAVAWARLGIAYATRGDYDRAIEYYQRALELDPNNEVARDA LEYARRVRRGQAGQGGGSGLNDIFEAQKIEWHECHHHHHH |
| 90 | MGGGGSAAAGDEAERWYDLGAAYAARGDYDRATEFYQRALELDPNDAWAWWELGIAYAAR GDYDRAIEYYQRALELDPNNAVAWARLGIAYAARGDYDRAIEYYQRALELDPNNEVARDA LEYARRARRGQAGQGGGSGLNDIFEAQKIEWHECHHHHHH |
| 91 | MGGGGSAAAGDEAERWYDLGAAYAARGDYDRAIEYYQRALELDPNDAWAWWELGVAYAAR GDYDRAIEYYQRALELDPNNAVAWARLGIAYAARGDYDRAIEYYQRALELDPNNEVARDA LEYARRARRGQAGQGGGSGLNDIFEAQKIEWHECHHHHHH |
| 92 | MGGGGSAAAGDEAERWYDLGAAYAARGDYDRAIEYYQRALEHDPNDAWAWWELGVAYAAR GDYDRAIEYYQRALELDTNNAVAWARLGIAYAARGDYDRAIEYYQRALELDPNNEVARDA LEYARRARRGQAGQGGGSGLNDIFEAQKIEWHECHHHHHH |
| 93 | MGGGGSAAAGDEAAHWEFQGAAYAARGDYDRAIEYYERALELDPNNAWAWEHLGWAYAAR GDYDRAIEYYERALELDPDDAHAWFRLGFAYAARGDYDRAIKYYERALELDPEDELARAE LESARRARQGQAGQGGGSGLNDIFEAQKIEWHECHHHHHH |
| 94 | MGGGGSAAAGDEAARWEVQGAAYAARGDYDRAIEYYERVLELDPNDAWAWEHLGWAYAAR GDYDRAIEYYERALEVDPDDAHAWFRLGFAYAARGDYDRAMEYYERALELDPEDELARAE LESARRARQGQAGQGGGSGLNDIFEAQKIEWHECHHHHHH |
| 95 | MGGGGSAAAGDEAARWEVQGAAYAARGDYDRAIEYYERALELDPNNAWAWEHLGWAYAAR GDYDRAIEYYERALELDPDDAHAWFRLGFAYAARGDYDRAIEYYERALELDPNNELAREQ LEHARRARRGQAGQGGGSGLNDIFEAQKIEWHECHHHHHH |
| 96 | MGGGGSAAAGDEAAHWEVQGAAYAARGDYDRAIEYYERALELDPNNAWAWEHLGWAYAAR GDFDRAIEYYERALELDPDDAHAWFRLGFAYAARGDYDRAIEYYERALELDPEDELARAE LESARRARQGQAGQGGGSGLNDIFEAQKIEWHECHHHHHH |
| 97 | MGGGGSAAAGDEAARWEVLGAAYAARGDYDRAIEYYERALELDPNNAWAWEHLGWAYAAR GDYDRAIEYYERALGLDPDDAHAWFRLGFAYAARGDYDRAIEYYERALELDPENELARAE LESARRARQGQAGQGGGSGLNDIFEAQKIEWHECHHHHHH |
| 98 | MGGGGSAAAGDEAARWEVQGAAYAARGDYDRAIEYYERALELDPNNAWAWEHLGWAYAAR GDYDRAIEYYERALELDPDDAHSWFRLGFAYAVRGDYDRAIEYYERALELDPEDELARAE LESARRARQGQAGQGGGSGLNDIFEAQKIEWHECHHHHHH |
| 99 | MGGGGSAAAGDEVARWEVQGAAYAARGGYDRAIEYYERALELDPNNAWAWEHLGWAYAAR GDYDRAIEYYERALELDPDDAHEWFRLGFAYAARGDYDRAIEYYERALELDPEDELARAE LESARRARQGQAGQGGGSGLNDIFEAQKIEWHECHHHHHH |
| 100 | MGGGGSAAAGDEAARWEVQGAAYAARGDYYRAIEYYERALELDPNNAWAWEHLGWAYAAR GDYDRAFEYYERALELDPDDAHVWFRLGFAYAVRGDYDRAIEYYERALELDPEDELARAE LESARRARQGQAGQGGGSGLNDIFEAQKIEWHECHHHHHH |

(continued)

| SEQUENCES | |
|---|---|
| SEQ ID | AA Sequence |
| 101 | MGGGGSAAAGDEAARWEVQGAAYAARGDYDRAIEYYERALELDPSNAWAWEHLAWAYAAR GDYDRAIECYERALELDPDDAHAWFRLGFSYAARGDYDRAIEYYERALELDPEDELARAE LESARRARQGQAGQGGGSGLNDIFEAQKIEWHECHHHHHH |
| 102 | MGGGGSAAAGDEVARWEVQGAAYAARGDYDRAIEYYERALELDPNNAWAWEHLGWAYAAR GDYDRAIEYYERALELDPDDAHEWFRLGFAYAARGDYDRAIEYYERALELDPEDELARAE LESARRARQGQAGQGGGSGLNDIFEAQKIEWHECHHHHHH |
| 103 | MGGGGSAAAGDEADRWADLGAAYAARGDYDRAIEYYQRALELDPNNAWAWWDLGVAYAAR GDYDRAIEYYQRALELDPNNTVAWRRSGHAYAARGDYDRATEYYQRALELDPNNELAREQ LEHARRARRGQAGQGGGSGLNDIFEAQKIEWHECHHHHHH |
| 104 | MGGGGSAAAGDEADRWADLGAAYAARGDYDRAIEYYQRALEIDPNNAWAWWDLGVAYAAR GDYDRAIEYYQRALELDPNNAVAWSRLGHAYAARGDYDRAIEYYQRALELDTNNEVARTL LEQARRARRGQAGQGGGSGLNDIFEAQKIEWHECHHHHHH |
| 105 | MGGGGSAAAGDEANRWYYLGAACAARGDYDRAIEYYQRALELDPNNAWAWWDLGVAYAAR GDYDRAIEYYQRALELDPNNAVAWRRLGHAYAARGDYDRATEYYQRALELDPNNELAREQ LEHARRARRGQAGQGGGSGLNDIFEAQKIEWHECHHHHHH |
| 106 | MGGGGSAAAGDEADRWADLGAAYAARGDYDRAIEYYQRALELDPNNAWAWWDLGVAYAAR GDYDRAIEYYQRALELDPNNSVAWRRLGHAYAARGDCDRATEHYQRALELDPNNELAREQ LEHARRARRGQAGQGGGSGLNDIFEAQKIEWHECHHHHHH |
| 107 | MGGGGSAAAGDEANRWYYLGAAYAARGDYDRAIEYYQLALELDPNNAWAWWDLGVAYAAR GDYDRAIEYYQRALELDPNNAVAWRRLGHAYAARGDYDRATEYYQRALELDPNNELAREQ LEHARRARRGQAGQGGGSGLNDIFEAQKIEWHECHHHHHH |
| 108 | MGGGGSAAAGDEANRWYYLGAAYAARGDFDRAIEYYQRALELDPNNAWAWWDLGVAYAAR GDFDRAIEYYQRALELDSNNAVVWRRLGHAYAARGDYDRATEYYQRALELDPNNELAREQ LEHARRARRGQAGQGGGSGLNDIFEAQKIEWHECHHHHHH |
| 109 | MGGGGSAAAGDEADRWADLGAAYAARGYYDRAIEYYQRALELDPNNAWAWWDLGVAYAAR GDYDRAIEYYQRALELDPNNAVVWRRLGHAYAARGDYDRATEYYQRALELDPNNELAREQ LEHARRARRGQAGQGGGSGLNDIFEAQKIEWHECHHHHHH |
| 110 | MGGGGSAAAGDEADRWADLGAAYAARGDYDRAIEYYQRALELDPNNAWAWWDLGVAYAAR GDFDRAIEYYQRALELDSNNAVVWRRLGHAYAARGDYDRATEYYQRALELDPNNELAREQ LEHARRARRGQAGQGGGSGLNDIFEAQKIEWHECHHHHHH |
| 111 | MGGGGSAAAGDEADRWADLGAAYAARGDYDRAIEYYQRALELDPNNAWAWWDLGVAYAAR GDYDRAIEYYQRALELDPNDAVVWRRLGHAYAARGDYDRATEYYQRALELDPNNELAREQ LEHARRARRGQAGQGGGSGLNDIFEAQKIEWHECHHHHHH |
| 112 | MGGGGSAAAGDEADRWADLGAAYAARGDYDRAIEYYQRALELDPNNAWAWWDLGVAYAAR GDYDRAIEYYQRALELDLNNTVVWRRLGHAYAARGDYDRATEYYQRALELDPNNELAREQ LEHARRARRGQAGQGGGSGLNDIFEAQKIEWHECHHHHHH |
| 113 | MGGGGSAAAGDEADRWADLGAAYAARGDYDRAIEYYQRALELDPNNAWAWWDLGVAYAAR GDYDRAIEYYQRALELDPNNSVAWRRSGHAYAARGDYDRATEYYQRALELDPNNELAREQ LEHARRARRGQAGQGGGSGLNDIFEAQKIEWHECHHHHHH |

(continued)

| SEQ ID | AA Sequence |
|---|---|
| | **SEQUENCES** |
| 114 | MGGGGSAAAGDEADRWADLGAAYAARGDYDRAIEYYQRALELDPNNAWAWWDLGVAYAAR GDYDRAIEYYQRALELDPNNAVTWRRLGHAYDARGDYDRATEYYQRALELDPNNELAREQ LEHARRARRGQAGQGGGSGLNDIFEAQKIEWHECHHHHHH |
| 115 | MGGGGSAAAGDEADRWADLGAAYAARGDYDRAIEYYQRALELDPNNGWAWWDLGVAYAAR GDYDRAIEYYQRALELDPNNAVWRRLGHAYAARGDYDRATEYYQRALELDPNNELAREQ LEHARRARRGQAGQGGGSGLNDIFEAQKIEWHECHHHHHH |
| 116 | MGGGGSAAAGDEADRWADLGAAYAARGDYDRAIEYYQRALELDPNNSWAWWDLGVAYAAR GDYDRAIEYYHRASELDPNNAVAWRRLGHAYAARGDYDRATEYYQRALELDPNNELAREQ LEHARRARRGQAGQGGGSGLNDIFEAQKIEWHECHHHHHH |
| 117 | MGGGGSAAAGDEANRWYYLGAAYAARGDYDSAIEYYQRALELDPNNAWAWWDLGVAYAAR GDYDRAIEYYQRALELDPNNAVAWRRLGHAYAARGDYDRATEYYQRALELDPNNELAREQ LEHARRARRGQAGQGGGSGLNDIFEAQKIEWHECHHHHHH |
| 118 | MDEALRWWDQGAAYAARGDYDRAIEYYERALELDPNNAWAWWDLGVAYAARGDYDRAIEY YERALELDPDDAHAWFRLGWAYAARGDYDRAIEYYERALELDPEDELAREELEEARRARQ GQAGQGWAEFRQRLAAIRTRLEALGGSEAELAAFEREIAAFESELQAYAGAGNPEVEALR REAAAIRDELQAYRHNHHHHHHC |
| 119 | MDEAARWEWQGAAYAARGDYDRAIEYYERALELDPNNAWAWWELGVAYAARGELDRAIEY YERALELDPDDAHAWFRLGWAYAARGDYDRAIEYYQRALELDPEDELARRILEDARRARQ GQAGQGWAEFRQRLAAIRTRLEALGGSEAELAAFEREIAAFESELQAYAGAGNPEVEALR REAAAIRDELQAYRHNHHHHHHC |
| 120 | MDEAERWYDLGAAYAARGDYDRAIEYYQRALELDPNNAWAWWELGVAYAARGDYDRAIEY YQRALELDPNNAVAWARLGIAYAARGDYDRAIEYYQRALELDPNNEVARDALEYARRARR GQAGQGWAEFRQRLAAIRTRLEALGGSEAELAAFEREIAAFESELQAYAGAGNPEVEALR REAAAIRDELQAYRHNHHHHHHC |
| 121 | MAQAAAGDEAARWEWQGAAYAARADYDRAIEYYERALELDPNNAWAWWELGVAYAARGEL DRAIEYYERALELDPDDAHAWFRLGWAYAARGDYDRAIEYYQRALELDPEDELAREELEE ARRARQGQAGQGGGSGLNDIFEAQKIEWHECHHHHHHRALELDPNNAWAWWELGVAYAAR GELDRAIEYYERALELDPDDAHAWFRLGWAYAARGDYDRAIEYYQRALELDPEDELAREE LEEARRARQGQAGQGGGSGLNDIFEAQKIEWHECHHHHHH |
| 122 | MGGGGSAAAGDEAARWEVQGAAYAARGDYDRAIEYYERALELDPNNAWAWEHLGWAYAAR GDYDRAIEYYERALELDPDDAHAWFRLGFAYAARGDYDRAIEYYERALELDPEDELARAE LESARRARQGQAGQGWAEFRQRLAAIRTRLEALGGSEAELAAFEREIAAFESELQAYAGA GNPEVEALRREAAAIRDELQAYRHNGQAGQGGGSGLNDIFEAQKIEWHECHHHHHH |
| 123 | MGGGGSAAAGDEAERWWRLGDAYRWRGDYDRAIEYYQRALELDPNNALAWVELGQAYTRR GDYDRAIEYYQRALGLDPNNAFAWHLLGEAYDERGDYDRAIEYYQRALELDPNNEWAREL LEWARRLRRGQAGQGGGSGLNDIFEAQKIEWHECHHHHHH |
| 124 | MGGGGSAEAGDEAVRWEIQGAAYAARGDYDRAIEYYERALELDPNNAWAWWELGVAYAAR GDYDRAIEYYERALELDPDDAHAWFRLGWAYAARGDYDRAIEYYERALELDPEDETARDV LEGARRARQGQAGQGGGSGLNDIFEAQKIEWHECHHHHHH |
| 125 | MGGGGSAAAGDEAERWRRLGHAYHVRGDYDRAIEYYQRALEPDPNNAYAWIELGHAYWRR GDYDRAIEYYQRALELDPNNAEAWVVLGIAYEQRGDYDRAIEYYQRALELDPNNEFARRH LEEARRERRGQAGQGGGSGLNDIFEAQKIEWHECHHHHHH |

(continued)

| SEQ ID | AA Sequence |
|---|---|
| | **SEQUENCES** |
| 126 | MDEAVRWEIQGAAYAARGDYDRAIEYYERALELDPNNAWAWWELGVAYAARGDYDRAIEY YERALELDPDDAHVWFRLGWAYATRGDYDRAIEYYERALELDPEDELARRILEDARRARQ GQAGQGWAEFRQRLAAIRTRLEALGGSEAELAAFEREIAAFESELQAYAGAGNPEVEALR REAAAIRDELQAYRHNHHHHHC |
| 127 | MDEAVRWEIQGAAYAARGDYDRAIEYYERALELDPNNAWAWWELGVAYAARGDYDRAIEY YERALELDPDDAHVWFRLGWAYAVRGDYDRAIEYYERALELDPEDELARRILEDARRARQ GQAGQGWAEFRQRLAAIRTRLEALGGSEAELAAFEREIAAFESELQAYAGAGNPEVEALR REAAAIRDELQAYRHNHHHHHC |
| 128 | MGDEAERWYDLGAAYAARGDFDRAIEYYRRTLELDPNDAWAWWELGVAYAARGDYDRAIE YYQRALELDPNNAVAWARLGIAYATRGDYDRAIEYYQRALELDPNNEVARDALEYARRVR RCHHHHHH |
| 129 | MGHHHHHHDEAERWYDLGAAYAARGDFDRAIEYYRRTLELDPNDAWAWWELGVAYAARGD YDRAIEYYQRALELDPNNAVAWARLGIAYATRGDYDRAIEYYQRALELDPNNEVARDALE YARRVRRC |
| 130 | MAWAEFRQRLAAIRTRLEALGGSEAELAAFEREIAAFESELQAYAGAGNPEVEALRREAA AIRDELQAYRHNDEAERWYDLGAAYAARGDFDRAIEYYRRTLELDPNDAWAWWELGVAYA ARGDYDRAIEYYQRALELDPNNAVAWARLGIAYATRGDYDRAIEYYQRALELDPNNEVAR DALEYARRVRRHHHHHH |
| 131 | MGLAEAAAREAAARAADEAERWYDLGAAYAARGDFDRAIEYYRRTLELDPNDAWAWWELG VAYAARGDYDRAIEYYQRALELDPNNAVAWARLGIAYATRGDYDRAIEYYQRALELDPNN EVARDALEYARRVRRHHHHHH |
| 132 | MGDEAERWYDLGAAYAARGDFDRAIEYYRRTLELDPNDAWAWWELGVAYAARGDYDRAIE YYQRALELDPNNAVAWARLGIAYATRGDYDRAIEYYQRALELDPNNEVARDALEYARRVR RC |
| 133 | MGDEAERWYDLGAAYAARGDYDRATEFYQRALELDPNDAWAWWELGIAYAARGDYDRAIE YYQRALELDPNNAVAWARLGIAYAARGDYDRAIEYYQRALELDPNNEVARDALEYARRAR RCHHHHHH |
| 134 | MGHHHHHHDEAERWYDLGAAYAARGDYDRATEFYQRALELDPNDAWAWWELGIAYAARGD YDRAIEYYQRALELDPNNAVAWARLGIAYAARGDYDRAIEYYQRALELDPNNEVARDALE YARRARRC |
| 135 | MAWAEFRQRLAAIRTRLEALGGSEAELAAFEREIAAFESELQAYAGAGNPEVEALRREAA AIRDELQAYRHNDEAERWYDLGAAYAARGDYDRATEFYQRALELDPNDAWAWWELGIAYA ARGDYDRAIEYYQRALELDPNNAVAWARLGIAYAARGDYDRAIEYYQRALELDPNNEVAR DALEYARRARRHHHHHH |
| 136 | MGLAEAAAREAAARAADEAERWYDLGAAYAARGDYDRATEFYQRALELDPNDAWAWWELG IAYAARGDYDRAIEYYQRALELDPNNAVAWARLGIAYAARGDYDRAIEYYQRALELDPNN EVARDALEYARRARRHHHHHH |
| 137 | MGDEAERWYDLGAAYAARGDYDRATEFYQRALELDPNDAWAWWELGIAYAARGDYDRAIE YYQRALELDPNNAVAWARLGIAYAARGDYDRAIEYYQRALELDPNNEVARDALEYARRAR RC |
| 138 | MGDEAERWYDLGAAYAARGDYDRAIEYYQRALELDPNDAWAWWELGVAYAARGDYDRAIE YYQRALELDPNNAVAWARLGIAYAARGDYDRAIEYYQRALELDPNNEVARDALEYARRAR RCHHHHHH |

(continued)

| SEQ ID | AA Sequence |
|---|---|
| | **SEQUENCES** |
| 139 | MGDEAERWYDLGAAYAARGDYDRAIEYYQRALEHDPNDAWAWWELGVAYAARGDYDRAIE<br>YYQRALELDTNNAVAWARLGIAYAARGDYDRAIEYYQRALELDPNNEVARGALEYARRAR<br>RCHHHHHH |
| 140 | MAWAEFRQRLAAIRTRLEALGGSEAELAAFEREIAAFESELQAYAGAGNPEVEALRREAA<br>AIRDELQAYRHNDEAERWYDLGAAYAARGDYDRAIEYYQRALEHDPNDAWAWWELGVAYA<br>ARGDYDRAIEYYQRALELDTNNAVAWARLGIAYAARGDYDRAIEYYQRALELDPNNEVAR<br>GALEYARRARRHHHHHH |
| 141 | MGLAEAAAREAAARAADEAERWYDLGAAYAARGDYDRAIEYYQRALEHDPNDAWAWWELG<br>VAYAARGDYDRAIEYYQRALELDTNNAVAWARLGIAYAARGDYDRAIEYYQRALELDPNN<br>EVARGALEYARRARRHHHHHH |
| 142 | MGDEAERWYDLGAAYAARGDYDRAIEYYQRALELDPNDAWAWWELGVAYAARGDYDRAIE<br>YYQRALELDLNNAVAWARLGIAYAARGDYDRAIEYYQRALELDPNNEVARDALEYARRAR<br>RCHHHHHH |
| 143 | MGHHHHHHDEAERWYDLGAAYAARGDYDRAIEYYQRALEHDPNDAWAWWELGVAYAARGD<br>YDRAIEYYQRALELDTNNAVAWARLGIAYAARGDYDRAIEYYQRALELDPNNEVARGALE<br>YARRARRC |
| 144 | MGHHHHHHDEAERWYDLGAAYAARGDYDRAIEYYQRALELDPNDAWAWWELGVAYAARGD<br>YDRAIEYYQRALELDLNNAVAWARLGIAYAARGDYDRAIEYYQRALELDPNNEVARDALE<br>YARRARRC |
| 145 | MAWAEFRQRLAAIRTRLEALGGSEAELAAFEREIAAFESELQAYAGAGNPEVEALRREAA<br>AIRDELQAYRHNDEAERWYDLGAAYAARGDYDRAIEYYQRALELDPNDAWAWWELGVAYA<br>ARGDYDRAIEYYQRALELDLNNAVAWARLGIAYAARGDYDRAIEYYQRALELDPNNEVAR<br>DALEYARRARRHHHHHH |
| 146 | MGLAEAAAREAAARAADEAERWYDLGAAYAARGDYDRAIEYYQRALELDPNDAWAWWELG<br>VAYAARGDYDRAIEYYQRALELDLNNAVAWARLGIAYAARGDYDRAIEYYQRALELDPNN<br>EVARDALEYARRARRHHHHHH |
| 147 | MGHHHHHHDEAERWYDLGAAYAARGDYDRAIEYYQRALELDPNDAWAWWELGVAYAARGD<br>YDRAIEYYQRALELDLNNAVAWARLGIAYAARGDYDRAIEYYQRALELDPNNEVARDALE<br>YARRARRAPKC |
| 148 | MGDEAERWYDLGAAYAARGDYDRAIEYYQRALELDPNDAWAWWELGVAYAARGDYDRAIE<br>YYQRALELDLNNAVAWARLGIAYAARGDYDRAIEYYQRALELDPNNEVARDALEYARRAR<br>RC |
| 149 | MDEAAHWEVQGAAYAARGDYDRAIEYYERALELDPNNAWAWEHLGWAYAARGDFDRAIEY<br>YERALELDPDDAHAWFRLGFAYAARGDYDRAIEYYERALELDPEDELARAELESARRARQ<br>GQAGQGWAEFRQRLAAIRTRLEALGGSEAELAAFEREIAAFESELQAYAGAGNPEVEALR<br>REAAIRDELQAYRHNHHHHHHC |
| 150 | MDEAARWEVQGAAYAARGDYDRAIEYYERVLELDPNDAWAWEHLGWAYAARGDYDRAIEY<br>YERALEVDPDDAHAWFRLGFAYAARGDYDRAMEYYERALELDPEDELARAELESARRARQ<br>GQAGQGWAEFRQRLAAIRTRLEALGGSEAELAAFEREIAAFESELQAYAGAGNPEVEALR<br>REAAIRDELQAYRHNHHHHHHC |

(continued)

| SEQ ID | AA Sequence |
|---|---|
| 151 | MDEAARWEVQGAAYAARGDYDRAIEYYERALELDPNNAWAWEHLGWAYAARGDYDRAIEY YERALELDPDDAHSWFRLGFAYAVRGDYDRAIEYYERALELDPEDELARAELESARRARQ GQAGQGWAEFRQRLAAIRTRLEALGGSEAELAAFEREIAAFESELQAYAGAGNPEVEALR REAAAIRDELQAYRHNHHHHHC |
| 152 | MDEVARWEVQGAAYAARGGYDRAIEYYERALELDPNNAWAWEHLGWAYAARGDYDRAIEY YERALELDPDDAHEWFRLGFAYAARGDYDRAIEYYERALELDPEDELARAELESARRARQ GQAGQGWAEFRQRLAAIRTRLEALGGSEAELAAFEREIAAFESELQAYAGAGNPEVEALR REAAAIRDELQAYRHNHHHHHC |
| 153 | &&&&&&&$X^{1}$RW$X^{2}X^{3}$&&&&&&&&&&&&&&&&&&&&&&&&&$X^{4}$AW$X^{5}X^{6}$LG$X^{7}$&&&&&& &&&&&&&&&&&&&&&&&&&$X^{8}$AW$X^{9}X^{10}$LG$X^{11}$&&&&&&&&&&&&&&&&&&&&&&&&&$X^{12}$ AR$X^{13}X^{14}$LE$X^{15}$ |
| 154 | MGDEADRWYDQGAAYAARGDYDRAIEYYERALELDPNDAWAWWELGVAYAARGDYDRAIE YYQRALELDPNDAVAWARLGHAYAARGDYDRAIEYYQRALELDPNNEAARSNLEYARRAA ACGLNDIFEAQKIEWHEHHHHHH |
| 155 | MGDEADRWYYQGAAYAARGDYDRAIEYYERALELDPNNAWAWWELGVAYAARGDYDRAIE YYQRALELDPNDAVAWARLGHAYAARGDYDRAIEYYQRALELDPNNEAARSNLEYARRAA ACGLNDIFEAQKIEWHEHHHHHH |
| 156 | MGDEADRWYDQGAAYAARGDYDRAIEYYQRALELDPNNAWAWWELGVAYAARGDYDRAIE YYQRALELDPNDAVAWSRLGHAYAARGDYDRAIEHYQRALELDPNNEVARHLLEVARRAA ACGLNDIFEAQKIEWHEHHHHHH |
| 157 | MGDEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVARRAA ACGLNDIFEAQKIEWHEHHHHHH |
| 158 | MGDEADRWYDQGAAYAARGDYDRAIEYYERALELDPNNAWAWWELGVAYAARGDYDRAIE YYQRALELDPNDAVAWARLGHAYAARGDYDRAIEYYQRALELDPNNEAARSNLEYARRAA ACGLNDIFEAQKIEWHEHHHHHH |
| 159 | MGDEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVASRAA ACGLNDIFEAQKIEWHEHHHHHH |
| 160 | MGDEGDRWYYQGAAYAARGDYDRAIEYYERALELDPNNAWAWWELGVAYAARGDYDRAIE YYQRALELDPNNTVAWARLGHAYAARGDYDRAIEYYQRALELDPNNEAARSNLEYSPPAA ACGLNDIFEAQKIEWHEHHHHHH |
| 161 | MGDEADRWYYQGAAYAARGDYDRALEYYERALELDPSNAWAWWELGVAYAARGDYDRAIE YYQRALELDPNDAVAWARLGHAYAARGDYDRAIEYYQRALELDPNNEAARSNLEYARRAA ACGLNDIFEAQKIEWHEHHHHHH |
| 162 | MGDEADRWYYQGAAYAARGDYDRAIEYYERALELDPNNAWAWWELGVAYAARGDYDRAIE YYQRALELDPNNTVAWARLGHAYAARGDYDRAIEYYQRALELDPNNEAARSNLEYARRAA ACGLNDIFEAQKIEWHEHHHHHH |
| 163 | MGDEADRWYYRGAAYAARGDYDRAIEYYERALELDPNNAWAWWELGVAYAARGDYDRAIE YYQRALELDPNNTVAWARLGHAYAARGDYDRAIEYYQRALELDPNNEAARSNLEYARRAA ACGLNDIFEAQKIEWHEHHHHHH |

(continued)

| SEQ ID | AA Sequence |
|---|---|
| | **SEQUENCES** |
| 164 | MGDEADRWYDQGAAYAARGDYDRAIEYYERALELDPNNAWAWWELGAAYAARGDYDRAIE<br>YYQRALELDPNNAVAWSRLGHAYAARGDYDRAIEYYQRALELDPNNEVARHLLEVARRAA<br>ACGLNDIFEAQKIEWHEHHHHHH |
| 165 | MGDEAERWYDLGAAFAARGDYDRAIEYYQRALELDPNDAWAWWELGVAYAARGDYDRAIE<br>YYQRALELDPNNAVAWFRLGWAYAERGDYDRAIEYYQRALELDPNNELARSELENARRAA<br>ACGLNDIFEAQKIEWHEHHHHHH |
| 166 | MGDEAERWYDLGAAYAARGDYDRAIEYYQRALELDPNDAWAWWELGVAYAARGDYDRAIE<br>YYQRALELDPNNAVAWFRLGWAYAARGDYDRAIEYYQRALELDPNNELARSELENARRAA<br>ACGLNDIFEAQKIEWHEHHHHHH |
| 167 | MGDEAERWYDLGAAYAARGDYDRAIEYYQRALELDPNDAWAWWELGVAYAARGDYDRAIE<br>YYQRALELDPNNAVAWFRLGWAYAERGDYDRAIEYYQRALELDPNNELARSELENARRAA<br>ACGLNDIFEAQKIEWHEHHHHHH |
| 168 | MGDEAERWYDLGAAYAARGDYDRAIEYYQRALELDPNNAWAWWELGVAYAARGDYDRAIE<br>YYQRALELDPNDAVAWFRLGWAYAARGDYDRAIEYYQRALELDPNNELARSELVNARRAA<br>ACGLNDIFEAQKIEWHEHHHHHH |
| 169 | MGDEAERWYDLGXAYAARGDYDRAIEYYQRALELDPNDAWAWWELGVAYAARGDYDRAIE<br>YYQRALELDPNNAVAWFRLGWAYAERGDYDRAIEYYQRALELDPNNELARSELENARRAA<br>ACGLNDIFEAQKIEWHEHHHHHH |
| 170 | MGDEAERWYDLGAAYAARGDFDRAIEYYQRALELDPNDAWAWWELGVAYAARGDYDRAIE<br>YYQRALELDPNNAVAWFRLGWAYAERGDYDRAIEYYQRALELDPNNELARSELENARRAA<br>ACGLNDIFEAQKIEWHEHHHHHH |
| 171 | MGDEAERWYDLGAAYAARGDYDRAIEYYQRALELDPNDAWAWWELGVAYAARGDYDRAIE<br>YYQRTLELDPNNAVAWFRLGWAYAERGDYDRAIEYYQRALELDPNNELARSELENARRAA<br>ACGLNDIFEAQKIEWHEHHHHHH |
| 172 | MGDEAERWYDLGAAYAARGDYDRAIEYYQRALELDPNNAWAWWELGVAYAARGDYDRAIE<br>YYQRALELDPNDAVAWFRLGWAYAARGDYDRAIEYYQRALELDPNNELARSELENARRAA<br>ACGLNDIFEAQKIEWHEHHHHHH |
| 173 | MGDEAERWYDLGAAYAARGDYDRAIEYYQRALELDPNDAWAWWELGVAYAARGDYDRAIE<br>YYQRALELDPNNAVAWFRLGWAYAARGDYDRAIEYYQRALELDPNNELARSELVNARRAA<br>ACGLNDIFEAQKIEWHEHHHHHH |
| 174 | MGDEASRWYHLGVAYAARGDYDRAIEYYQRALELDPNNAWAWWELGVAYAARGDYDRAIE<br>YYQRALELDPNNAIAWSRLGHAYAARGDYDRAIEYYQRALELDPNNEVARHLLEYARRAA<br>ACGLNDIFEAQKIEWHEHHHHHH |
| 175 | MGDEADRWYDQGAAYAARGDYDRAIEYYQRALELDPNNAWAWWELGVAYAARGDYDRAIE<br>YYQRALELDPNDAVAWSRLGHAYAARGDYDRAIEYYQRALELDPNNEVARHLLEVARRLE<br>DEADRWYDQGAAYAARGDYDRAIEYYQRALELDPNNAWAWWELGVAYAARGDYDRAIEYY |
| | QRALELDPNDAVAWSRLGHAYAARGDYDRAIEYYQRALELDPNNEVARHLLEVYRRAAAH<br>HHHHHC |

(continued)

| SEQUENCES | |
| --- | --- |
| SEQ ID | AA Sequence |
| 176 | MGDEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVARRAA AHHHHHHC |
| 177 | MGDEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVARRLE GGGGSDEADRWYDQGAAYAARGDYDRAIEYYQRALELDPNNAWAWWELGVAYAARGDYDR AIEYYQRALELDPNDAVAWSRLGHAYAARGDYDRAIEYYQRALELDPNNEVARHLLEVAR RAAAHHHHHHC |
| 178 | MAQHDKIQQAADKEILHLPNLTEEQRNKFRQSLRDDPSVSAEILAEAKKLNDAQAPKAMG DEADRWYDQGAAYAARGDYDRAIEYYQRALELDPNNAWAWWELGVAYAARGDYDRAIEYY QRALELDPNDAVAWSRLGHAYAARGDYDRAIEYYQRALELDPNNEVARHLLEVARRAAAH HHHHHC |
| 179 | MAQHDKIQQAADKEILHLPNLTEEQRNKFRQSLRDDPSVSAEILAEAKKLNDAQAPKAQH DKIQQAADKEILHLPNLTEEQRNKFRQSLRDDPSVSAEILAEAKKLNDAQAPKAMGDEAD RWYDQGAAYAARGDYDRAIEYYQRALELDPNNAWAWWELGVAYAARGDYDRAIEYYQRAL ELDPNDAVAWSRLGHAYAARGDYDRAIEYYQRALELDPNNEVARHLLEVARRAAAHHHHH HC |
| 180 | MGDEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVARRAA AAQHDKIQQAADKEILHLPNLTEEQRNKFRQSLRDDPSVSAEILAEAKKLNDAQAPKHHH HHHC |
| 181 | MGDEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVARRAA AAQHDKIQQAADKEILHLPNLTEEQRNKFRQSLRDDPSVSAEILAEAKKLNDAQAPKAQH DKIQQAADKEILHLPNLTEEQRNKFRQSLRDDPSVSAEILAEAKKLNDAQAPKHHHHHHC |
| 182 | MGDEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVARRAA ADEADRWYDQGAAYAARGDYDRAIEYYQRALELDPNNAWAWWELGVAYAARGDYDRAIEY YQRALELDPNDAVAWSRLGHAYAARGDYDRAIEYYQRALELDPNNEVARHLLEVARRAAA GLNDIFEAQKIEWHEHHHHHHC |
| 183 | MGDEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVARRAA ADEADRWYDQGAAYAARGDYDRAIEYYQRALELDPNNAWAWWELGVAYAARGDYDRAIEY YQRALELDPNDAVAWSRLGHAYAARGDYDRAIEYYQRALELDPNNEVARHLLEVARRAMG DEADRWYDQGAAYAARGDYDRAIEYYQRALELDPNNAWAWWELGVAYAARGDYDRAIEYY QRALELDPNDAVAWSRLGHAYAARGDYDRAIEYYQRALELDPNNEVARHLLEVARRAAAD EADRWYDQGAAYAARGDYDRAIEYYQRALELDPNNAWAWWELGVAYAARGDYDRAIEYYQ RALELDPNDAVAWSRLGHAYAARGDYDRAIEYYQRALELDPNNEVARHLLEVARRAAAGL NDIFEAQKIEWHEHHHHHHC |
| 184 | MADEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVAQRAA AAQHDKIQQAADKEILHLPNLTEEQRNKFRQSLRDDPSVSAEILAEAKKLNDAQAPKC |

(continued)

| SEQUENCES | |
|---|---|
| **SEQ ID** | **AA Sequence** |
| 185 | MADEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVAQAPK VDAKFDKEQQAARAEILHLPNLTEEQRNKFIQSLKDDPSQSANLLAEAKKLNDAQAPKVD AKFDKEQQAARAEILHLPNLTEEQRNKFIQSLKDDPSQSANLLAEAKKLNDAQAPKVDAK FDKEQQAARAEILHLPNLTEEQRNKFIQSLKDDPSQSANLLAEAKKLNDAQAPKC |
| 186 | MAQHDKIQQAADKEILHLPNLTEEQRNKFRQSLRDDPSVSAEILAEAKKLNDAQAPKADE ADRWYDQGAAYAARGDYDRAIEYYQRALELDPNNAWAWWELGVAYAARGDYDRAIEYYQR ALELDPNDAVAWSRLGHAYAARGDYDRAIEYYQRALELDPNNEVARHLLEVAQAPKAQHD KIQQAADKEILHLPNLTEEQRNKFRQSLRDDPSVSAEILAEAKKLNDAQAPKIAAQHDKI QQAADKEILHLPNLTEEQRNKFRQSLRDDPSVSAEILAEAKKLNDAQAPKC |
| 187 | MADEADRWYDQGAAYAARGDYDRAIEYYQRALELDPNNAWAWWELGVAYAARGDYDRAIE YYQRALELDPNDAVAWSRLGHAYAARGDYDRAIEYYQRALELDPNNEVARHLLEVAQRAA AAQHDKIQQAADKEILHLPNLTEEQRNKFRQSLRDDPSVSAEILAEAKKLNDAQAPKAQH DKIQQAADKEILHLPNLTEEQRNKFRQSLRDDPSVSAEILAEAKKLNDAQAPKC |
| 188 | MGDEADRWYDQGAAYAARGDYDRAIEYYQRALELDPNNAWAWWELGVAYAARGDYDRAIE YYQRALELDPNDAVAWSRLGHAYAARGDYDRAIEHYQRALELDPNNEVARHLLEVARRAA AHHHHHHC |
| 189 | MGDEADRWYDQGAAYAARGDYDRAIEYYQRALELDPNNAWAWWELGVAYAARGDYDRAIE YYQRALELDPNDAVAWSRLGHAYAARGDYDRAIEHYQRALELDPNNEVARHLLEVARRLE DEADRWYDQGAAYAARGDYDRAIEYYQRALELDPNNAWAWWELGVAYAARGDYDRAIEYY QRALELDPNDAVAWSRLGHAYAARGDYDRAIEHYQRALELDPNNEVARHLLEVSRRAAAH HHHHHC |
| 190 | MGDEADRWYYQGAAYAARGDYDRAIEYYERALELDPNNAWAWWELGVAYAARGDYDRAIE YYQRALELDPNDAVAWARLGHAYAARGDYDRAIEYYQRALELDPNNEAARSNLEYARRAA AHHHHHHC |
| 191 | MGDEADRWYYQGAAYAARGDYDRAIEYYERALELDPNNAWAWWELGVAYAARGDYDRAIE YYQRALELDPNDAVAWARLGHAYAARGDYDRAIEYYQRALELDPNNEAARSNLEYARRLE DEADRWYYQGAAYAARGDYDRAIEYYERALELDPNNAWAWWELGVAYAARGDYDRAIEYY QRALELDPNDAVAWARLGHAYAARGDYDRAIEYYQRALELDPNNEAARSNLEYARRAAAH HHHHRC |
| 192 | MAQHDKIQQAADKEILHLPNLTEEQRNKFRQSLRDDPSVSAEILAEAKKLNDAQAPKDEA DRWYYQGAAYAARGDYDRAIEYYERALELDPNNAWAWWELGVAYAARGDYDRAIEYYQRA LELDPNDAVAWARLGHAYAARGDYDRAIEYYQRALELDPNNEAARSNLEYARRAAAHHHH HHC |
| 193 | MAQHDKIQQAADKEILHLPNLTEEQRNKFRQSLRDDPSVSAEILAEAKKLNDAQAPKDEA DRWYYQGAAYAARGDYDRAIEYYERALELDPNNAWAWWELGVAYAARGDYDRAIEYYQRA LELDPNDAVAWARLGHAYAARGDYDRAIEYYQRALELDPNNEAARSNLEYARRAAAQHDK IQQAADKEILHLPNLTEEQRNKFRQSLRDDPSVSAEILAEAKKLNDAQAPKHHHHHHC |
| 194 | MGDEADRWYDQGAAYAARGDYDRAIEYYERALELDPNDAWAWWELGVAYAARGDYDRAIE YYQRALELDPNDAVAWARLGHAYAARGDYDRAIEYYQRALELDPNNEAARSNLEYARRAA AHHHHHHC |

(continued)

| SEQUENCES | |
|---|---|
| **SEQ ID** | **AA Sequence** |
| 195 | MAQHDKIQQAADKEILHLPNLTEEQRNKFRQSLRDDPSVSAEILAEAKKLNDAQAPKDEA DRWYDQGAAYAARGDYDRAIEYYERALELDPNDAWAWWELGVAYAARGDYDRAIEYYQRA LELDPNDAVAWARLGHAYAARGDYDRAIEYYQRALELDPNNEAARSNLEYARRAAAHHHH HHC |
| 196 | MGDEADRWYDQGAAYAARGDYDRAIEYYERALELDPNDAWAWWELGVAYAARGDYDRAIE YYQRALELDPNDAVAWARLGHAYAARGDYDRAIEYYQRALELDPNNEAARANLEYARRAA AGLNDIFEAQKIEWHEHHHHHHC |
| 197 | MGDEADRWYDQGAAYAARGDYDRAIEYYERALELDPNDAWAWWELGVAYAARGDYDRAIE YYQRALELDPNDAVAWARLGHAYAARGDYDRAIEYYQRALELDPNNEAARSALEYARRAA AGLNDIFEAQKIEWHEHHHHHHC |
| 198 | MGDEADRWYDQGAAYAARGDYDRAIEYYERALELDPNDAWAWWELGVAYAARGDYDRAIE YYQRALELDPNDAVAWARLGHAYAARGDYDRAIEYYQRALELDPNNEAARSNLEAARRAA AGLNDIFEAQKIEWHEHHHHHHC |
| 199 | MGDEADRWYDQGAAYAARGDYDRAIEYYERALELDPNDAWAWWELGVAYAARGDYDRAIE YYQRALELDPNDAVAWARLGHAYAARGDYDRAIEYYQRALELDPNNEAARAALEYARRAA AGLNDIFEAQKIEWHEHHHHHHC |
| 200 | MGDEADRWYDQGAAYAARGDYDRAIEYYERALELDPNDAWAWWELGVAYAARGDYDRAIE YYQRALELDPNDAVAWARLGHAYAARGDYDRAIEYYQRALELDPNNEAARANLEAARRAA AGLNDIFEAQKIEWHEHHHHHHC |
| 201 | MGDEADRWYDQGAAYAARGDYDRAIEYYERALELDPNDAWAWWELGVAYAARGDYDRAIE YYQRALELDPNDAVAWARLGHAYAARGDYDRAIEYYQRALELDPNNEAARSALEAARRAA AGLNDIFEAQKIEWHEHHHHHHC |
| 202 | MGDEADRWYDQGAAYAARGDYDRAIEYYERALELDPNDAWAWWELGVAYAARGDYDRAIE YYQRALELDPNDAVAWARLGHAYAARGDYDRAIEYYQRALELDPNNEAARAALEAARRAA AGLNDIFEAQKIEWHEHHHHHHC |
| 203 | MGDEADRWYDQGAAYAARGDYDRAIEYYERALELDPNNAWAWWELGVAYAARGDYDRAIE YYQRALELDPNNAVAWARLGHAYAARGDYDRAIEYYQRALELDPNNEAARSNLEYARRAA ACGLNDIFEAQKIEWHEHHHHHH |
| 204 | MGDEADRWYDQGAAYAARGDYDRAIEYYERALELDPNNAWAWWELGTAYAARGDYDRAIE YYQRALELDPNNAVAWSRLGHAYAARGDYDRAIEYYQRALELDPNNEVARHLLEVARRAA ACGLNDIFEAQKIEWHEHHHHHH |

**Claims**

1. An affinity ligand comprising an amino acid sequence represented by the formula, from N-terminus to C-terminus,
[A]-AERWYDLGAAYAARGDX$_{17a}$DRAX$_{21a}$EX$_{23a}$YX$_{25a}$RX$_{27a}$LEX$_{30a}$DPND-

AWAWWELGX$_{9b}$AYAARGDYDRAIEYYQRALELDX$_{32b}$NN- AVAWARLGIAYAX$_{13c}$RGDYDRAIEYYQRALELDPNN- EVARX$_{5d}$ALEYARRX$_{13d}$-[B]

wherein

X$_{17a}$ is F or Y; X$_{21a}$ is I or T; X$_{23a}$ is Y or F; X$_{25a}$ is R or Q; X$_{27a}$ is T or A; and

$X_{30a}$ is L or H;
wherein
$X_{9b}$ is V or I; and $X_{32b}$ is L, P or T;
wherein
$X_{13c}$ is T or A;
wherein
$X_{5d}$ is D or G; and $X_{13d}$ is A or V; and
wherein
[A] is present or absent, and when present comprises M, MDE, MGGGGSAAAGDE, MGDE, MGHHHHHHDE, MGLAEAAAREAAARAADE, or MAWAEFRQRLAAIRTRLEALGGSEAELAAFEREIAAFESELQAYAGAGNPE VEALRR EAAAIRDELQAYRHNDE; and
[B] is present or absent, and when present comprises a cysteine, RRC, RRCHHHHHH, or RRGQAGQGGGSGLNDIFEAQKIEWHECHHHHHH.

2. The affinity ligand of Claim 1, wherein $X_{17a}$ is F; $X_{21a}$ is I; $X_{23a}$ is Y; $X_{25a}$ is R; $X_{27a}$ is T; $X_{30a}$ is L; $X_{9b}$ is V; $X_{32b}$ is P; $X_{13c}$ is T; $X_{5d}$ is D; and $X_{13d}$ is V, or

wherein $X_{17a}$ is Y; $X_{21a}$ is T; $X_{23a}$ is F; $X_{25a}$ is Q; $X_{27a}$ is A; $X_{30a}$ is L; $X_{9b}$ is I; $X_{32b}$ is P; $X_{13c}$ is A; $X_{5d}$ is D; and $X_{13d}$ is A, or
wherein $X_{17a}$ is Y; $X_{21a}$ is I; $X_{23a}$ is Y; $X_{25a}$ is Q; $X_{27a}$ is A; $X_{30a}$ is H; $X_{9b}$ is V; $X_{32b}$ is T; $X_{13c}$ is A; $X_{5d}$ is G; and $X_{13d}$ is A, or
wherein $X_{17a}$ is Y; $X_{21a}$ is I; $X_{23a}$ is Y; $X_{25a}$ is Q; $X_{27a}$ is A; $X_{30a}$ is L; $X_{9b}$ is V; $X_{32b}$ is L; $X_{13c}$ is A; $X_{5d}$ is D; and $X_{13d}$ is A.

3. The affinity ligand of claims 1 or 2, wherein [A] is MGHHHHHHDE and [B] is RRC or wherein [A] is MGLAEAAAREAAARAADE and [B] is RRCHHHHHH.

4. The affinity ligand of Claim 2 or 3, wherein said ligand comprises any one of SEQ ID NOS: 89, 128, 130, 131 or 132. (Ligand 129 group),
or

wherein said ligand comprises any one of SEQ ID NOS: 90, 133, 135, 136 or 137. (Ligand 134 group),
or
wherein said ligand comprises any one of SEQ ID NOS: 139, 140, 141 or 143. (Ligand 141 group),
or
wherein said ligand comprises any one of SEQ ID NOS: 86, 91, 138, 142, 144, 145, 146, 147 or 148. (Ligand 144 group),
or
wherein said ligand comprises any one of SEQ ID NOS: 129, 134, 141 or 144, and/ or which affinity ligand further comprises a C-terminal cysteine or lysine.

5. A multimer comprising a plurality of affinity ligands according to any one of the preceding claims, preferably which multimer is a dimer, trimer, tetramer, pentamer, hexamer, heptamer, octamer or nonamer.

6. The affinity ligand or multimer of any one of claims 1-5, wherein said ligand or multimer further comprises at least one heterologous agent operably linked to said affinity ligand to thereby form a conjugate or a fusion protein, preferably wherein said heterologous agent is selected from the group consisting of one or more small molecule diagnostic or therapeutic agents; a peptide tag, a DNA, RNA, or hybrid DNA-RNA molecule; traceable marker; radioactive agent; an antibody; a single chain variable domain; and an immunoglobulin fragment.

7. A separation matrix comprising at least one affinity ligand of any one of claims 1-4, or at least one multimer of claim 5 or 6, preferably wherein the affinity ligands or the multimers are coupled to a solid support, more preferably wherein the affinity ligands or multimers are coupled to the solid support via thiol linkages, preferably wherein the solid support is a chromatography resin or matrix, more preferably wherein the solid support is a cross-linked agarose matrix.

8. A method of isolating an antibody comprising a CH1 domain, or a fragment thereof comprising a CH1 domain, which comprises contacting said antibody or said fragment with a separation matrix of claim 7 and recovering said antibody or fragment thereof,

preferably

which comprises (a) contacting a separation matrix of claim 7 with a composition comprising said antibody or said fragment, (b) washing said separation matrix with a washing buffer, (c) eluting said antibody or said fragment from the separation matrix with an elution buffer, and (d) recovering said antibody or said fragment,
preferably
which further comprises (e) treating said separation matrix with an alkaline cleaning solution for a time sufficient to clean said matrix of residual material and to regenerate at least 80% of the antibody- or the antibody fragment-binding capacity of said separation matrix
more preferably
wherein the alkaline cleaning solution comprises from 0.1 M NaOH to 0.5 M NaOH, even more preferably
wherein said separation matrix retains at least 80% of its antibody- or the antibody fragment-binding capacity when steps (a)-(e) are repeated at least 10 times, and/or wherein steps (a)-(e) are repeated at least 10 times with a single batch of separation matrix.

9. A nucleic acid or vector encoding an affinity ligand of any one of claims 1-4 or a multimer of claim 5 or 6.

10. An expression vector comprising the nucleic acid or vector of claim 9, wherein the coding region of said affinity ligand is operably linked to one or more expression control elements.

11. A host cell which comprises a nucleic acid or vector of claim 9 or 10, preferably wherein
the host cell is *E. coli* or *P. pastoris.*

12. A method of making a separation matrix comprising conjugating a ligand according to any of claims 1-4 or a multimer of claim 5 or 6 to a solid surface.

13. An affinity agent comprising a ligand comprising a TPR ligand comprising 7 helices in which the 1st helix comprises the sequence SEQ ID No: 1

$X^1RWX^2X^3$, where $X^1$ is A, D, E, G, N, Q, S or V, ; $X^2$ is A, E, W or Y; $X^3$ is D, G, H, I, N, T, V, W or Y;
The 3rd helix comprises the sequence SEQ ID No: 2
$X^4AWX^5X^6LGX^7$ where $X^4$ is W; $X^5$ is E or W; $X^6$ is D, E or H; $X^7$ is T, V, W;
The 5th helix comprises the sequence SEQ ID No: 3
$X^8AWX^9X^{10}LGX^{11}$, where $X^8$ is A, H, I, T or V; $X^9$ is A, F, N, S or R; $X^{10}$ is R; $X^{11}$ is F, I, H or W;
The 7th helix comprises the sequence SEQ ID No: 4
$X^{12}ARX^{13}X^{14}LEX^{15}$, where $X^{12}$ is A, L, T or V; $X^{13}$ is A, D, E, H, Q, R, S or Y; $X^{14}$ is A, E, I, L, N or Q; $X^{15}$ is A, D, E, H, ,N R, S, V or Y preferably that binds to monoclonal antibodies, antibody fragments and antibody conjugates containing a CH1 domain.

14. The affinity agent of claim 13 that comprises multimer polypeptides comprising at least two subunits, each subunit being a polypeptide according to claim 13 preferably with one or more of the following:

where the sub-units are not all the same;
wherein the ligand is attached to a solid surface;
wherein the solid surface is a resin or bead;
wherein the solid surface is a membrane;
wherein the solid surface is a monolith;
wherein the ligand is conjugated to the solid surface via a linker;
that is used for purification of a monoclonal antibodies, antibody fragments and antibody conjugates containing a CH1 domain.

15. A method of making an affinity agent comprising conjugating a ligand according to any of claims 1-6 to a solid surface.

**Patentansprüche**

1. Affinitätsligand, umfassend eine Aminosäuresequenz, dargestellt durch die Formel, vom N-Terminus zum C-Terminus,

[A]-AERWYDLGAAYAARGDX$_{17a}$DRAX$_{21a}$EX$_{23a}$YX$_{25a}$RX$_{27a}$LEX$_{30a}$DPND-AWAW-WELGX$_{9b}$AYAARGDYDRAIEYYQRALELDX$_{32b}$NN-AVAWARLGIAYAX$_{13c}$RGDYDRAIEYYQRALELDPNN-EVARX$_{5d}$ALEYARRX$_{13d}$-[B]

worin

X$_{17a}$ F oder Y ist; X$_{21a}$ I oder T ist; X$_{23a}$ Y oder F ist; X$_{25a}$ R oder Q ist; X$_{27a}$ T oder A ist; und X$_{30a}$ L oder H ist;
worin
X$_{9b}$ V oder I ist; und X$_{32b}$ L, P oder T ist;
worin
X$_{13c}$ T oder A ist;
worin
X$_{5d}$ D oder G ist; und X$_{13d}$ A oder V ist; und
worin
[A] vorhanden ist oder nicht vorhanden ist und, wenn vorhanden, M, MDE, MGGGGSAAAGDE, MGDE, MGHHHHHHDE, MGLAEAAAREAAARAADE oder MAWAEFRQRLAAIRTRLEALGGSEAELAAFEREIAAFE SELQAYAGAGNPEVEAL RR EAAAIRDELQAYRHNDE umfasst; und
[B] vorhanden ist oder nicht vorhanden ist und, wenn vorhanden, ein Cystein, RRC, RRCHHHHHH oder RRGQAGQGGGSGLNDIFEAQKIEWHECHHHHHH umfasst.

2. Affinitätsligand nach Anspruch 1, wobei X$_{17a}$ F ist; X$_{21a}$ I ist; X$_{23a}$ Y ist; X$_{25a}$ R ist; X$_{27a}$ T ist; X$_{30a}$ L ist; X$_{9b}$ V ist; X$_{32b}$ P ist; X$_{13c}$ T ist; X$_{5d}$ D ist; und X$_{13d}$ V ist, oder

wobei X$_{17a}$ Y ist; X$_{21a}$ T ist; X$_{23a}$ F ist; X$_{25a}$ Q ist; X$_{27a}$ A ist; X$_{30a}$ L ist; X$_{9b}$ I ist; X$_{32b}$ P ist; X$_{13c}$ A ist; X$_{5d}$ D ist; und X$_{13d}$ A ist, oder
wobei X$_{17a}$ Y ist; X$_{21a}$ I ist; X$_{23a}$ Y ist; X$_{25a}$ Q ist; X$_{27a}$ A ist; X$_{30a}$ H ist; X$_{9b}$ V ist; X$_{32b}$ T ist; X$_{13c}$ A ist; X$_{5d}$ G ist; und X$_{13d}$ A ist, oder
wobei X$_{17a}$ Y ist; X$_{21a}$ I ist; X$_{23a}$ Y ist; X$_{25a}$ Q ist; X$_{25a}$ A ist; X$_{30a}$ L ist; X$_{9b}$ V ist; X$_{32b}$ L ist; X$_{13c}$ A ist; X$_{5d}$ D ist; und X$_{13d}$ A ist.

3. Affinitätsligand nach Anspruch 1 oder 2, wobei [A] MGHHHHHHDE und [B] RRC ist oder wobei [A] MGLAEAAAREAAARAADE und [B] RRCHHHHHH ist.

4. Affinitätsligand nach Anspruch 2 oder 3, wobei der Ligand eine der SEQ ID NOS: 89, 128, 130, 131 oder 132 umfasst (Ligand-129-Gruppe),
oder

wobei der Ligand eine der SEQ ID NOS: 90, 133, 135, 136 oder 137 umfasst (Ligand-134-Gruppe),
oder
wobei der Ligand eine der SEQ ID NOS: 139, 140, 141 oder 143 umfasst (Ligand-141-Gruppe),
oder
wobei der Ligand eine der SEQ ID NOS: 86, 91, 138, 142, 144, 145, 146, 147 oder 148 umfasst (Ligand-144-Gruppe),
oder
wobei der Ligand eine der SEQ ID NOS: 129, 134, 141 oder 144 umfasst, und/oder der Affinitätsligand ferner ein C-terminales Cystein oder Lysin umfasst.

5. Multimer, umfassend eine Vielzahl von Affinitätsliganden nach einem der vorstehenden Ansprüche, wobei das Multimer vorzugsweise ein Dimer, Trimer, Tetramer, Pentamer, Hexamer, Heptamer, Octamer oder Nonamer ist.

6. Affinitätsligand oder Multimer nach einem der Ansprüche 1 bis 5, wobei der Ligand oder das Multimer ferner mindestens ein heterologes Mittel umfasst, das funktionsfähig mit dem Affinitätsliganden verknüpft ist, um dadurch ein Konjugat oder ein Fusionsprotein zu bilden, wobei das heterologe Mittel vorzugsweise aus der Gruppe ausgewählt ist, bestehend aus einem oder mehreren niedermolekularen diagnostischen oder therapeutischen Mitteln; einem Peptid-Tag, einem DNA-, RNA- oder hybriden DNA-RNA-Molekül; rückverfolgbarem Marker; radioaktivem Mittel; einem Antikörper; einer variable Domäne der schweren Kette; und einem Immunglobulinfragment.

7. Trennmatrix, umfassend mindestens einen Affinitätsliganden nach einem der Ansprüche 1 bis 4 oder mindestens ein Multimer nach Anspruch 5 oder 6, wobei die Affinitätsligenden oder Multimere vorzugsweise an einen festen Träger gekoppelt sind, wobei die Affinitätsligenden oder Multimere mehr bevorzugt über Thiolverknüpfungen an den festen Träger gekoppelt sind, wobei der feste Träger vorzugsweise ein Chromatographieharz oder eine Chromatographiematrix ist, wobei der feste Träger mehr bevorzugt eine vernetzte Agarosematrix ist.

8. Verfahren zum Isolieren eines Antikörpers, der eine CH1-Domäne umfasst, oder eines Fragments davon, das eine CH1-Domäne umfasst, das das Inkontaktbringen des Antikörpers oder des Fragments mit einer Trennmatrix nach Anspruch 7 und das Rückgewinnen des Antikörpers oder des Fragments davon umfasst, vorzugsweise

das (a) Inkontaktbringen einer Trennmatrix nach Anspruch 7 mit einer Zusammensetzung, die den Antikörper oder das Fragment umfasst, (b) Waschen der Trennmatrix mit einem Waschpuffer, (c) Eluieren des Antikörpers oder des Fragments von der Trennmatrix mit einem Elutionspuffer und (d) Rückgewinnen des Antikörpers oder des Fragments umfasst,
vorzugsweise
das ferner (e) Behandeln der Trennmatrix mit einer alkalischen Reinigungslösung für eine Zeit, die ausreicht, um die Matrix von Restmaterial zu reinigen und mindestens 80 % der Antikörper- oder der Antikörperfragment-Bindungskapazität der Trennmatrix zu regenerieren, umfasst
mehr bevorzugt
wobei die alkalische Reinigungslösung 0,1 M NaOH bis 0,5 M NaOH umfasst, noch mehr bevorzugt
wobei die Trennmatrix mindestens 80 % ihrer Antikörper- oder der Antikörperfragment-Bindungskapazität behält, wenn die Schritte (a)-(e) mindestens 10-mal wiederholt werden, und/oder wobei die Schritte (a)-(e) mindestens 10-mal mit einer einzigen Charge der Trennmatrix wiederholt werden.

9. Nukleinsäure oder Vektor, die bzw. der einen Affinitätsliganden nach einem der Ansprüche 1 bis 4 oder ein Multimer nach Anspruch 5 oder 6 kodiert.

10. Expressionsvektor, umfassend die Nukleinsäure oder den Vektor nach Anspruch 9, wobei die kodierende Region des Affinitätsliganden funktionsfähig mit einem oder mehreren Expressionskontrollelementen verknüpft ist.

11. Wirtszelle, die eine Nukleinsäure oder einen Vektor nach Anspruch 9 oder 10 umfasst, wobei die Wirtszelle vorzugsweise *E. coli* oder *P. pastoris* ist.

12. Verfahren zur Herstellung einer Trennmatrix, umfassend Konjugieren eines Liganden nach einem der Ansprüche 1 bis 4 oder eines Multimers nach Anspruch 5 oder 6 an eine feste Oberfläche.

13. Affinitätsmittel, umfassend einen Liganden, der einen TPR-Liganden mit 7 Helixen umfasst, in denen die 1. Helix die Sequenz SEQ ID No: 1

$X^1RWX^2X^3$ umfasst, wobei $X^1$ A, D, E, G, N, Q, S oder V ist; $X^2$ A, E, W oder Y ist; $X^3$ D, G, H, I, N, T, V, W oder Y ist;
die 3. Helix die Sequenz SEQ ID No: 2
$X^4AWX^5X^6LGX^7$ umfasst, wobei $X^4$ W ist; $X^5$ E oder W ist; $X^6$ D, E oder H ist; $X^7$ T, V, W ist;
die 5. Helix die Sequenz SEQ ID No: 3
$X^8AWX^9X^{10}LGX^{11}$ umfasst, wobei $X^8$ A, H, I, T oder V ist; $X^9$ A, F, N, S oder R ist; $X^{10}$ R ist; $X^{11}$ F, I, H oder W ist;
die 7. Helix die Sequenz SEQ ID No: 4
$X^{12}ARX^{13}X^{14}LEX^{15}$ umfasst, wobei $X^{12}$ A, L, T oder V ist; $X^{13}$ A, D, E, H, Q, R, S oder Y ist; $X^{14}$ A, E, I, L, N oder Q ist; $X^{15}$ A, D, E, H, ,N R, S, V oder Y ist, das vorzugsweise an monoklonale Antikörper, Antikörperfragmente und Antikörperkonjugate bindet, die eine CH1-Domäne enthalten.

14. Affinitätsmittel nach Anspruch 13, das Multimerpolypeptide umfasst, die mindestens zwei Untereinheiten umfassen, wobei jede Untereinheit ein Polypeptid nach Anspruch 13 ist, vorzugsweise mit einem oder mehreren der Folgenden:

wobei die Untereinheiten nicht alle gleich sind;
wobei der Ligand an eine feste Oberfläche gebunden ist;
wobei die feste Oberfläche ein Harz oder ein Kügelchen ist;
wobei die feste Oberfläche eine Membran ist;
wobei die feste Oberfläche ein Monolith ist;
wobei der Ligand über einen Linker an die feste Oberfläche konjugiert ist;

das zur Reinigung von monoklonalen Antikörpern, Antikörperfragmenten und Antikörperkonjugaten verwendet wird, die eine CH1-Domäne enthalten.

15. Verfahren zur Herstellung eines Affinitätsmittels, umfassend Konjugieren eines Liganden nach einem der Ansprüche 1 bis 6 an eine feste Oberfläche.

**Revendications**

1. Ligand d'affinité comprenant une séquence d'acides aminés représentée par la formule, en allant de la terminaison N à la terminaison C,

$$[A]\text{-AERWYDLGAAYAARGD}X_{17a}\text{DRA}X_{21a}\text{E}X_{23a}\text{Y}X_{25a}\text{R}X_{27a}\text{LE}X_{30a}\text{DPND-AWAW-WELG}X_{9b}\text{AYAARGDVDRAIEYYQRALELD}X_{32b}\text{NN-AVAWARLGIAYA}X_{13c}\text{RGDYDRAIEYYQRALELDPNN-EVAR}X_{5d}\text{ALEYARR}X_{13d}\text{-[B]}$$

dans lequel

$X_{17a}$ est F ou Y ; $X_{21a}$ est I ou T ; $X_{23a}$ est Y ou F ; $X_{25a}$ est R ou Q ; $X_{27a}$ est T ou A ; et $X_{30a}$ est L ou H ;
dans lequel
$X_{9b}$ est V ou I ; et $X_{32b}$ est L, P ou T ;
dans lequel
$X_{13c}$ est T ou A ;
dans lequel
$X_{5d}$ est D ou G ; et $X_{13d}$ est A ou V ; et
dans lequel
[A] est présent ou absent, et lorsqu'il est présent comprend M, MDE, MGGGGSAAAGDE, MGDE, MGHHHHHHDE, MGLAEAAAREAAARAADE, ou MAWAEFRQRLAAIRTRLEALGGSEAELAAFEREIAAFES ELQAYAGAGNPEVEAL RR EAAAIRDELQAYRHNDE ; et
[B] est présent ou absent, et lorsqu'il est présent comprend une cystéine, RRC, RRCHHHHHH, ou RRGQAGQGGGSGLNDIFEAQKIEWHECHHHHHH.

2. Ligand d'affinité selon la revendication 1, dans lequel $X_{17a}$ est F ; $X_{21a}$ est I ; $X_{23a}$ est Y ; $X_{25a}$ est R ; $X_{27a}$ est T ; $X_{30a}$ est L ; $X_{9b}$ est V ; $X_{32b}$ est P ; $X_{13c}$ est T ; $X_{5d}$ est D ; et $X_{13d}$ est V, ou

dans lequel $X_{17a}$ est Y ; $X_{21a}$ est T ; $X_{23a}$ est F ; $X_{25a}$ est Q ; $X_{27a}$ est A ; $X_{30a}$ est L ; $X_{9b}$ est I ; $X_{32b}$ est P ; $X_{13c}$ est A ; $X_{5d}$ est D ; et $X_{13d}$ est A, ou
dans lequel $X_{17a}$ est Y ; $X_{21a}$ est I ; $X_{23a}$ est Y ; $X_{25a}$ est Q ; $X_{27a}$ est A ; $X_{30a}$ est H ; $X_{9b}$ est V ; $X_{32b}$ est T ; $X_{13c}$ est A ; $X_{5d}$ est G ; et $X_{13d}$ est A, ou
dans lequel $X_{17a}$ est Y ; $X_{21a}$ est I ; $X_{23a}$ est Y ; $X_{25a}$ est Q ; $X_{25a}$ est A ; $X_{30a}$ est L ; $X_{9b}$ est V ; $X_{32b}$ est L ; $X_{13c}$ est A ; $X_{5d}$ est D ; et $X_{13d}$ est A.

3. Ligand d'affinité selon les revendications 1 ou 2, dans lequel [A] est MGHHHHHHDE et [B] est RRC ou dans lequel [A] est MGLAEAAAREAAARAADE et [B] est RRCHHHHHH.

4. Ligand d'affinité selon la revendication 2 ou 3, dans lequel ledit ligand comprend l'une quelconque des SEQ ID NO: 89, 128, 130, 131 ou 132. (groupe de ligand 129),
ou

dans lequel ledit ligand comprend l'une quelconque des SEQ ID NO: 90, 133, 135, 136 ou 137. (groupe de ligand 134),
ou
dans lequel ledit ligand comprend l'une quelconque des SEQ ID NO: 139, 140, 141 ou 143. (groupe de ligand 141),
ou
dans lequel ledit ligand comprend l'une quelconque des SEQ ID NO: 86, 91, 138, 142, 144, 145, 146, 147 ou 148. (groupe de ligand 144),

ou

dans lequel ledit ligand comprend l'une quelconque des SEQ ID NO: 129, 134, 141 ou 144, et/ou ce ligand d'affinité comprenant en outre une cystéine ou lysine de terminaison C.

5. Multimère comprenant une pluralité de ligands d'affinité selon l'une quelconque des revendications précédentes, de préférence ce multimère étant un dimère, trimère, tétramère, pentamère, hexamère, heptamère, octamère ou nonamère.

6. Ligand d'affinité ou multimère selon l'une quelconque des revendications 1 à 5, dans lequel ledit ligand ou multimère comprend en outre au moins un agent hétérologue lié fonctionnellement audit ligand d'affinité pour former de ce fait un conjugué ou une protéine de fusion, de préférence dans lequel ledit agent hétérologue est choisi dans le groupe constitué d'un ou plusieurs agents diagnostiques ou thérapeutiques à petite molécule ; une étiquette peptidique, une molécule d'ADN, d'ARN, ou d'ADN-ARN hybride ; un marqueur traçable ; un agent radioactif ; un anticorps ; un domaine variable à simple chaîne ; et un fragment d'immunoglubuline.

7. Matrice de séparation comprenant au moins un ligand d'affinité selon l'une quelconque des revendications 1 à 4, ou au moins un multimère selon la revendication 5 ou 6, de préférence dans laquelle les ligands d'affinité ou les multimères sont couplés à un support solide, plus préférablement dans laquelle les ligands d'affinité ou multimères sont couplés au support solide par l'intermédiaire de liaisons thiol, de préférence dans laquelle le support solide est une résine ou matrice de chromatographie, plus préférablement dans laquelle le support solide est une matrice d'agarose réticulée.

8. Procédé d'isolement d'un anticorps comprenant un domaine CH1, ou un fragment de celui-ci comprenant un domaine CH1, qui comprend la mise en contact dudit anticorps ou dudit fragment avec une matrice de séparation selon la revendication 7 et la récupération dudit anticorps ou fragment de celui-ci, de préférence

qui comprend (a) la mise en contact d'une matrice de séparation selon la revendication 7 avec une composition comprenant ledit anticorps ou ledit fragment, (b) le lavage de ladite matrice de séparation avec un tampon de lavage, (c) l'élution dudit anticorps ou dudit fragment par rapport à la matrice de séparation avec un tampon d'élution, et (d) la récupération dudit anticorps ou dudit fragment,
de préférence
qui comprend en outre (e) le traitement de ladite matrice de séparation avec une solution de nettoyage alcaline pendant un temps suffisant pour nettoyer ladite matrice de matériau résiduel et pour régénérer au moins 80 % de la capacité de liaison d'anticorps ou de fragment d'anticorps de ladite matrice de séparation
plus préférablement
dans lequel la solution de nettoyage alcaline comprend de 0,1 M NaOH à 0,5 M NaOH, même plus préférablement
dans lequel ladite matrice de séparation conserve au moins 80 % de sa capacité de liaison d'anticorps ou de fragment d'anticorps lorsque les étapes (a) à (e) sont répétées au moins 10 fois, et/ou dans lequel les étapes (a) à (e) sont répétées au moins 10 fois avec un lot unique de matrice de séparation.

9. Acide nucléique ou vecteur codant pour un ligand d'affinité selon l'une quelconque des revendications 1 à 4 ou un multimère selon la revendication 5 ou 6.

10. Vecteur d'expression comprenant l'acide nucléique ou le vecteur selon la revendication 9, dans lequel la région de codage dudit ligand d'affinité est liée fonctionnellement à un ou plusieurs éléments de commande d'expression.

11. Cellule hôte qui comprend un acide nucléique ou un vecteur selon la revendication 9 ou 10, de préférence dans laquelle la cellule hôte est *E. coli* ou *P. pastoris.*

12. Procédé de fabrication d'une matrice de séparation comprenant la conjugaison d'un ligand selon l'une quelconque des revendications 1 à 4 ou d'un multimère selon la revendication 5 ou 6 à une surface solide.

13. Agent d'affinité comprenant un ligand comprenant un ligand TPR comprenant 7 hélices dans lequel la 1$^{re}$ hélice comprend la séquence SEQ ID No: 1

$X^1$ RW$X^2X^3$, où $X^1$ est A, D, E, G, N, Q, S ou V ; $X^2$ est A, E, W ou Y ; $X^3$ est D, G, H, I, N, T, V, W ou Y ;
la 3$^e$ hélice comprend la séquence SEQ ID No: 2
$X^4$AW$X^5X^6$LG$X^7$où $X^4$ est W ; $X^5$ est E ou W ; $X^6$ est D, E ou H ; $X^7$ est T, V, W ;

la 5$^e$ hélice comprend la séquence SEQ ID No: 3

X$^8$AWX$^9$X$^{10}$LGX$^{11}$, où X$^8$ est A, H, I, T ou V ; X$^9$ est A, F, N, S ou R ; X$^{10}$ est R ; X$^{11}$ est F, I, H ou W ;

la 7$^e$ hélice comprend la séquence SEQ ID No: 4

X$^{12}$ARX$^{13}$X$^{14}$LEX$^{15}$, où X$^{12}$ est A, L, T ou V ; X$^{13}$ est A, D, E, H, Q, R, S ou Y ; X$^{14}$ est A, E, I, L, N ou Q ; X$^{15}$ est A, D, E, H, N, R, S, V ou Y de préférence qui se lie à des anticorps monoclonaux, des fragments d'anticorps et des conjugués d'anticorps contenant un domaine CH1.

14. Agent d'affinité selon la revendication 13 qui comprend des polypeptides multimères comprenant au moins deux sous-unités, chaque sous-unité étant un polypeptide selon la revendication 13 de préférence avec un ou plusieurs de ce qui suit :

où les sous-unités ne sont pas toutes les mêmes ;
dans lequel le ligand est fixé à une surface solide ;
dans lequel la surface solide est une résine ou une perle ;
dans lequel la surface solide est une membrane ;
dans lequel la surface solide est un monolithe ;
dans lequel le ligand est conjugué à la surface solide par l'intermédiaire d'un lieur ;
qui est utilisé pour la purification d'anticorps monoclonaux, de fragments d'anticorps et de conjugués d'anticorps contenant un domaine CH1.

15. Procédé de fabrication d'un agent d'affinité comprenant la conjugaison d'un ligand selon l'une quelconque des revendications 1 à 6 à une surface solide.

Fig. 1

## SEQ ID: 89

## SEQ ID: 90

## SEQ ID: 92

**Fig. 2**

SEQ ID: 89

SEQ ID: 90

SEQ ID: 92

Fig. 3

SEQ ID: 129

**Fig. 4**

SEQ ID: 134

**Fig. 5**

SEQ ID: 141

Fig. 6

**Fig. 7**

Fig. 8

Fig. 9

**Fig. 10**

Fig. 11

**Fig. 12**

**Fig. 13**

**Fig. 14**

**Fig. 15**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012105833 A1
- WO 8605807 A
- WO 8901036 A
- US 5122464 A
- US 6815184 B
- US 365769
- US 60368047
- WO 2004057002 A
- WO 2004024927 A
- WO 2003078614 A
- US 5350674 A
- US 5585362 A
- US 20040009530 A

**Non-patent literature cited in the description**

- **KING** ; **STANSFIELD**. A dictionary of genetics. Oxford University Press, 2002
- **BRUMMELL**. *Biochem.*, 1993, vol. 32, 1180-1187
- **KOBAYASHI**. *Protein Eng.*, 1999, vol. 12 (10), 879-884
- **BURKS**. *PNAS*, 1997, vol. 94, 412-417
- **NERI D et al.** *Tibtech*, 1996, vol. 14, 465-470
- **JANSSON M et al.** *J Biol Chem*, 1997, vol. 272, 8189-8197
- **MEYER et al.** *Biomacromolecules*, 2002, vol. 3, 357-367
- **KURIHARA et al.** *Biotechnol. Lett.*, 2005, vol. 27, 665-670
- **HAIDER et al.** *Mol. Pharm.*, 2005, vol. 2, 139-150
- **MCMILLAN et al.** *Macromolecules*, 1999, vol. 32 (11), 3643-3646
- **STUDIER**. *J. Mol. Biol.*, 1991, vol. 219, 37
- **SCHOEPFER**. *Gene*, 1993, vol. 124, 83
- **ROSENBERG et al.** *Gene*, 1987, vol. 56, 125-135
- **CHANG et al.** *Nature*, 1978, vol. 275, 615
- **GOEDDEL et al.** *Nature*, 1979, vol. 281, 544
- **GOEDDEL et al.** *Nucl. Acids Res.*, 1980, vol. 8, 4057
- **SAMBROOK et al.** Molecular Cloning, A Laboratory Manual. Cold Spring Harbor Laboratory, 1990
- **HITZEMAN**. *J. Biol. Chem.*, 1980, vol. 255, 2073
- **FLEER**. *Gene*, 1991, vol. 107, 285-195
- **HINNEN**. *PNAS*, 1978, vol. 75, 1929
- **BORTH et al.** *Biotechnol. Bioen*, 2000, vol. 71 (4), 266-73
- **WERNER et al.** *Arzneimittelforschung/Drug Res.*, 1998, vol. 48 (8), 870-80
- **ANDERSEN et al.** *Curr. Op. Biotechnol.*, 2002, vol. 13, 117-123
- **CHADD et al.** *Curr. Op, Biotechnol.*, 2001, vol. 12, 188-194
- **GIDDINGS**. *Curr. Op. Biotechnol.*, 2001, vol. 12, 450-454
- **LOGAN et al.** *PNAS*, 1984, vol. 81, 355-359
- **BIRTNER et al.** *Methods Enzymol.*, 1987, vol. 153, 51-544
- **SAMBROOK et al.** Molecular Cloning: A Laboratory Manual. Cold Spring Harbor Laboratory, 2001
- **GHOSH et al.** *Glycobiology*, 1991, vol. 5, 505-510
- **UI-TEI et al.** *FEBS Lett.*, 2000, vol. 479, 79-82
- **LOWY et al.** *Cell*, 1980, vol. 22, 817
- **TAM et al.** *J. Am. Chem. Soc.*, 1983, vol. 105, 6442
- **MERRIFIELD**. *Science*, 1986, vol. 232, 341-347
- **BARANY** ; **MERRIFIELD**. The Peptides. Academic Press, 1-284
- **BARANY et al.** *Int. J. Pep. Protein Res.*, 1987, vol. 30 (705), 739
- **KELLEY et al.** Genetic Engineering Principles and Methods. Plenum Press, 1990, vol. 12, 1-19
- **STEWART et al.** Solid-Phase Peptide Synthesis. W.H. Freeman Co, 1989
- Creighton, Proteins: Structures and Molecular Properties. W.H. Freeman and Co, 1992
- Postranslational Covalent Modification of Proteins. Academic Press, 1983, 1-12
- **SEIFTER**. *Meth. Enzymol.*, 1990, vol. 182, 626-646
- **RATTAN**. *Ann. NY Acad. Sci.*, 1992, vol. 663, 48-62
- **SHUKLA** ; **HINCKLEY**. *Biotechnol Prog*, September 2008, vol. 24 (5), 1115-21